# EUROPEAN PATENT APPLICATION

(11) **EP 2 692 740 A1**
(43) Date of publication of application: **05.02.2014**
(21) Application number: 12305937.0
(22) Date of filing: 30.07.2012
(51) Int. Cl.: C08B 37/00, A61K 38/02

(54) **Glycan compositions, processes for preparing the same and their uses as a drug**

(71) Applicant: Le Centre National de la Recherche Scientifique, 75794 Paris Cedex 16 (FR); National University Corporation Nagoya University, Aichi 464-8601 (JP)
(72) Inventor: Guerardel, Yann, 59790 Ronchin (FR); Kitajima, Ken, Nagoya 464-8601 (JP); Sawada, Hitochi, Toba, Mie 517-0004 (JP); Megraud, Francis, 39000 Bordeaux (FR); Ferrand, Jonathan, St Kilda, VIC, 3182 (AU)
(74) Representative: Grosset-Fournier, Chantal Catherine

(57) **Abstract**

The present invention relates to glycan compositions, processes for preparing the same and their uses as a drug.

## Description

The present invention relates to glycan compositions, processes for preparing the same and their uses as a drug.

*Helicobacter pylori* is a Gram-negative, microaerophilic bacterium that can cause infection of the stomach. This infection can contribute to the development of diseases, such as dyspepsia (upper digestive pain), gastritis (inflammation of the stomach), and ulcers in the stomach and duodenum. *H. pylori* usually resides within the protective mucous layer lining the stomach epithelium, where it is protected from outside harm. Furthermore it produces long threads that attach to the underlying stomach cells. These bacteria do not actually invade the stomach cells but exert their deleterious effect from the surface of the epithelium. Stomach and duodenal ulcers that are the most common disease induced by *H. pylori* infection are found in 5 to 10 percent of patients infected. It is probably the result of a weakening of the protective mucous layer of the stomach that allows acid to seep in and injure the underlying stomach cells. In addition, *H. pylori* can produce and release several bioactive factors that may directly affect the stomach cells. However *H. pylori* may be responsible for more serious pathologies including duodenal and gastric ulcers, stomach cancers and gastric lymphomas. Whereas the mortality rate related to *H. pylori* infection due to the complications of the infection, such as gastric ulcer perforation and gastric cancer is thought to be low (ie, approximately 2-4% of all infected people), the morbidity associated to this infection is extremely high.

Treatment of *H. pylori* infection is achieved by combining antibiotics, and drugs designed to reduce stomach acidity, essentially proton pump inhibitors (omeprazole, lansoprazole, rabeprazole, pantoprazole). Eradication treatment typically consists of triple therapies as exemplified by the combinations: omeprazole, amoxicillin, and clarithromycin (DAC) for 10 to 14 days; bismuth subsalicylate, metronidazole, and tetracycline (BMT) for 14 days. All the eradication treatments have a high incidence of certain adverse effects (e.g., nausea, metallic taste, skin rash, diarrhea). Up to 50 percent of patients have side effects while taking H. pylori treatment. Side effects are usually mild, and fewer than 10 percent of patients stop treatment because of side effects.

Aforesaid proton pump inhibitor (PPI)-based triple therapy generally combining two antibiotics such as clarithromycin and amoxicillin has been used as first-line treatment of choice for over a decade. In recent years, increasing antimicrobial resistance has resulted in falling eradication rates with standard therapies. Up to 30 percent of patients with *H. pylori* infection are not cured after completing their first course of treatment. In particular, clarithromycin resistance rates are higher than 20% in most European countries (except Northern Europe). Furthermore, the prevalence of secondary clarithromycin resistance, i.e. after failure of a treatment including this drug, is extremely high, up to 60%. Then, resistance to metronidazole in developing countries is about 35% and much higher in developing countries.

*Halocynthia roretzi* is an edible ascidian consumed in Korea and in Japan. *Halocynthia roretzi* is farmed by food industry in Korea and Hokkaido since 1984. The worldwide production of *Halocynthia roretzi* in 2006 was about 21,000 tons, exclusively devoted to human consumption.

One objective of the present invention is to provide glycan compositions liable to treat *H. pylori* infection in an alternative way compared to common treatments, without adverse side effects.

Another aim of the present invention is to provide glycan compositions enabling to reduce the intake of antibiotics when treating *H. pylori* infection.

Another aim of the invention is to provide glycan compositions liable to constitute adjuvant treatment of *H. pylori* infection.

Another aim of the invention is to provide glycan compositions for use as anti-adherence drug, in particular towards *H. pylori.*

Still another aim of the invention is to provide glycan compositions isolated from safe and easily available sources, in particular from *Halocynthia roretzi.*

The present invention relates to a composition, for use as anti-adherence drug to treat infectious disease(s), comprising at least two glycans or glycopeptides comprising independently of each other at least one motif of structure I: 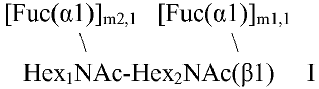 wherein:
m_{1,1} and m_{2,1} are integers equal to 0 or 1,
Hex₁ and Hex₂ represent Glc or Gal,
provided that:
   - at least one of m_{1,1} and m_{2,1} is equal to 1, and
   - when Hex₁ is Glc, Hex₂ is Gal,
   - and when Hex₁ is Gal, Hex₂ is Glc.

When Hex₁ is Gal and Hex₂ is Glc, structure I corresponds to structure I-1: 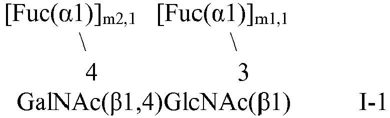 wherein m_{1,1} and m_{2,1} have the meaning indicated above.

When Hex₁ is Glc and Hex₂ is Gal, structure I corresponds to structure I-2: 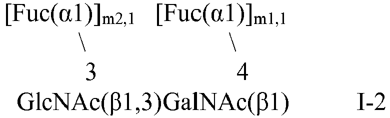 wherein m_{1,1} and m_{2,1} have the meaning indicated above.

By "anti-adherence drug" is meant a drug inhibiting adherence of pathogenic organisms.

By "glycan" is meant a polysaccharide or oligosaccharide.

By "glycopeptide" is meant a peptide which contains one or more glycan(s) covalently attached to a peptide side chains or a single amino acid.

The abbreviation "Fuc" refers to fucose.

The abbreviation "Glc" refers to glucose.

The abbreviation "Gal" refers to galactose.

The abbreviation "GlcNAc" refers to N-acetylglucosamine.

The abbreviation "GalNAc" refers to N-acetylgalactosamine.

Aforesaid at least two glycans or glycopeptides comprising independently of each other at least one motif of structure I are of different structures, which are under separate form i.e. which are not covalently linked to each other.

Interestingly, the inventors have found that glycan or glycopeptide compositions of the invention inhibit *H. pylori* adherence to epithelial cells. The inhibition was observed with both glycans and glycopeptides.

In an advantageous embodiment, the present invention relates to a composition, for use as anti-adherence drug to treat infectious disease(s), comprising at least two glycans or glycopeptides comprising at least one motif of structure I-1: 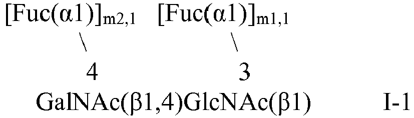 wherein m_{1,1} and m_{2,1} have the meaning indicated above.

In an advantageous embodiment, the present invention relates to a composition, for use as anti-adherence drug to treat infectious disease(s), comprising at least two glycans or glycopeptides comprising at least one motif of structure I-2: 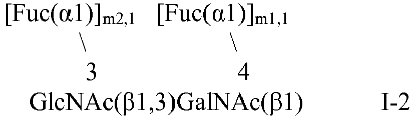 wherein m_{1,1} and m_{2,1} have the meaning indicated above.

In an advantageous embodiment, the present invention relates to a composition for use as anti-adherence drug to treat infectious disease(s) caused by bacteria or virus known to use fucosylated epitopes as ligands, in particular the bacteria species *Helicobacter pylori,* the bacteria of the *Burkholderia* genus, rotaviruses and notaviruses.

In an advantageous embodiment, the present invention relates to a composition for use as anti-adherence drug to treat infectious disease(s) caused by the bacteria species *Helicobacter pylori.*

In an advantageous embodiment, the present invention relates to a composition, for use as anti-adherence drug to treat infectious disease(s), comprising at least two glycans or glycopeptides comprising at least one motif of structure I, wherein m_{1,1} is equal to 1 and m_{2,1} is equal to 0, of particular formula II:

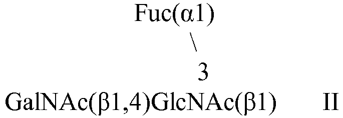

In an advantageous embodiment, the present invention relates to a composition, for use as anti-adherence drug to treat infectious disease(s), comprising at least two glycans or glycopeptides comprising at least one motif of structure I, wherein m_{1,1} is equal to 0 and m_{2,1} is equal to 1, of particular formula III:

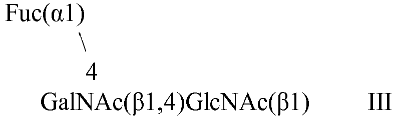

In an advantageous embodiment, the present invention relates to a composition, for use as anti-adherence drug to treat infectious disease(s), comprising at least two glycans or glycopeptides comprising at least one motif of structure I, wherein m_{1,1} is equal to 0 and m_{2,1} is equal to 1, of particular formula III-2:

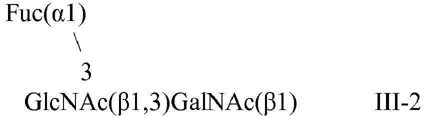

In an advantageous embodiment, the present invention relates to a composition, for use as anti-adherence drug to treat infectious disease(s), comprising at least two glycans or glycopeptides comprising at least one motif of structure I, wherein m_{1,1} and m_{2,1} are equal to 1, of particular formula IV:

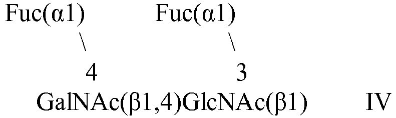

In an advantageous embodiment, the present invention relates to a composition, for use as anti-adherence drug to treat infectious disease(s), comprising at least two glycans or glycopeptides comprising at least one motif of structure I, wherein m_{1,1} and m_{2,1} are equal to 1, of particular formula IV-2:

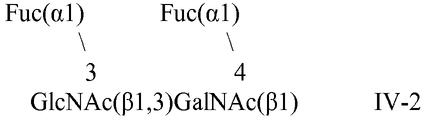

In an advantageous embodiment, the present invention relates to a composition, for use as anti-adherence drug to treat infectious disease(s), comprising at least two glycans or glycopeptides comprising at least one motif of structure V: 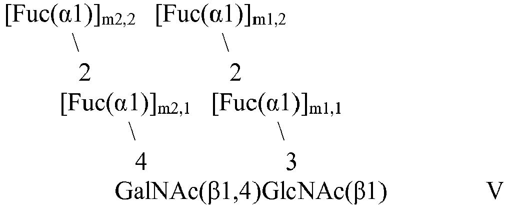 wherein:
m_{1,1}; m_{1,2}; m_{2,1} and m_{2,2} are integers equal to 0 or 1,
provided that at least one of m_{1,1} m_{1,2}; m_{2,1} and m_{2,2} is equal to 1.

In an advantageous embodiment, the present invention relates to a composition, for use as anti-adherence drug to treat infectious disease(s), comprising at least two glycopeptides comprising independently of each other a motif of structure VI: 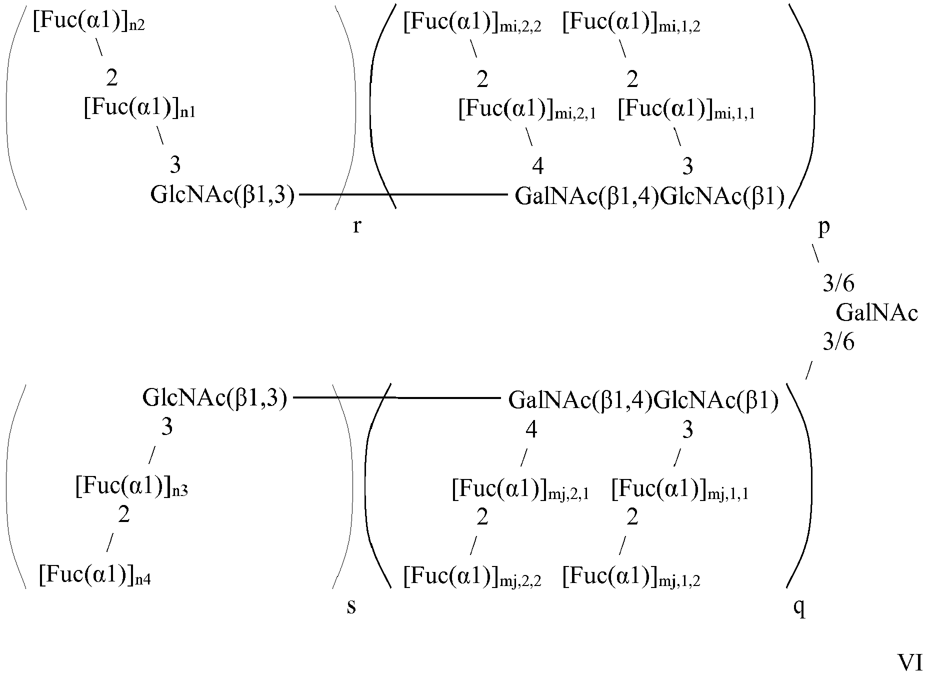 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2},₁ m_{j,2},₂ ; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and M_{i,2,2} is equal to 1, and/or
   - q > 0 and there is at least one j such as at least one of m_{j,1,1}; m_{j,1,2}; m_{j,2,1} and m_{j,2,2} is equal to 1, and/or
   - p > 0, r > 0 and there at least one of m_{i,1,1}; m_{i,1,2}; m_{i,2,1}; m_{i,2,2}; n₁; n₂; n₃ and n₄ is equal to 1, and/or
   - q > 0, s > 0 and there at least one of m_{i,1,1}; m_{i,1,2}; m_{i,2,1}; m_{i,2,2}; n₁; n₂; n₃ and n₄ is equal to 1.

By the terms "3/6" is meant that the two substituents of the terminal GalNac substitute aforesaid GalNac in respectively position 3 and 6, or 6 and 3. A compound of structure VI is consequently of one of the two following formulae: 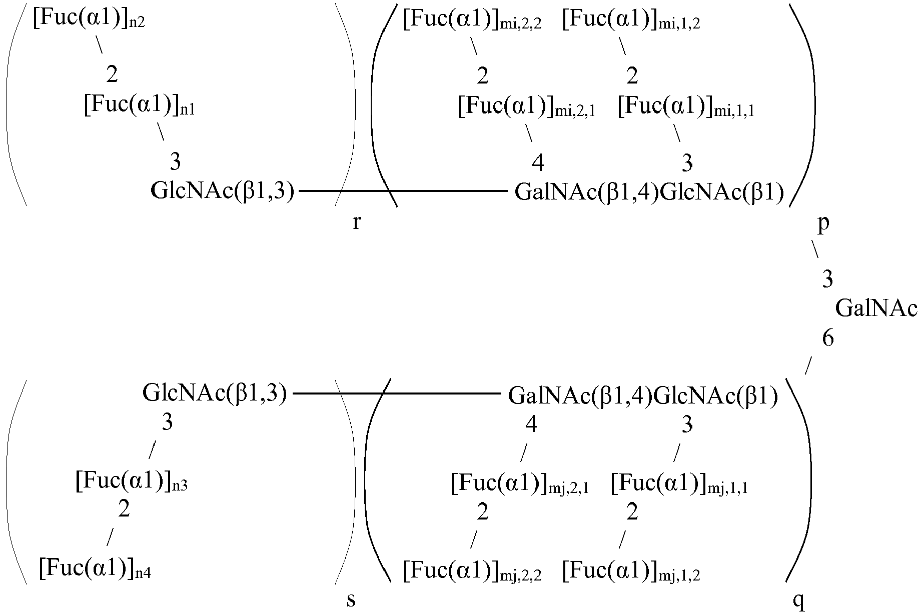 and 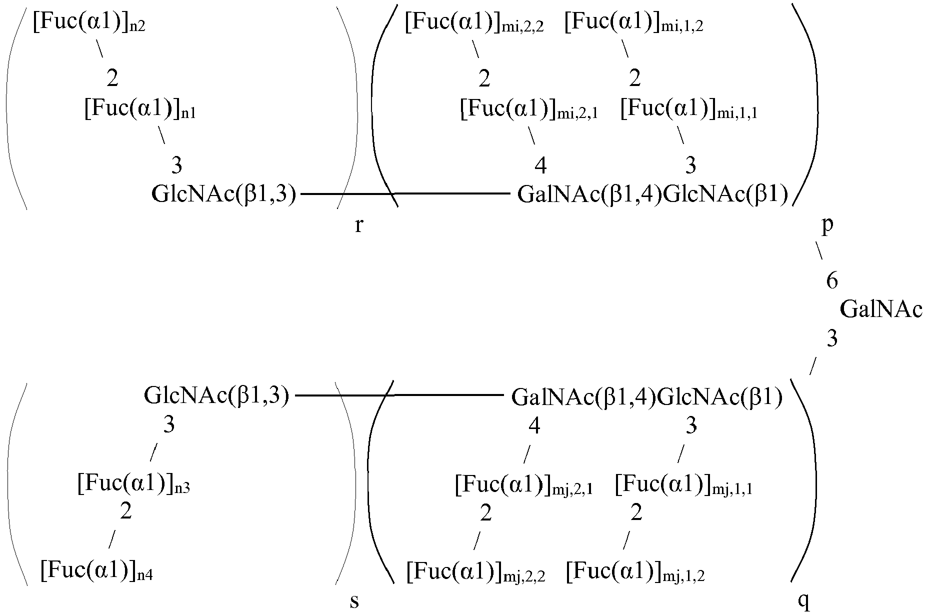 wherein r, p, s, q, m_{i,1,1}; m_{i,1,2}; m_{i,2,1}; m_{i,2,2}; m_{j,1,1}; m_{j,1,2}; m_{j,2,1}; m_{j,2,2}; n₁; n₂; n₃ and n₄ have the meaning indicated above.

When p is equal to 1, the pattern: 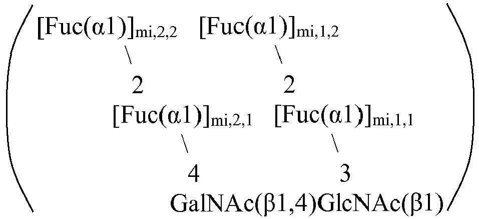 is present one time, corresponding to i=1.

When p is equal to 2, aforesaid pattern is present p=2 times, the pattern corresponding to i=1 and the pattern corresponding to i=2 being linked through a β1,3 bond.

Similarly, when q is equal to 1, the pattern: 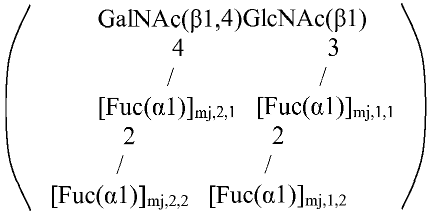 is present one time, corresponding to j=1.

When q is equal to 2, aforesaid pattern is present q=2 times, the pattern corresponding to j=1 and the pattern corresponding to j=2 being linked through a β1,3 bond.

When for example p is equal to 2, and q, r and s are equal to 0, said at least two glycopeptides of structure VI are of the following particular structure:

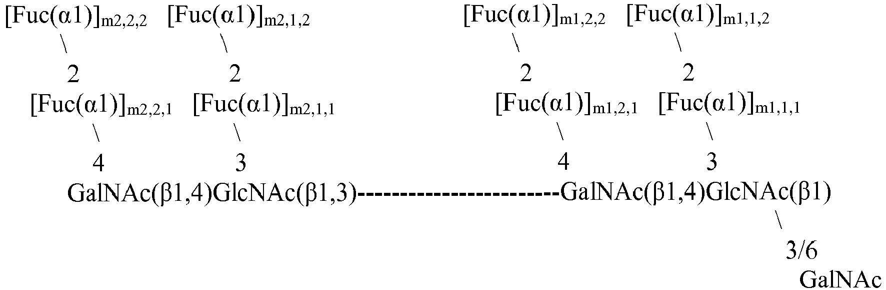

In an advantageous embodiment, the present invention relates to a composition, for use as anti-adherence drug to treat infectious disease(s), comprising at least two glycopeptides comprising independently of each other a motif of structure VI: 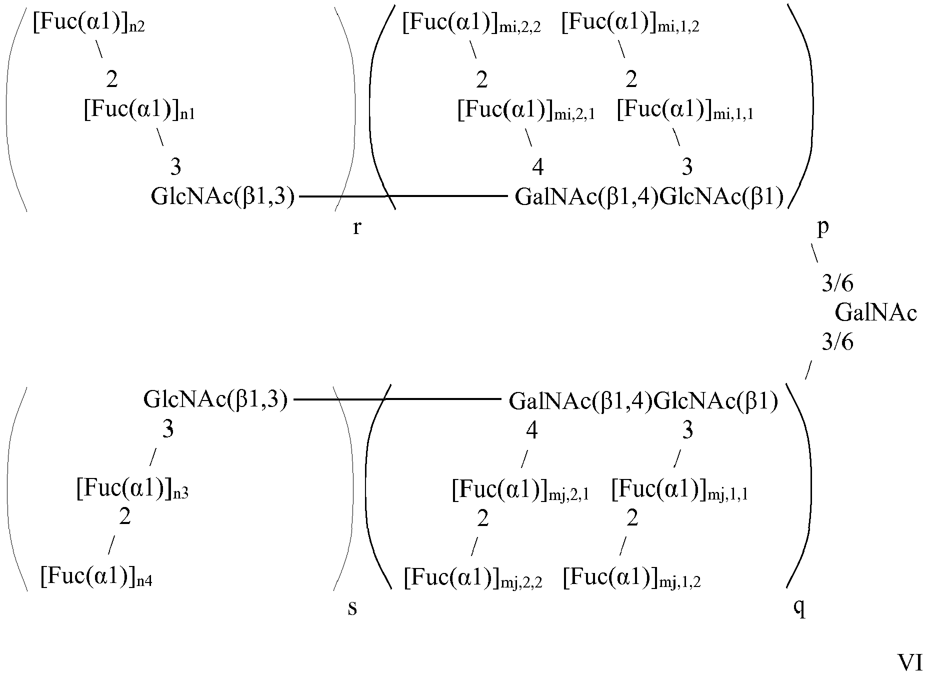 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and m_{i,2,2} is equal to 1, and/or
   - q > 0 and there is at least one j such as at least one of m_{j,1,1}; m_{j,1,2}; m_{j,2,1} and m_{j,2,2} is equal to 1.

In an advantageous embodiment, the present invention relates to a composition, for use as anti-adherence drug to treat infectious disease(s), comprising at least two glycopeptides comprising independently of each other a motif of structure VII: 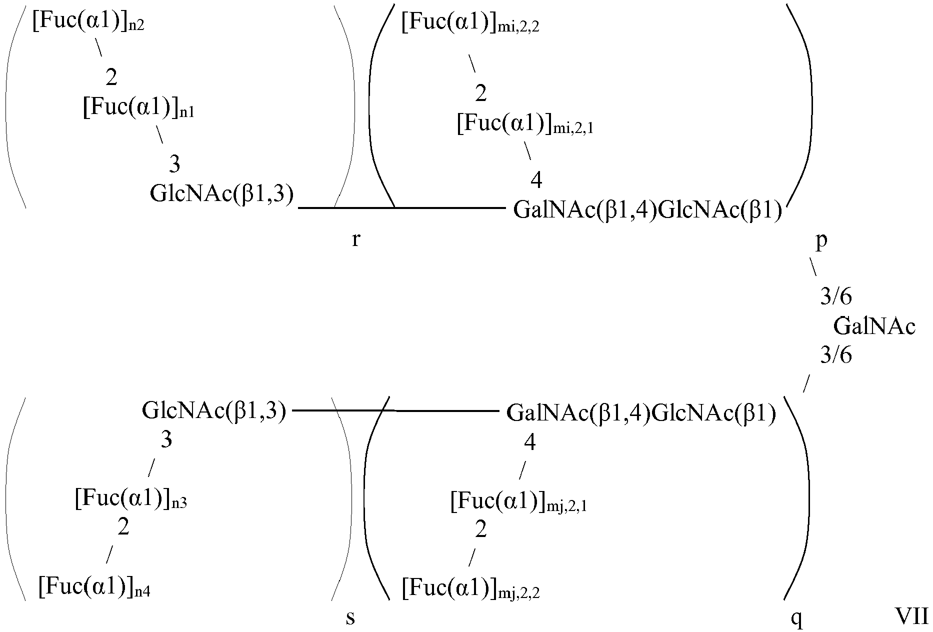 wherein r, p, q, s, m_{i,2,1}; m_{i,2,2}; m_{j,2,1}; m_{j,2,2}; n₁; n₂; n₃ and n₄ have the meaning indicated above.

In an advantageous embodiment, the present invention relates to a composition, for use as anti-adherence drug to treat infectious disease(s), comprising at least two glycopeptides comprising independently of each other a motif of structure VIII: 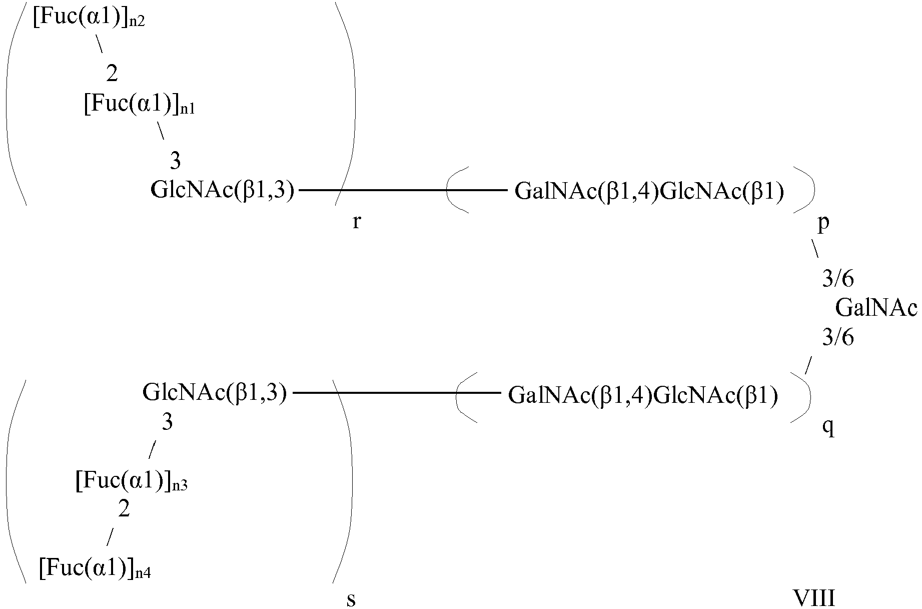 wherein r, p, s, q, n₁; n₂; n₃ and n₄ have the meaning indicated above.

In an advantageous embodiment, the present invention relates to a composition, for use as anti-adherence drug to treat infectious disease(s), comprising at least two glycopeptides comprising independently of each other a motif of structure IX: 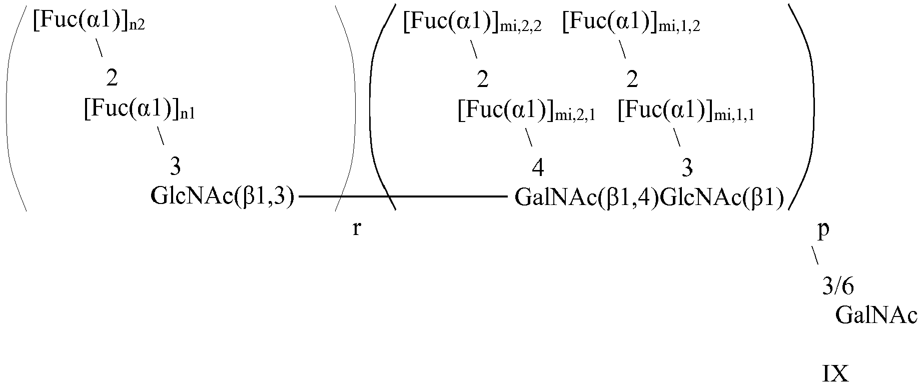 wherein r, p, m_{i,1,1}; m_{i,1,2}; m_{i,2,1}; m_{i,2,2}; n₁ and n₂ have the meaning indicated above.

In an advantageous embodiment, the present invention relates to a composition, for use as anti-adherence drug to treat infectious disease(s), comprising at least two glycopeptides comprising independently of each other a motif of structure X: 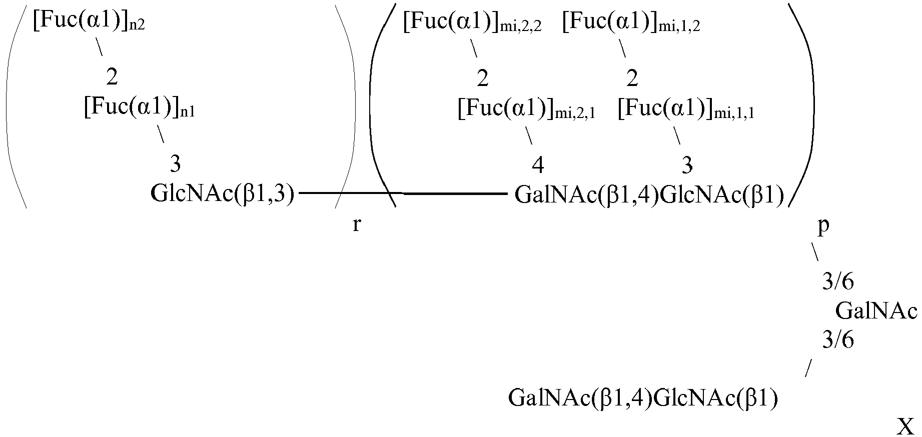 wherein r, p, m_{i,1,1}; m_{i,1,2}; m_{i,2,1}; m_{i,2,2}; n₁ and n₂ have the meaning indicated above.

In an advantageous embodiment, the present invention relates to a composition, for use as anti-adherence drug to treat infectious disease(s), comprising at least two glycopeptides comprising independently of each other a motif of structure XI: 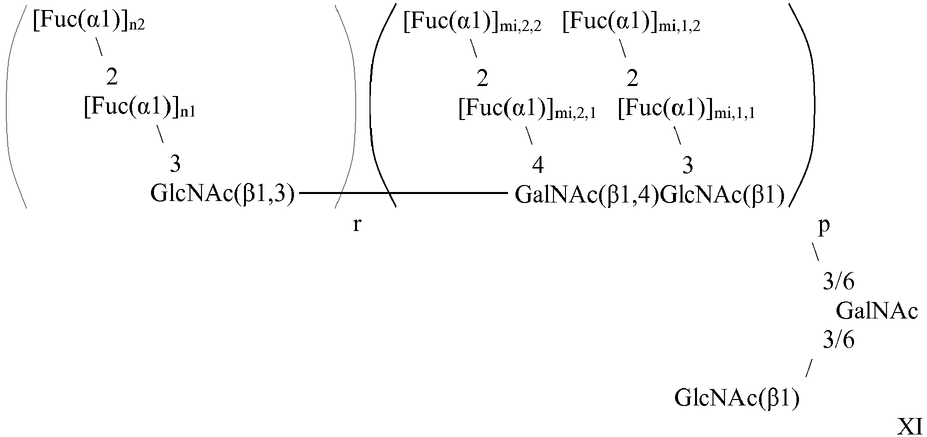 wherein r, p, m_{i,1,1}; m_{i,1,2}; m_{i,2,1}; m_{i,2,2}; n₁ and n₂ have the meaning indicated above.

In an advantageous embodiment, the present invention relates to a composition, for use as anti-adherence drug to treat infectious disease(s), comprising at least two glycans of structure VI-2: 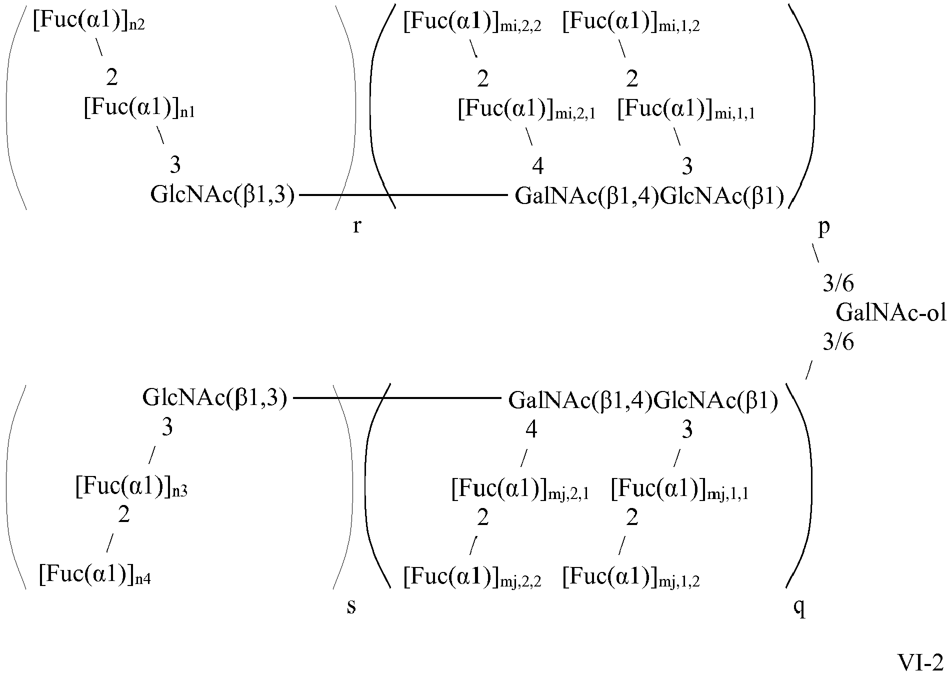 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2} ; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and m_{i,2,2} is equal to 1, and/or
   - q > 0 and there is at least one j such as at least one of m_{i,1,1}; m_{j,1,2}; m_{j,2,1} and m_{j,2,2} is equal to 1, and/or
   - p > 0, r > 0 and there at least one of m_{i,1,1}; m_{i,1,2}; m_{i,2,1}; m_{i,2,2}; n₁; n₂; n₃ and n₄ is equal to 1, and/or
   - q > 0, s > 0 and there at least one of m_{i,1,1}; m_{i,1,2}; m_{i,2,1}; m_{i,2,2}; n₁; n₂; n₃ and n₄ is equal to 1.

The abbreviation "GalNAc-ol" refers to N-acetylgalactosaminitol.

Glycans of structure VI-1 are O-glycans: they originate from glycopeptides wherein the glycan part(s) is (are) linked to the peptide by a covalent bond involving an atom of oxygen.

In an advantageous embodiment, the present invention relates to a composition, for use as anti-adherence drug to treat infectious disease(s), comprising at least two glycopeptides comprising independently of each other a motif of structure VI-2: 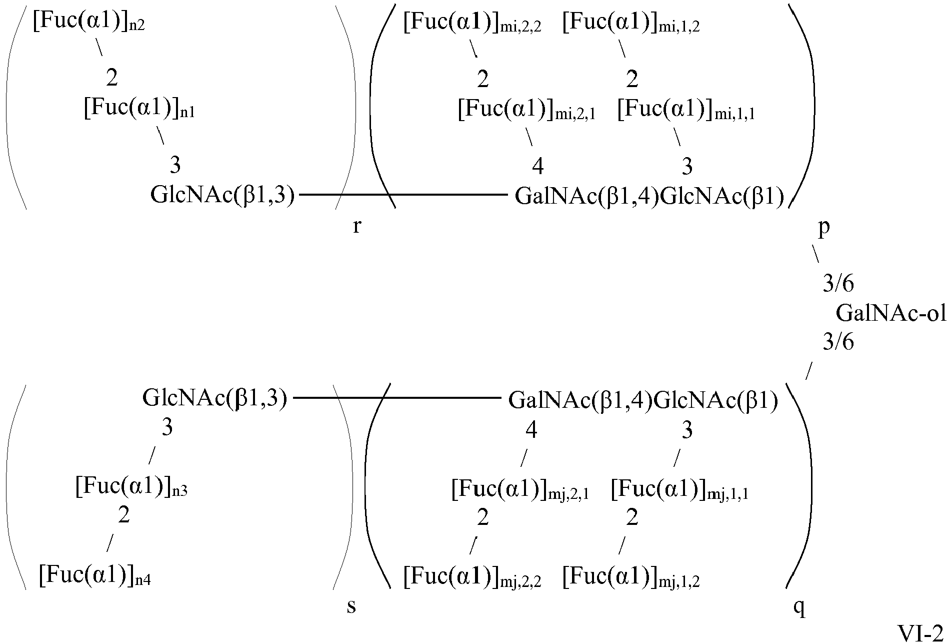 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{i,1,1}; m_{j,1,2}; m_{i,2,1}; m_{j,2,2}; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and m_{i,2,2} is equal to 1, and/or
   - q > 0 and there is at least one j such as at least one of m_{j,1,1}; m_{j,1,2}; m_{j,1,2} and m_{j,2,2} is equal to 1.

In an advantageous embodiment, the present invention relates to a composition, for use as anti-adherence drug to treat infectious disease(s), comprising at least two glycans of structure VII-2: 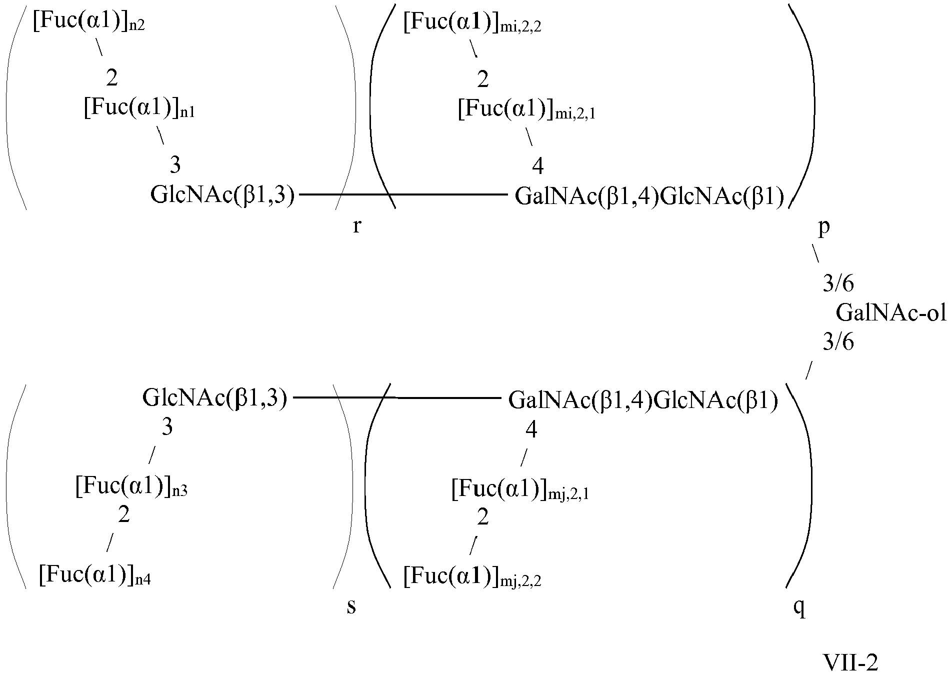 wherein r, p, s, q, m_{i,2,1}; m_{i,2,2}; m_{j,2,1}; m_{j,2,2}; n₁; n₂; n₃ and n₄ have the meaning indicated above.

In an advantageous embodiment, the present invention relates to a composition, for use as anti-adherence drug to treat infectious disease(s), comprising at least two glycans of structure VIII-2 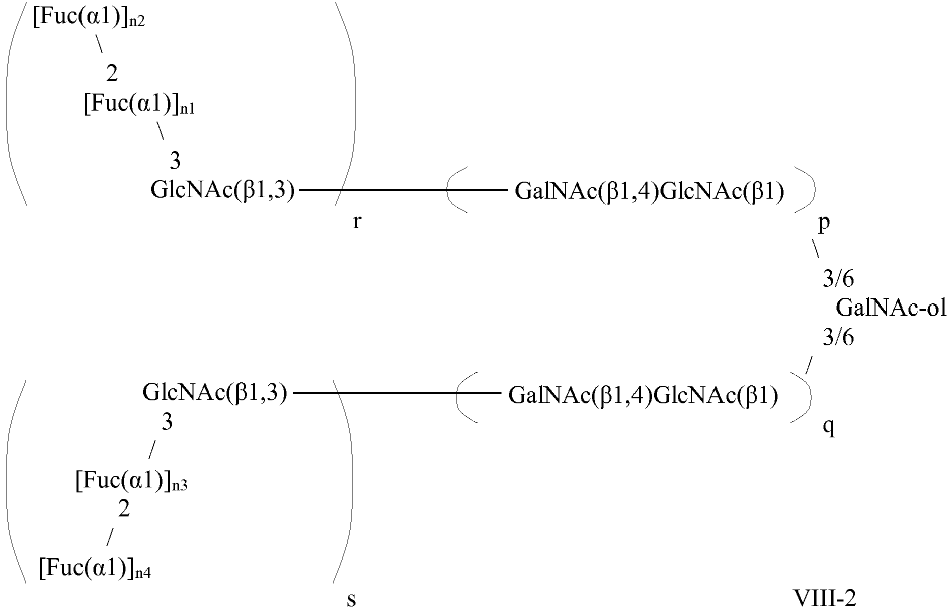 wherein r, p, s, q, n₁; n₂; n₃ and n₄ have the meaning indicated above.

In an advantageous embodiment, the present invention relates to a composition, for use as anti-adherence drug to treat infectious disease(s), comprising at least two glycans of structure IX-2: 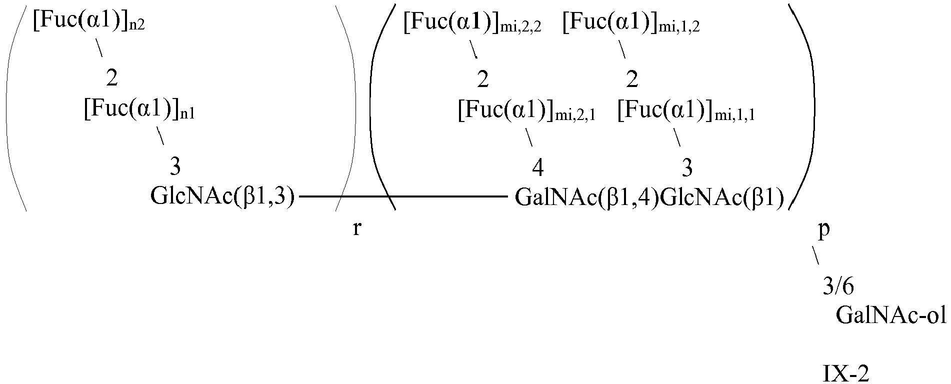 wherein r, p, m_{i,1,1}; m_{i,1,2}; m_{i,2,1}; m_{i,2,2}; n₁ and n₂ have the meaning indicated above.

In an advantageous embodiment, the present invention relates to a composition, for use as anti-adherence drug to treat infectious disease(s), comprising at least two glycans of structure X-2: 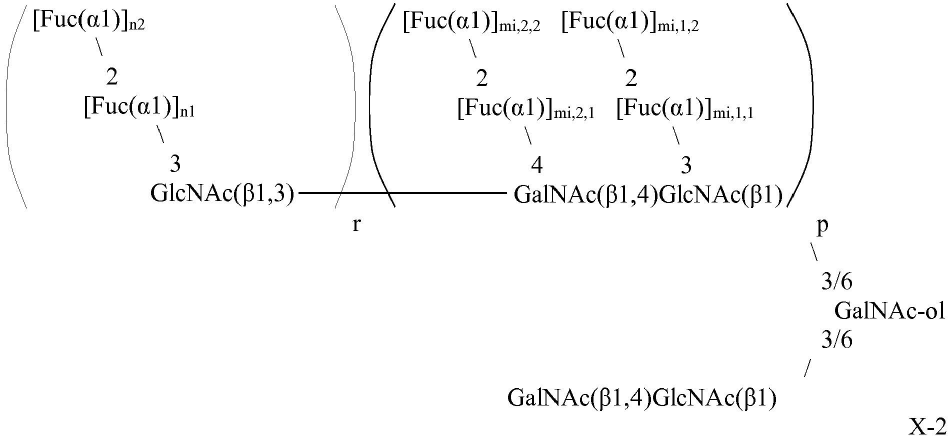 wherein r, p, m_{i,1,1}; m_{i,1,2}; m_{i,2,1}; m_{i,2,2}; n₁ and n₂ have the meaning indicated above.

In an advantageous embodiment, the present invention relates to a composition, for use as anti-adherence drug to treat infectious disease(s), comprising at least two glycans of structure XI-2: 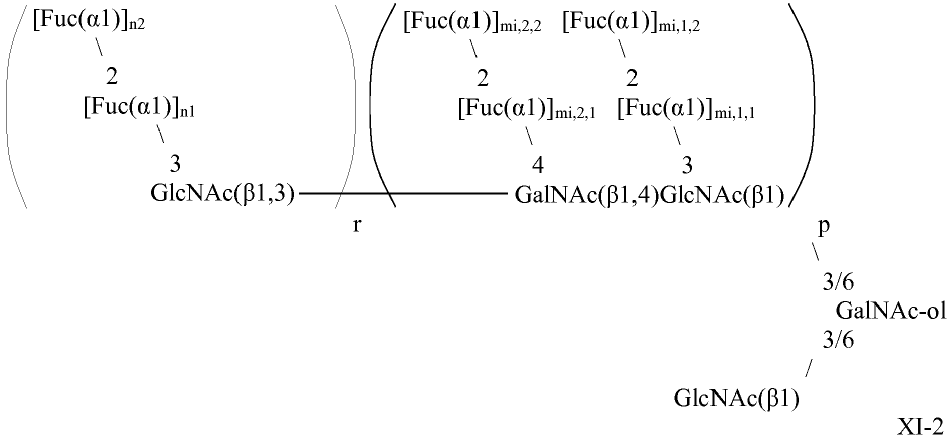 wherein r, p, m_{i,1,1}; m_{i,1,2}; m_{i,2,1}; m_{i,2,2}; n₁ and n₂ have the meaning indicated above.

In an advantageous embodiment, the present invention relates to a composition, for use as anti-adherence drug to treat infectious disease(s), comprising at least two glycopeptides comprising independently of each other a motif of structure VI, of the following particular formula XII: 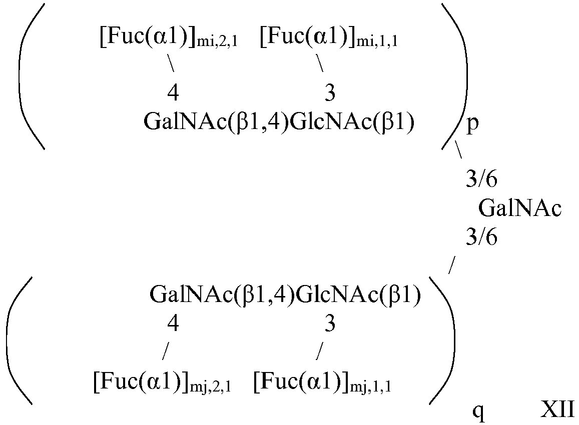 wherein p, q, m_{i,1,1}; m_{i,2,1}; m_{j,1,1} and m_{j,2,1} have the meaning indicated above.

In an advantageous embodiment, the present invention relates to a composition, for use as anti-adherence drug to treat infectious disease(s), comprising at least two glycans of structure VI-2, of the following particular formula XII-2: 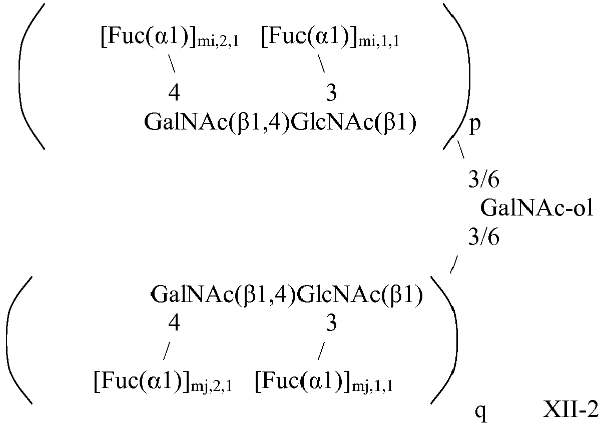 wherein p, q, m_{i,1,1}; m_{i,2,1} ; m_{j,1,1} and m_{j,2,1} have the meaning indicated above.

In an advantageous embodiment, the present invention relates to a composition, for use as anti-adherence drug to treat infectious disease(s), comprising at least two glycopeptides comprising independently of each other a motif of structure VI, of the following particular formula XIII: 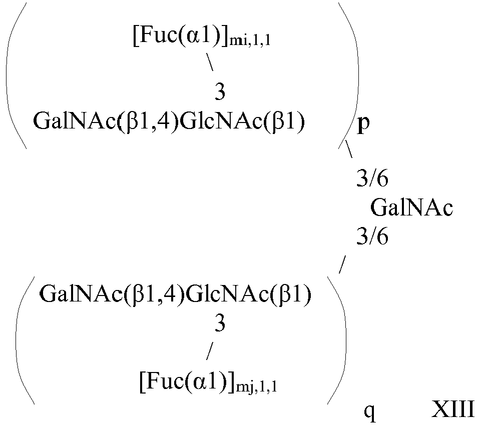 wherein p, q, m_{i,1,1} and m_{j,1,1} have the meaning indicated above.

In an advantageous embodiment, the present invention relates to a composition, for use as anti-adherence drug to treat infectious disease(s), comprising at least two glycans of structure VI-2, of the following particular formula XIII-2: 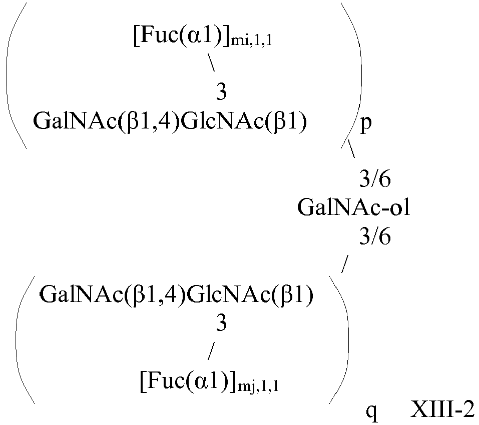 wherein p, q, m_{i,1,1} and m_{j,1,1} have the meaning indicated above.

In an advantageous embodiment, the present invention relates to a composition, for use as anti-adherence drug to treat infectious disease(s), comprising at least two glycopeptides comprising independently of each other a motif of structure VI, of the following particular formula XIV: 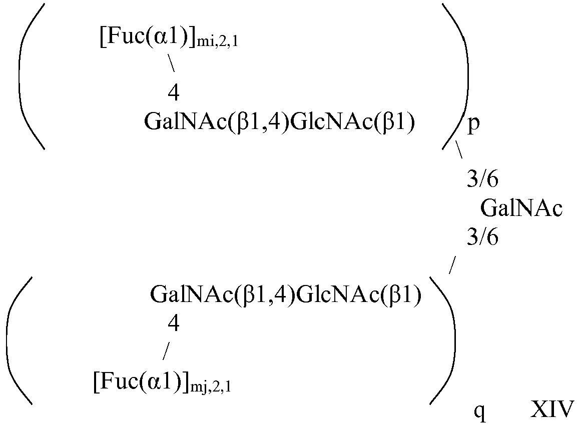 wherein p, q, m_{i,2,1} and m_{j,2,1} have the meaning indicated above.

In an advantageous embodiment, the present invention relates to a composition, for use as anti-adherence drug to treat infectious disease(s), comprising at least two glycans of structure VI-2, of the following particular formula XIV-2: 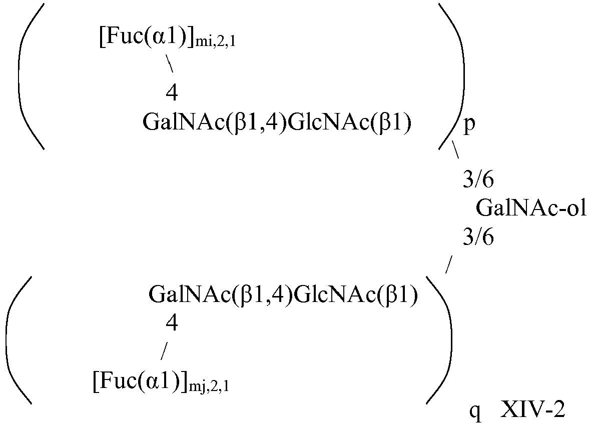 wherein p, q, m_{i,2,1} and m_{j,2,1} have the meaning indicated above.

In an advantageous embodiment, the present invention relates to a composition, for use as anti-adherence drug to treat infectious disease(s), comprising at least two glycans of structure XV or glycopeptides comprising independently of each other a motif of structure XV: 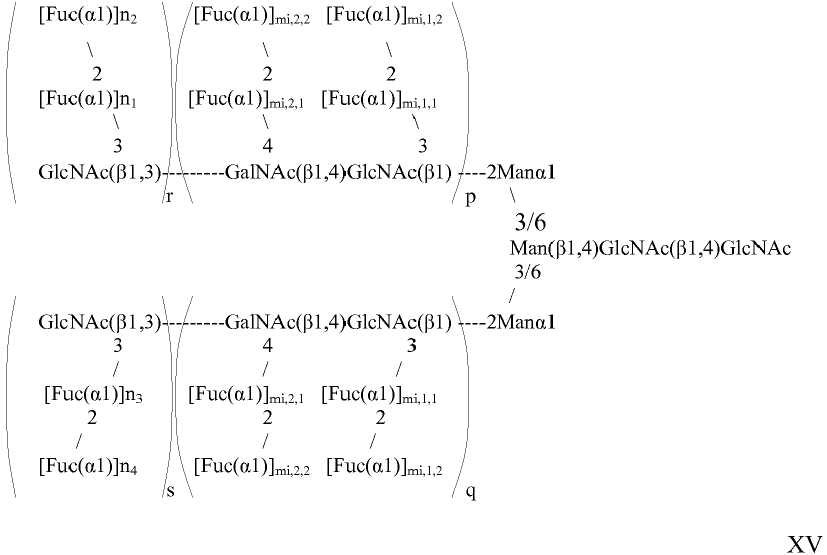 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1} ; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
   - p > 0 and there is at least one i such as at least one of m_{i,1,1} m_{i,1,2}; m_{i,2,1} and m_{i,2,2} is equal to 1, and/or
   - q > 0 and there is at least one j such as at least one of m_{j,1,1}; m_{j,1,2}; m_{j,2,1} and m_{j,2,2} is equal to 1, and/or
   - p > 0, r > 0 and there at least one of m_{i,1,1}; m_{i,1,2} mᵢ,₂,ᵢ m_{i,2,2}; n₁; n₂; n₃ and n₄ is equal to 1, and/or
   - q > 0, s > 0 and there at least one of m_{i,1,1}; m_{i,1,2}; m_{i,2,1}; m_{i,2,2}; n₁; n₂; n₃ and n₄ is equal to 1.

The abbreviation "Man" refers to mannose.

When p is equal to 1, the corresponding pattern is present one time, corresponding to i=1.

When p is equal to 2, the corresponding pattern is present p=2 times, the pattern corresponding to i=1 and the pattern corresponding to i=2 being linked through a β1,3 bond.

Similarly, when q is equal to 1, the corresponding pattern is present one time, corresponding to j=1.

When q is equal to 2, the corresponding pattern is present q=2 times, the pattern corresponding to j=1 and the pattern corresponding to j=2 being linked through a β1,3 bond.

Glycans of structure VII are N-glycans: they originate from glycopeptides wherein the glycan part(s) is (are) linked to the peptide by a covalent bond involving an atom of nitrogen.

In an advantageous embodiment, the present invention relates to a composition, for use as anti-adherence drug to treat infectious disease(s), comprising at least two glycans of structure XV or glycopeptides comprising independently of each other a motif of structure XV: 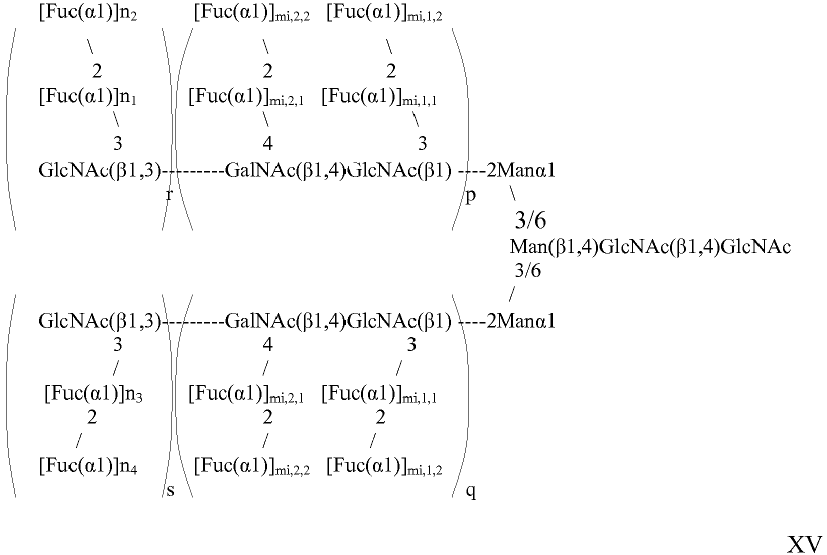 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2} ; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and m_{i,2,2} is equal to 1, and/or
   - q > 0 and there is at least one j such as at least one of m_{j,1,1}; m_{j,1,2}; m_{j,2,1} and m_{j,2,2} is equal to 1.

In an advantageous embodiment, the present invention relates to a composition, for use as anti-adherence drug to treat infectious disease(s), comprising at least two glycans of structure XV or glycopeptides comprising independently of each other a motif of structure XV, of particular structure XVI: 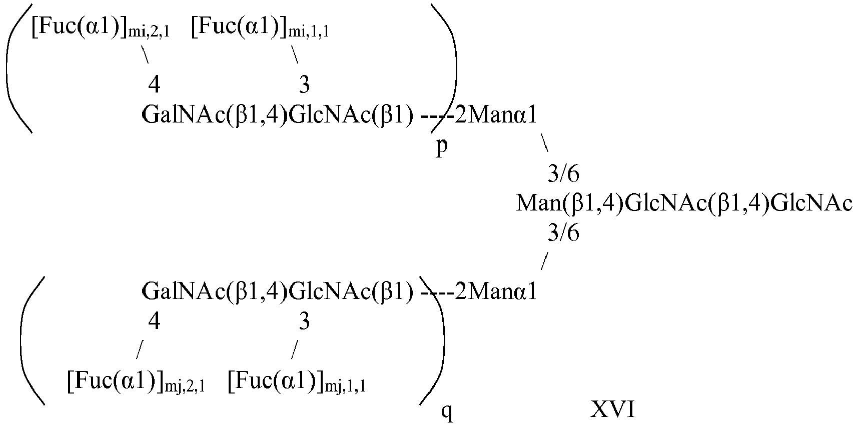 wherein p, q, m_{i,1,1}; m_{i,2,1} ; m_{j,1,1} and m_{j,2,1} have the meaning indicated above.

In an advantageous embodiment, the present invention relates to a composition, for use as anti-adherence drug to treat infectious disease(s), comprising at least two glycans of structure XV or glycopeptides comprising independently of each other a motif of structure XV of particular structure XVII: 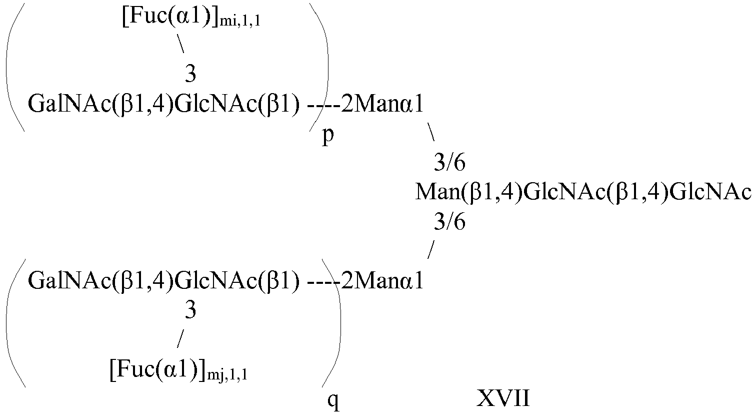 wherein p, q, m_{i,1,1} and m_{j,1,1} have the meaning indicated above.

In an advantageous embodiment, the present invention relates to a composition, for use as anti-adherence drug to treat infectious disease(s), comprising at least two glycans of structure XV or glycopeptides comprising independently of each other a motif of structure XV, of particular structure XVIII: 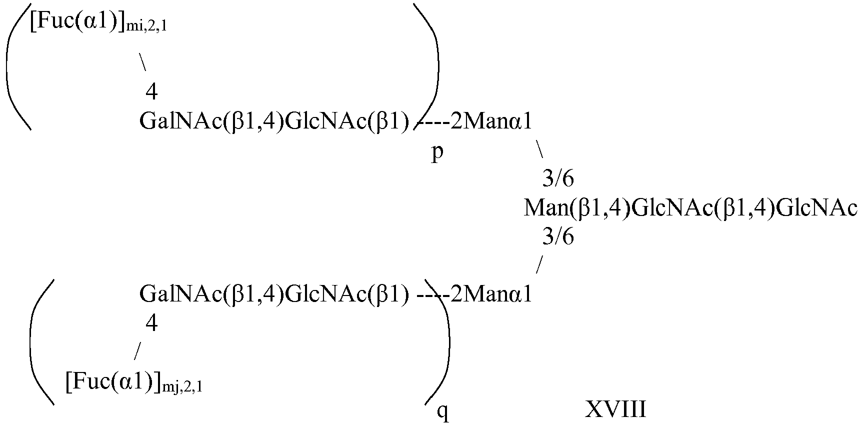 wherein p, q, m_{i,2,1} and m_{j,2,1} have the meaning indicated above.

In an advantageous embodiment, the present invention relates to a composition, for use as anti-adherence drug to treat infectious disease(s), wherein the total content of Fuc, GalNAc and GlcNAc are, in molar percentages, as follows:
Fuc: from 5 to 25, in particular from 10 to 25, more particularly from 18 to 25;
GalNac: from 5 to 15;
GlcNAc: from 30 to 50.

In an advantageous embodiment, the present invention relates to a composition, for use as anti-adherence drug to treat infectious disease(s), wherein said glycans and/or glycopeptides are isolated from *Halocynthia roretzi.*

In an advantageous embodiment, the present invention relates to a composition for use as anti-adherence drug to treat infectious disease(s) caused by bacteria of the species *Helicobacter pylori,* said diseases belonging to the group comprising:
- infection of the stomach,
- dyspepsia,
- gastritis,
- ulcers, in particular ulcers of the stomach or the duodenum.

In an advantageous embodiment, the present invention relates to a combination of a composition as described below and an antibiotic selected from the group comprising beta-lactamines, tetracyclines, macrolides, fluoroquinolones, rifamycins, and nitromidazoles for simultaneous, separated or sequential use in treatment of said diseases.

Examples of antibiotics are amoxicillin, clarithromycin, metronidazole and tetracycline.

In an advantageous embodiment, the present invention relates to a combination of a composition as described below and a drug reducing stomach acidity, in particular a proton pump inhibitor, for simultaneous, separated or sequential use in treatment of said diseases.

Examples of proton pump inhibitors are omeprazole, lansoprazole, rabeprazole, and pantoprazole.

In an advantageous embodiment, the present invention relates to a combination of a composition as described below, an antibiotic selected from the group comprising beta-lactams, aminoglycosides, tetracyclines, glycylcyclines, macrolides, azalides, ketolides, synergistins, lincosanides, fluoroquinolones, phenicols, rifamycins, sulfamides, trimethoprim, glycopeptides, oxazolidinones and lipopeptides, and a drug reducing stomach acidity, in particular a proton pump inhibitor, for simultaneous, separated or sequential use in treatment of said diseases.

In an advantageous embodiment, the present invention relates to a composition, for use as anti-adherence drug to treat infectious disease(s), comprising at least two glycopeptides comprising independently of each other a motif, the structure of which being chosen in the group comprising:

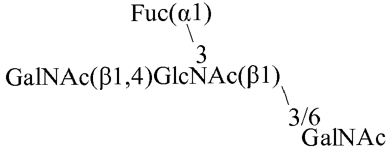

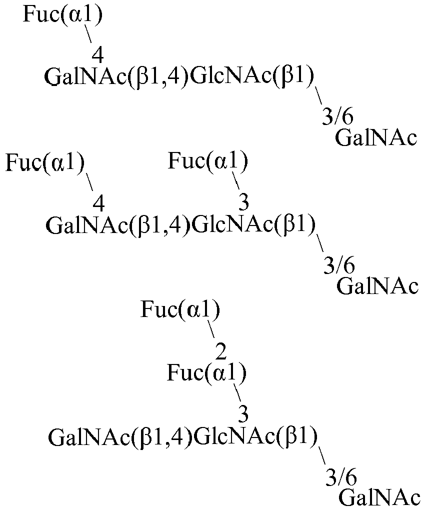

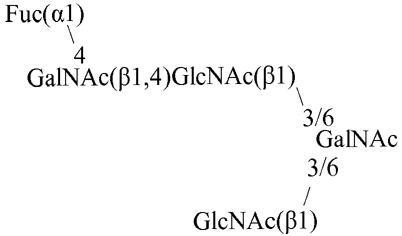

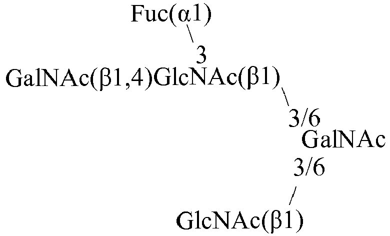

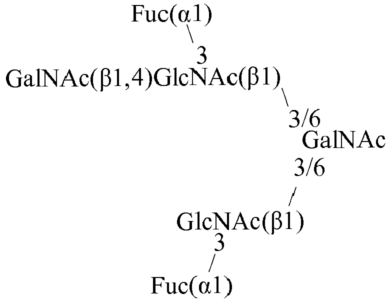

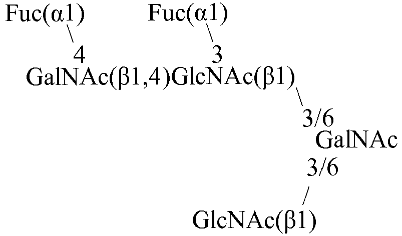

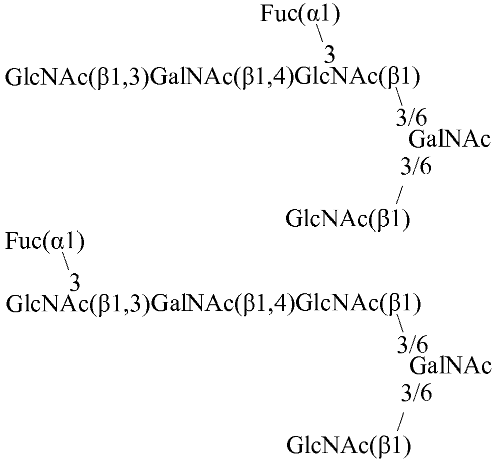

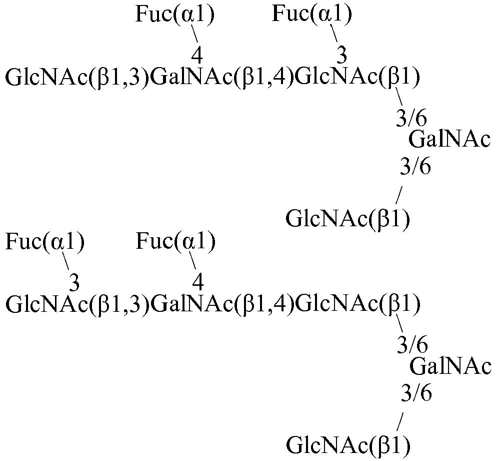

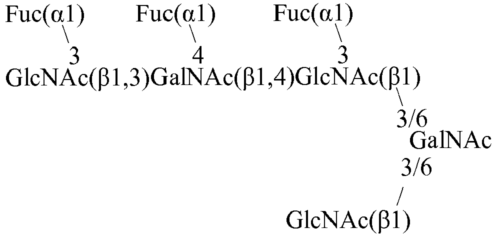

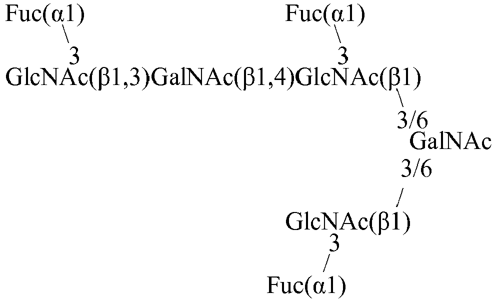

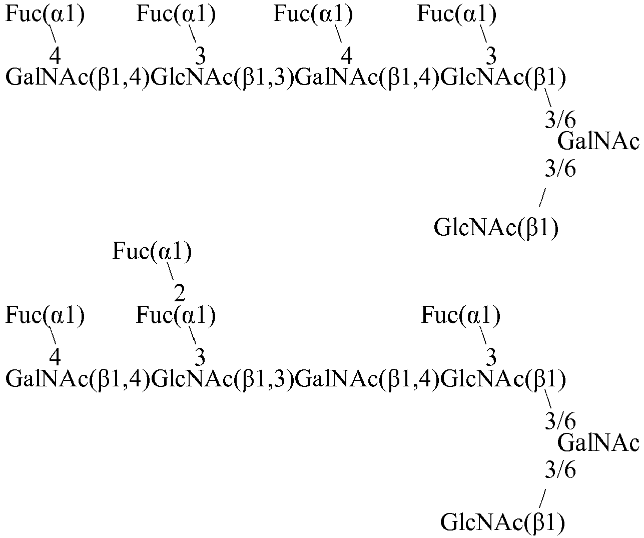

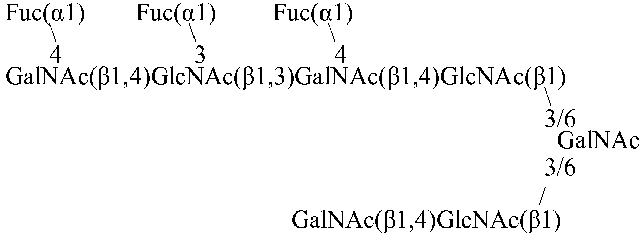

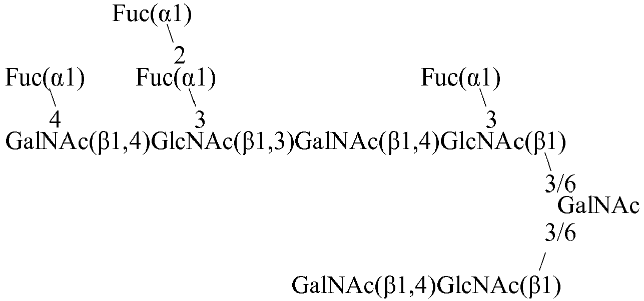

In an advantageous embodiment, the present invention relates to a composition, for use as anti-adherence drug to treat infectious disease(s), comprising at least two glycans, said glycans being chosen in the group comprising:

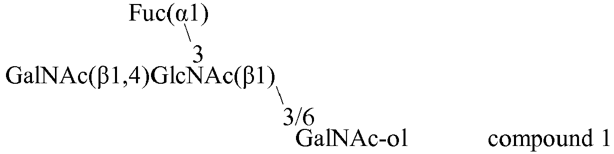

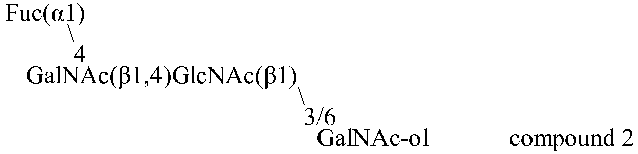

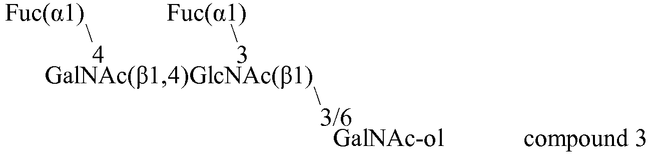

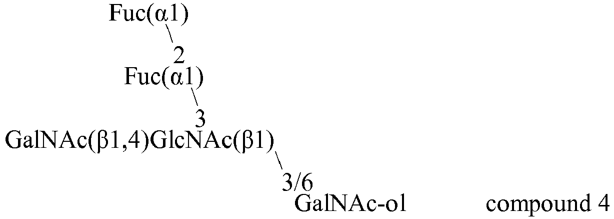

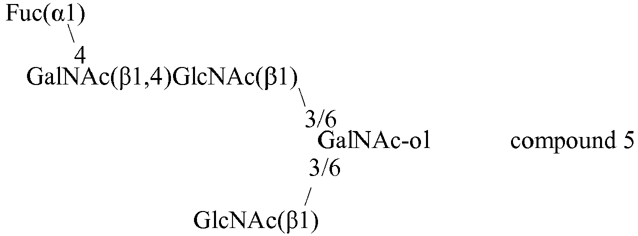

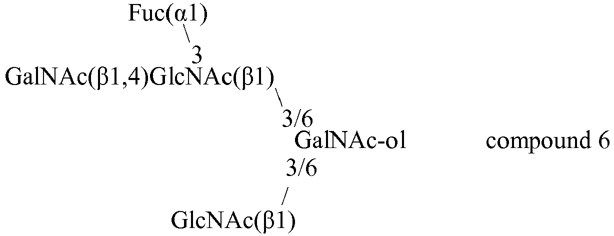

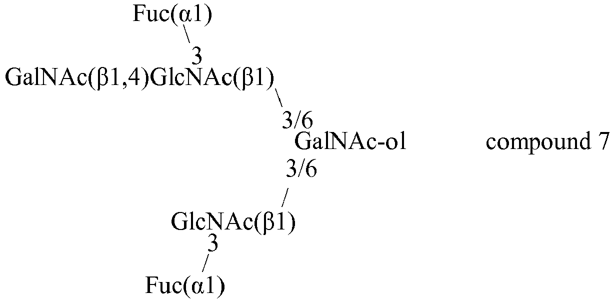

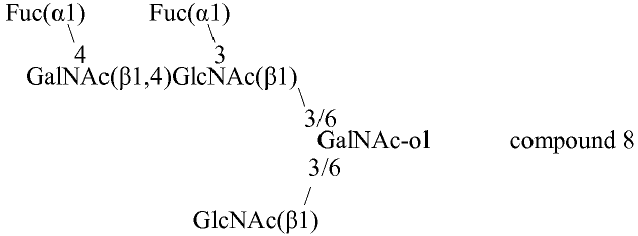

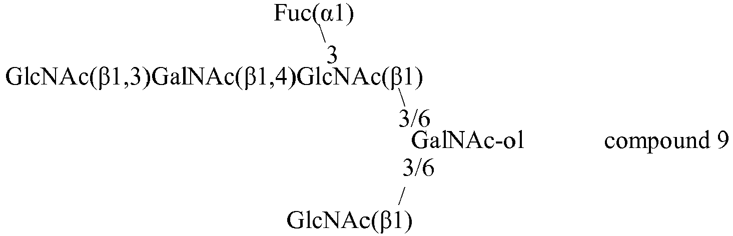

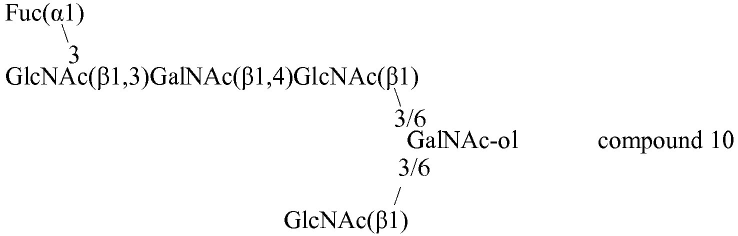

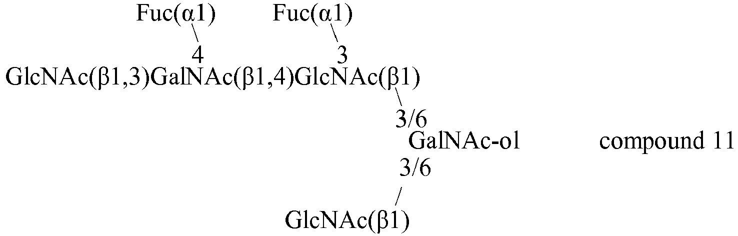

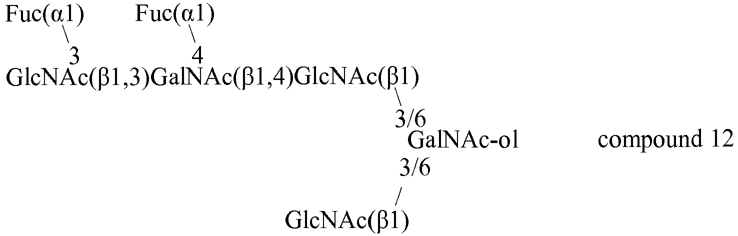

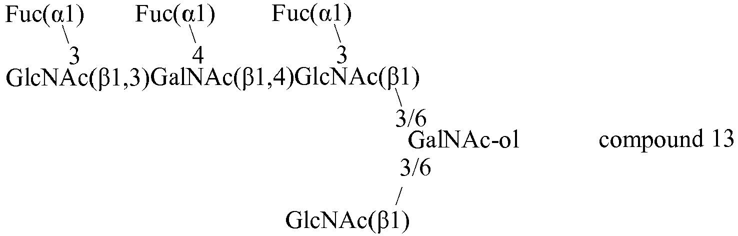

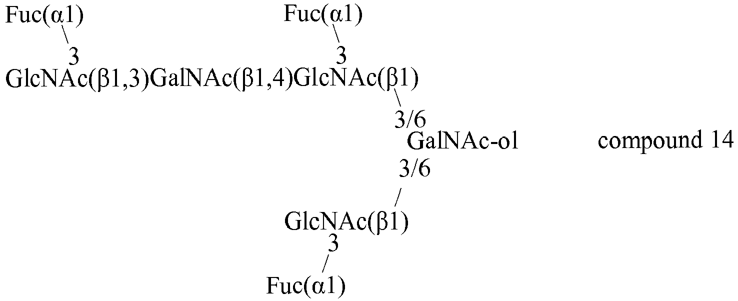

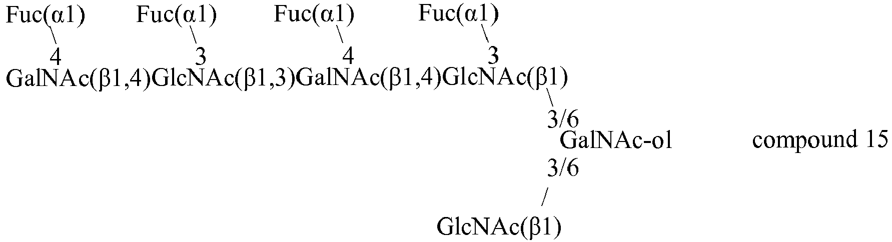

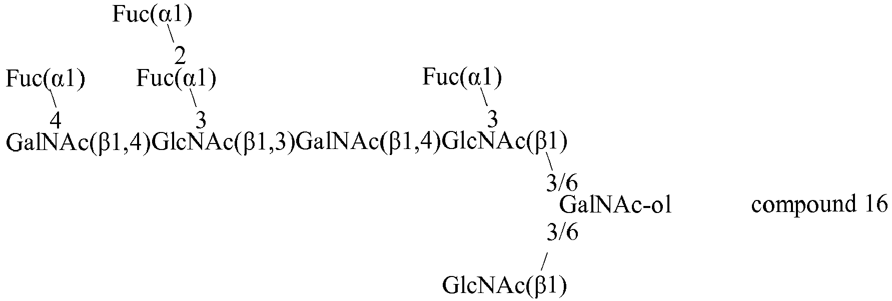

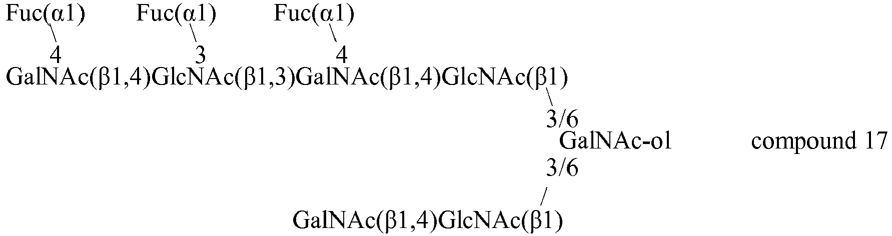

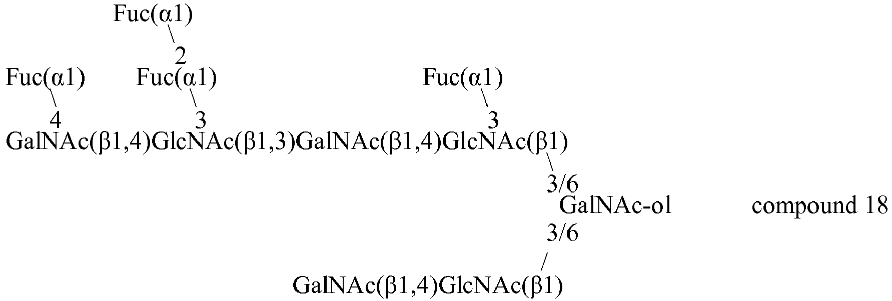

In an advantageous embodiment, the present invention relates to a composition, for use as anti-adherence drug to treat infectious disease(s), consisting in or comprising 2, 3, 4, 5 or all of the following compounds:

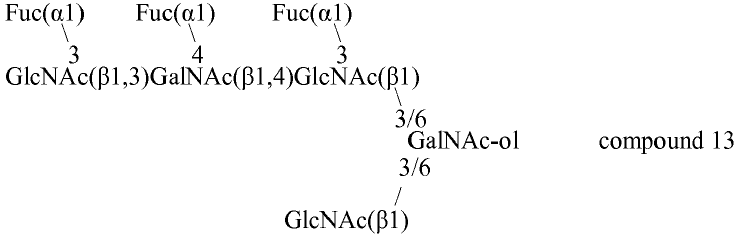

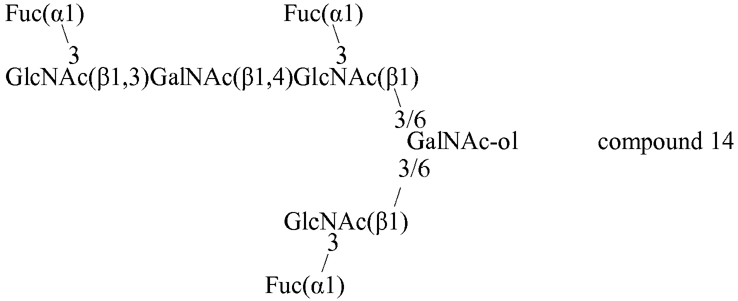

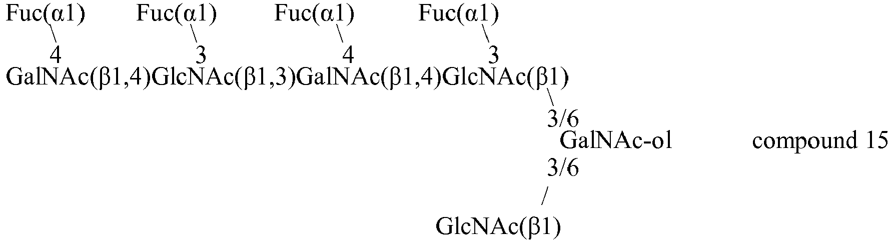

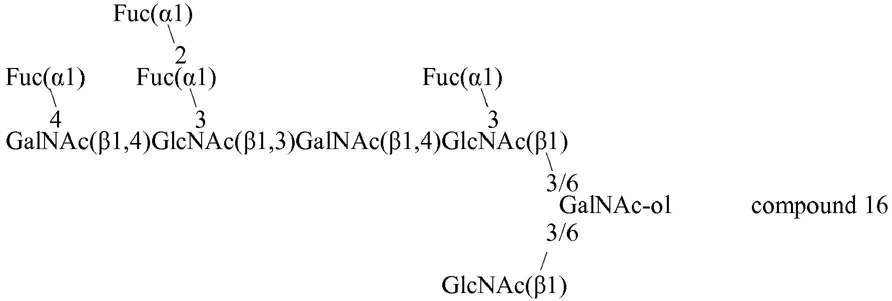

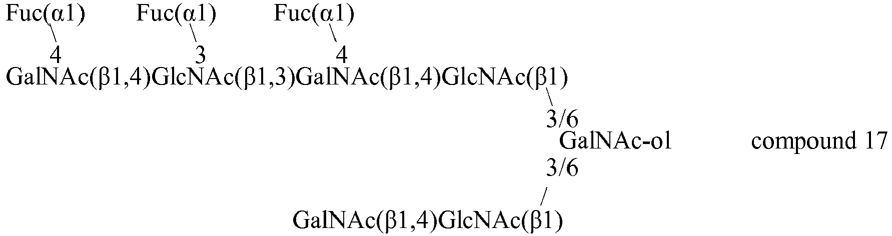

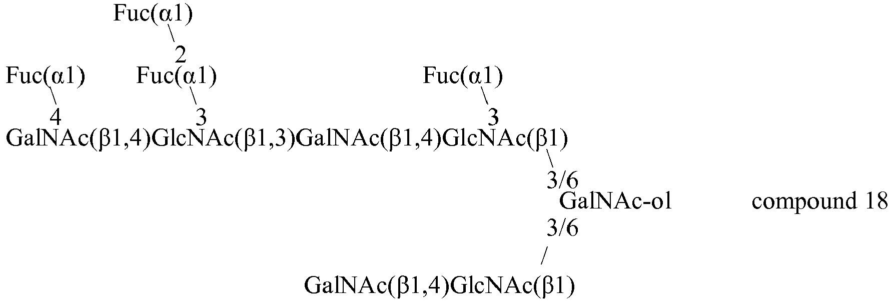

In an advantageous embodiment, the present invention relates to a composition, for use as anti-adherence drug to treat infectious disease(s), comprising at least two glycopeptides comprising independently of each other a motif chosen in the group comprising:

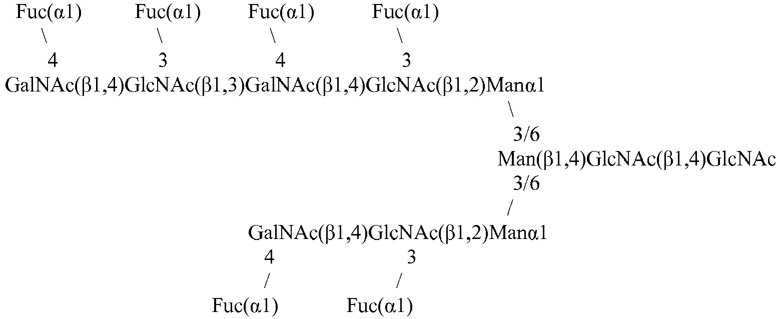

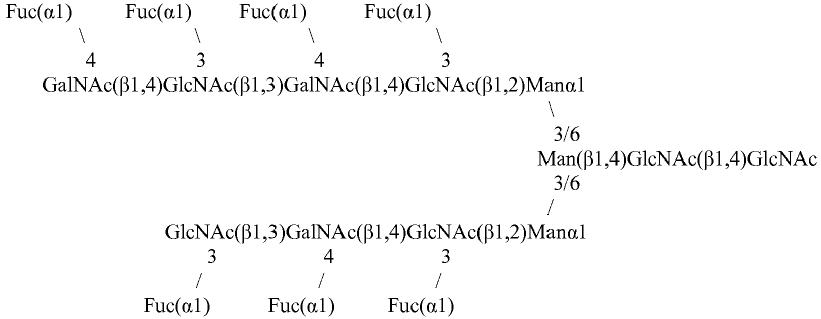

In an advantageous embodiment, the present invention relates to a composition, for use as anti-adherence drug to treat infectious disease(s), comprising at least two glycans of the following formulae:

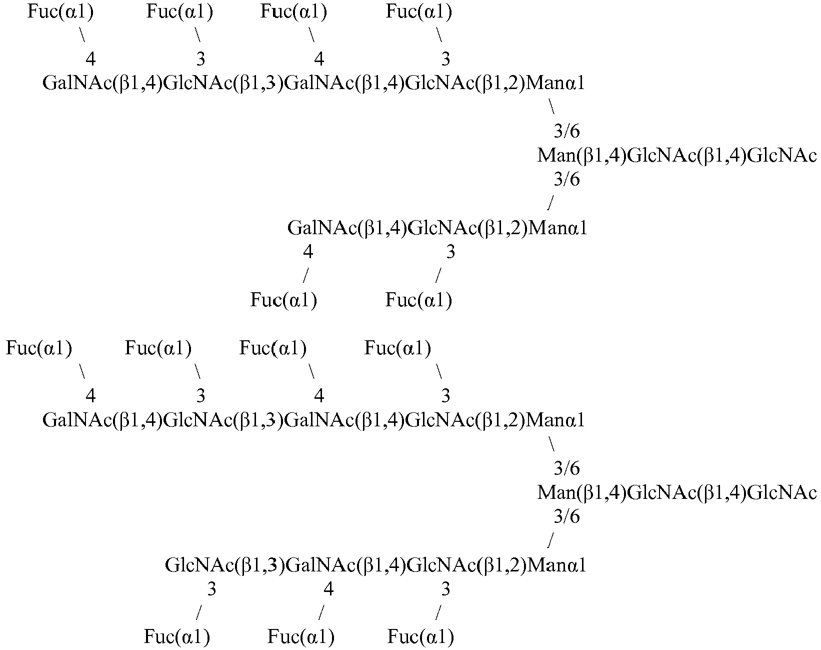

In another aspect, the present invention relates to a pharmaceutical composition comprising, as active substance, at least two glycans or glycopeptides comprising independently of each other at least one motif of structure I: 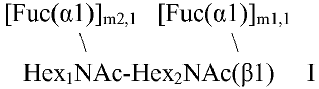 wherein:
m_{1,1} and m_{2,1} are integers equal to 0 or 1,
Hex₁ and Hex₂ represent Glc or Gal,
provided that:
   - at least one of m_{1,1} and m_{2,1} is equal to 1, and
   - when Hex₁ is Glc, Hex₂ is Gal,
   - and when Hex₁ is Gal, Hex₂ is Glc,
in association with a pharmaceutically acceptable vehicle.

In an advantageous embodiment, the present invention relates to a pharmaceutical composition comprising at least two glycans or glycopeptides comprising at least one motif of structure I-1: 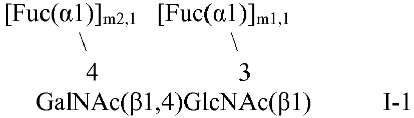 wherein m_{1,1} and m_{2,1} have the meaning indicated above.

In an advantageous embodiment, the present invention relates to a pharmaceutical composition comprising at least two glycans or glycopeptides comprising at least one motif of structure I-2: 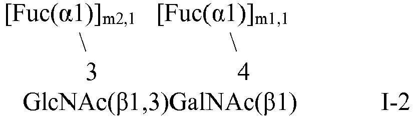 wherein m_{1,1} and m_{2,1} have the meaning indicated above.

In an advantageous embodiment, the present invention relates to a pharmaceutical composition comprising at least two glycans or glycopeptides comprising at least one motif of structure I, wherein m_{1,1} is equal to 1 and m_{2,1} is equal to 0, of particular formula II:

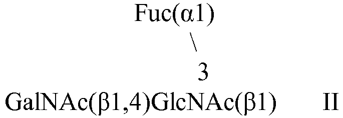

In an advantageous embodiment, the present invention relates to a pharmaceutical composition comprising at least two glycans or glycopeptides comprising at least one motif of structure I, wherein m_{1,1} is equal to 0 and m_{2,1} is equal to 1, of particular formula III:

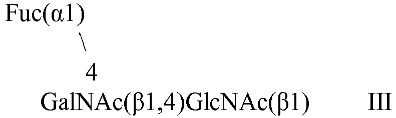

In an advantageous embodiment, the present invention relates to a pharmaceutical composition at least two glycans or glycopeptides comprising at least one motif of structure I, wherein m_{1,1} is equal to 0 and m_{2,1} is equal to 1, of particular formula III-2:

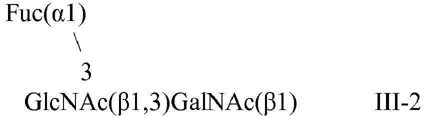

In an advantageous embodiment, the present invention relates to a pharmaceutical composition comprising at least two glycans or glycopeptides comprising at least one motif of structure I, wherein m_{1,1} and m_{2,1} are equal to 1, of particular formula IV:

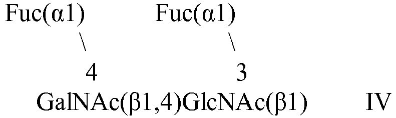

In an advantageous embodiment, the present invention relates to a pharmaceutical composition comprising at least two glycans or glycopeptides comprising at least one motif of structure I, wherein m_{1,1} and m_{2,1} are equal to 1, of particular formula IV-2:

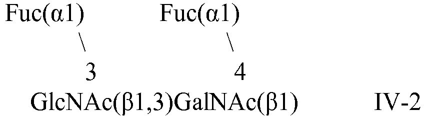

In an advantageous embodiment, the present invention relates to a pharmaceutical composition comprising at least two glycans or glycopeptides comprising at least one motif of structure V: 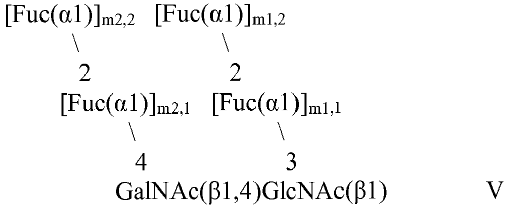 wherein:
m_{1,1}; m_{1,2}; m_{2,1} and m_{2,2} are integers equal to 0 or 1,
provided that one of m_{1,1}; m_{1,2}; m_{2,1} and m_{2,2} is equal to 1.

In an advantageous embodiment, the present invention relates to a pharmaceutical composition comprising at least two glycopeptides comprising independently of each other a motif of structure VI: 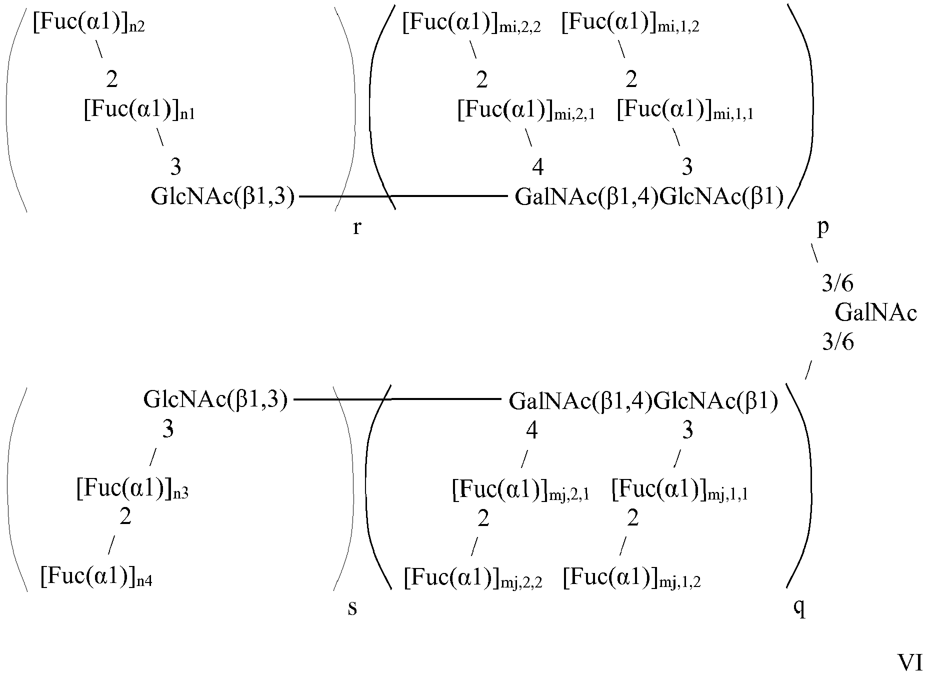 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2} ; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and m_{i,2,2} is equal to 1, and/or
   - q > 0 and there is at least one j such as at least one of m_{j,1,1}; m_{j,1,2}; m_{j,2,1} and m_{j,1,2} is equal to 1, and/or
   - p > 0, r > 0 and there at least one of m_{i,1,1}; m_{i,1,2}; m_{i,2,1}; m_{i,2,2}; n₁; n₂; n₃ and n₄ is equal to 1, and/or
   - q > 0, s > 0 and there at least one of m_{i,1,1}; m_{i,1,2}; m_{i,2,1}; m_{i,2,2}; n₁; n₂; n₃ and n₄ is equal to 1.

In an advantageous embodiment, the present invention relates to a pharmaceutical composition comprising at least two glycopeptides comprising independently of each other a motif of structure VI: 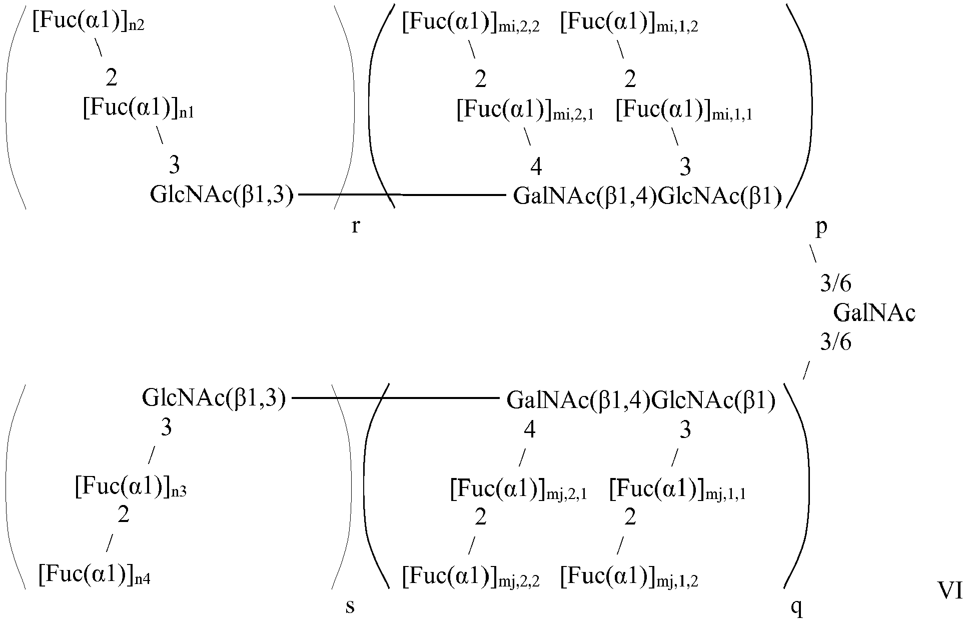 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,2}; m_{j,1,2}; m_{j,1,2}; m_{j,2,2}; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and m_{i,2,2} is equal to 1, and/or
   - q > 0 and there is at least one j such as at least one of m_{j,1,1}; m_{j,1,2}; m_{j,2,1} and m_{j,2,2} is equal to 1.

In an advantageous embodiment, the present invention relates to a pharmaceutical composition comprising at least two glycopeptides comprising independently of each other a motif of structure VII: 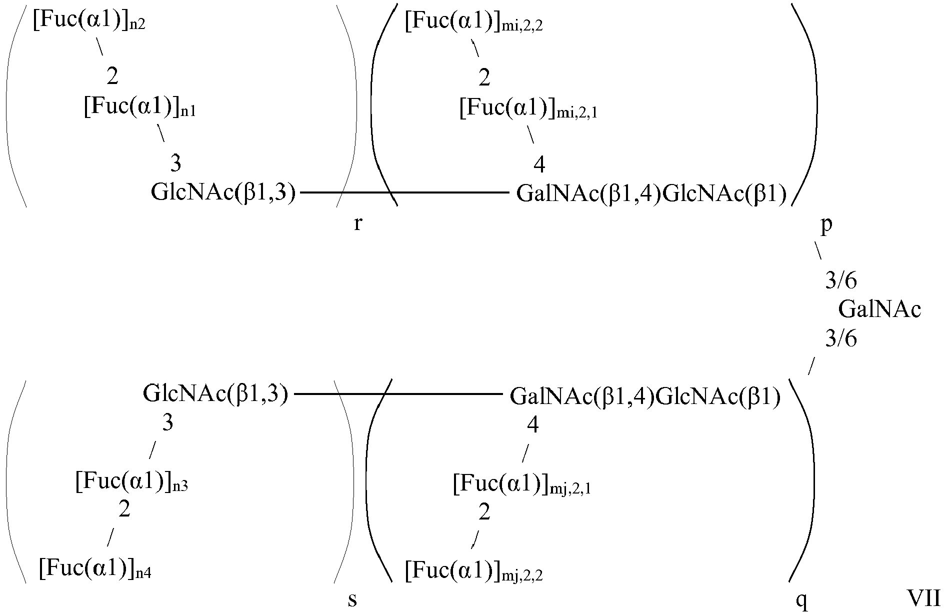 wherein r, p, q, s, m_{i,2,1}; m_{i,2,2}; m_{j,2,1}; m_{j,2,2}; n₁; n₂; n₃ and n₄ have the meaning indicated above.

In an advantageous embodiment, the present invention relates to a pharmaceutical composition comprising at least two glycopeptides comprising independently of each other a motif of structure VIII: 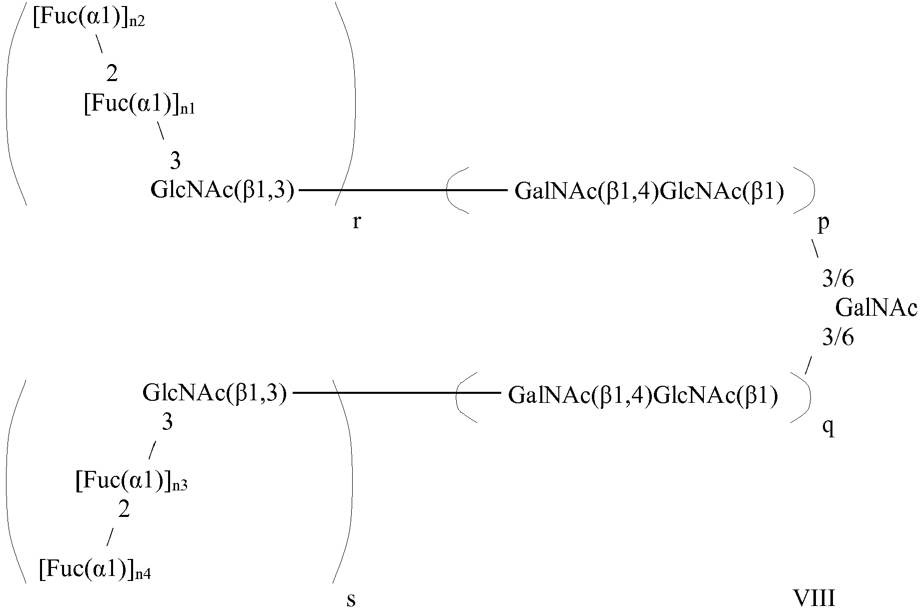 wherein r, p, s, q, n₁; n₂; n₃ and n₄ have the meaning indicated above.

In an advantageous embodiment, the present invention relates to a pharmaceutical composition comprising at least two glycopeptides comprising independently of each other a motif of structure IX: 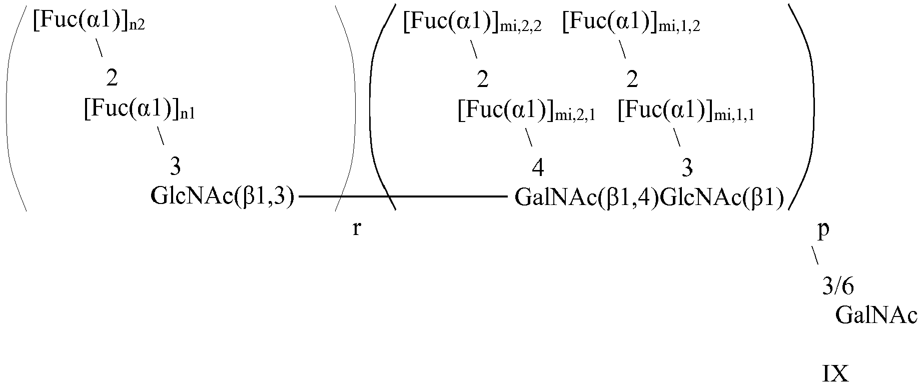 wherein r, p, m_{i,1,1}; m_{i,1,2}; m_{i,2,1}; m_{i,2,2}; n₁ and n₂ have the meaning indicated above.

In an advantageous embodiment, the present invention relates to a pharmaceutical composition comprising at least two glycopeptides comprising independently of each other a motif of structure X: 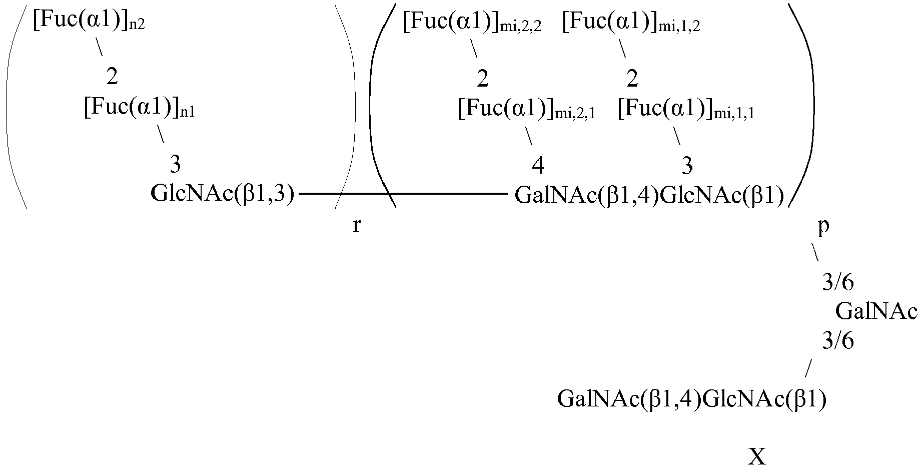 wherein r, p, m_{i,1,1}; m_{i,1,2}; m_{i,2,1}; m_{i,2,2}; n₁ and n₂ have the meaning indicated above.

In an advantageous embodiment, the present invention relates to a pharmaceutical composition comprising at least two glycopeptides comprising independently of each other a motif of structure XI: 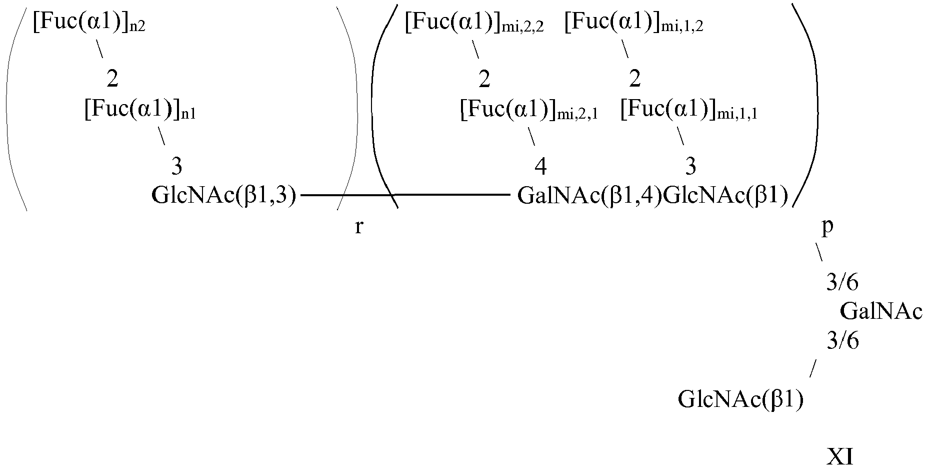 wherein r, p, m_{i,1,1}; m_{i,1,2}; m_{i,2,1}; m_{i,2,2}; n₁ and n₂ have the meaning indicated above.

In an advantageous embodiment, the present invention relates to a pharmaceutical composition comprising at least two glycans of structure VI-2: 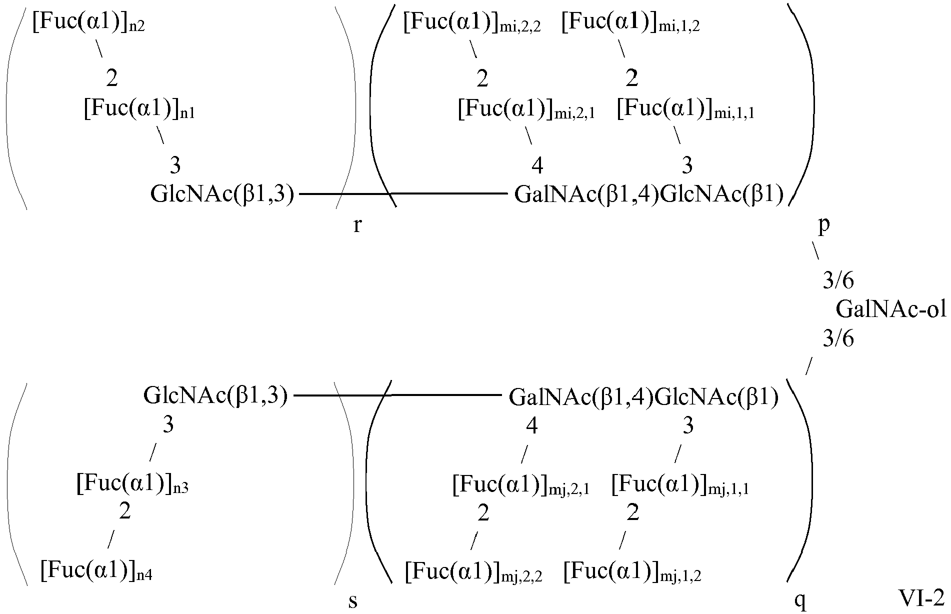 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and m_{i,2,2} is equal to 1, and/or
   - q > 0 and there is at least one j such as at least one of m_{j,1,1}; m_{j,1,2}; m_{j,2,1} and m_{j,2,2} is equal to 1, and/or
   - p > 0, r > 0 and there at least one of m_{i,1,1}; m_{i,1,2}; m_{i,2,1}; m_{i,2,2}; n₁; n₂; n₃ and n₄ is equal to 1, and/or
   - q > 0, s > 0 and there at least one of m_{i,1,1}; m_{i,1,2}; m_{i,2,1}; m_{i,2,2}; n₁; n₂; n₃ and n₄ is equal to 1.

In an advantageous embodiment, the present invention relates to a pharmaceutical composition comprising at least two glycans of structure VI-2: 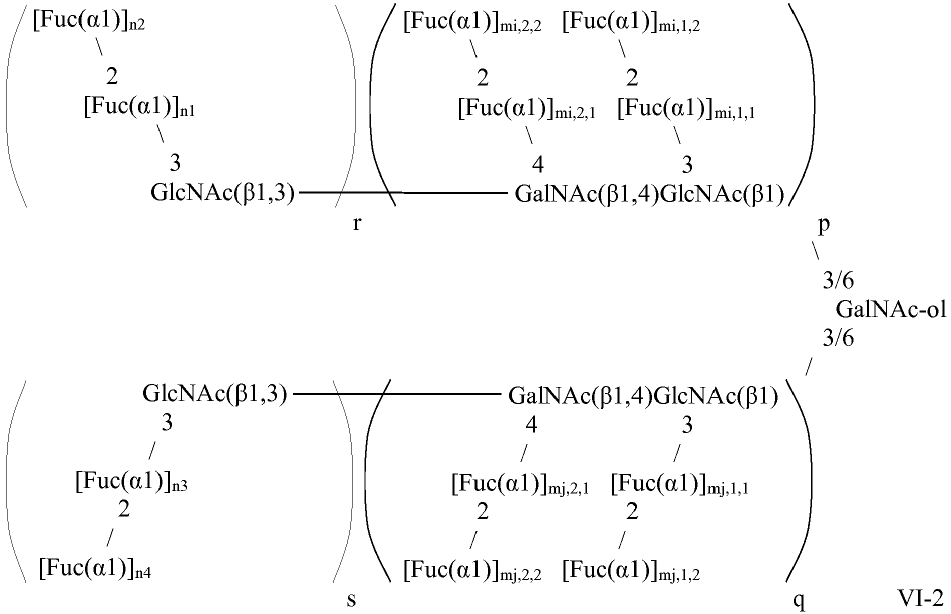 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,1,2}; m_{j,2,2}; n₁; n₂; n₃ and n₄ are integers equal to 0 or 1,
provided that p + q > 0, and:
   - p > 0 and there is at least one i such as at least one of m_{i,1,1}; m¡,ₗ,₂; m_{i,2,1} and m_{i,2,2} is equal to 1, and/or
   - q > 0 and there is at least one j such as at least one of m_{j,1,1}; m_{j,1,2}; m_{j,2,1} and m_{j,2,2} is equal to 1.

In an advantageous embodiment, the present invention relates to a pharmaceutical composition comprising at least two glycans of structure VII-2: 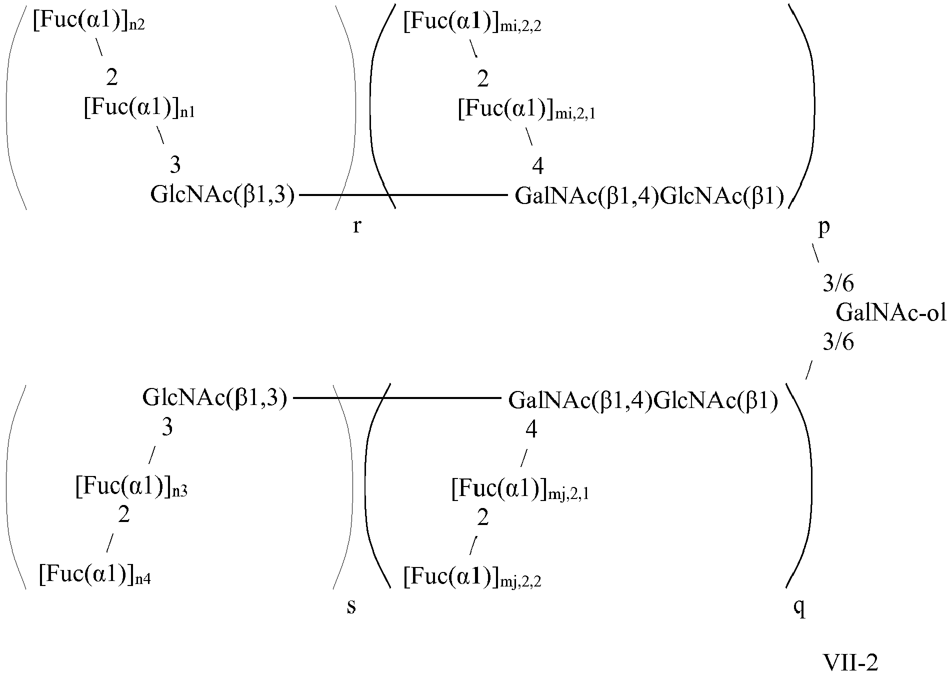 wherein r, p, s, q, m_{i,2,1}; m_{i,2,2}; m_{j,2,1}; m_{j,2,2} ; n₁; n₂; n₃ and n₄ have the meaning indicated above.

In an advantageous embodiment, the present invention relates to a pharmaceutical composition comprising at least two glycans of structure VIII-2: 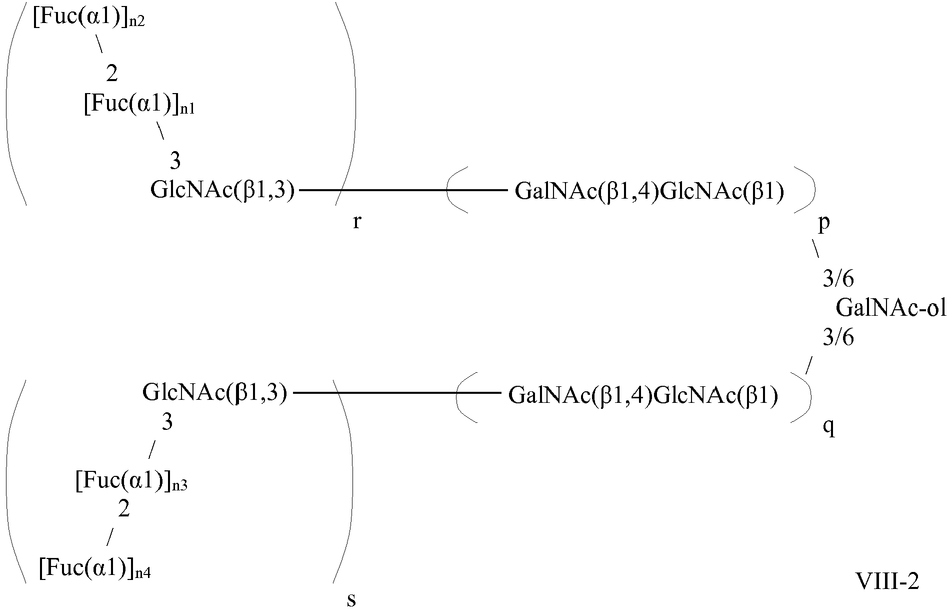 wherein r, p, s, q, n₁; n₂; n₃ and n₄ have the meaning indicated above.

In an advantageous embodiment, the present invention relates to a pharmaceutical composition comprising at least two glycans of structure IX-2: 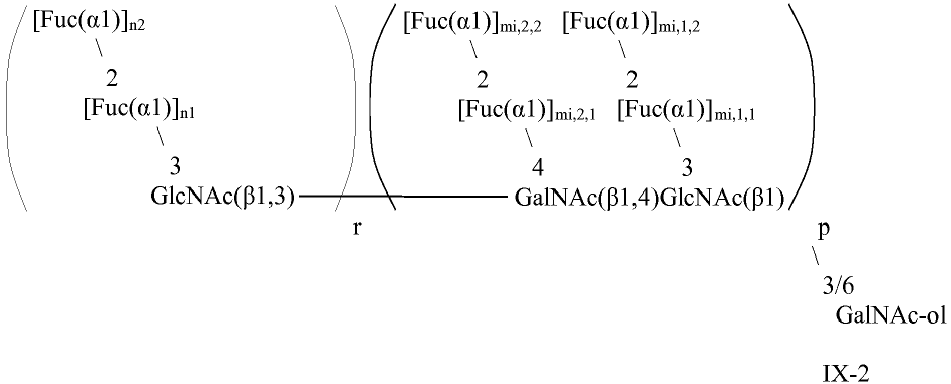 wherein r, p, m_{i,1,1}; m_{i,1,2}; m_{i,2,1} ; m_{i,2,2}; n₁ and n₂ have the meaning indicated above.

In an advantageous embodiment, the present invention relates to a pharmaceutical composition comprising at least two glycans of structure X-2: 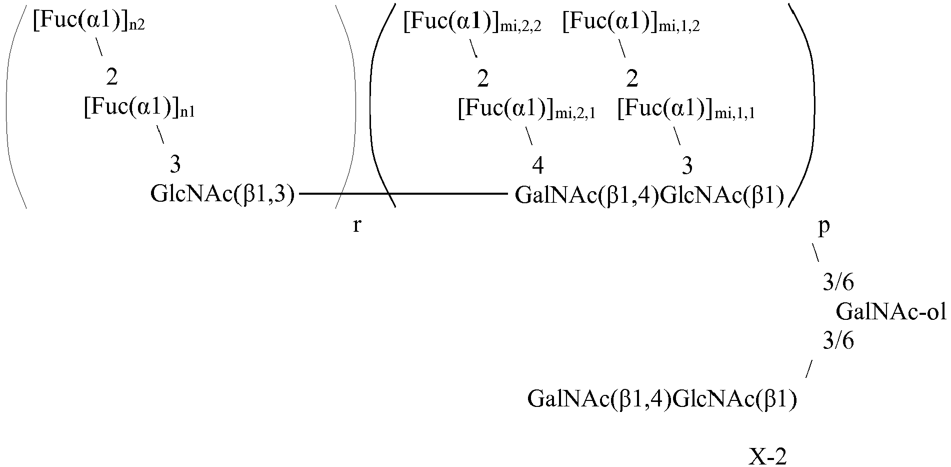 wherein r, p, m_{i,1,1}; m_{i,1,2}; m_{i,2,1}; m_{i,2,2}; n₁ and n₂ have the meaning indicated above.

In an advantageous embodiment, the present invention relates to a pharmaceutical composition comprising at least two glycans of structure XI-2: 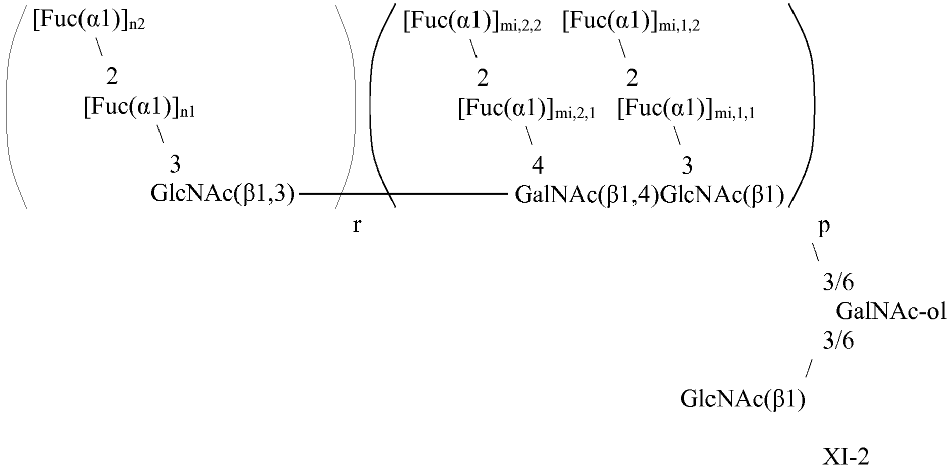 wherein r, p, m_{i,1,1}; m_{i,1,2}; m_{i,2,1}; m_{i,2,2}; n₁ and n₂ have the meaning indicated above.

In an advantageous embodiment, the present invention relates to a pharmaceutical composition comprising at least two glycopeptides comprising independently of each other a motif of structure VI, of the following particular formula XII: 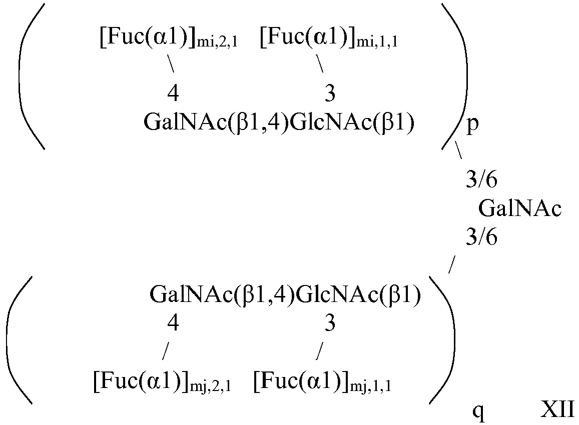 wherein p, q, m_{i,1,1}; m_{i,2,1}; m_{j,1,1}, and m_{j,2,1} have the meaning indicated above.

In an advantageous embodiment, the present invention relates to a pharmaceutical composition comprising at least two glycans of structure VI-2, of the following particular formula XII-2: 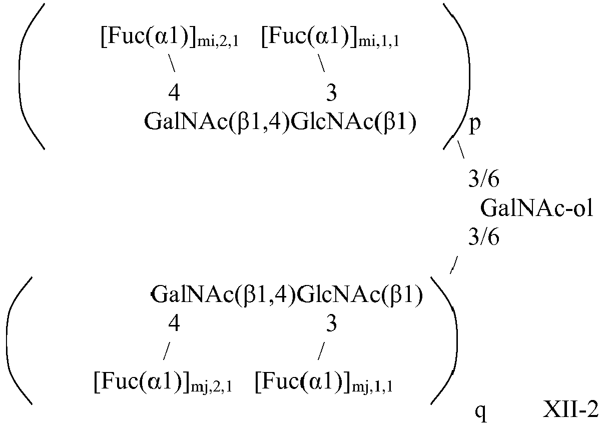 wherein p, q, m_{i,1,1}; m_{i,2,1}; m_{j,1,1} and m_{j,2,1} have the meaning indicated above.

In an advantageous embodiment, the present invention relates to a pharmaceutical composition comprising at least two glycopeptides comprising independently of each other a motif of structure VI, of the following particular formula XIII: 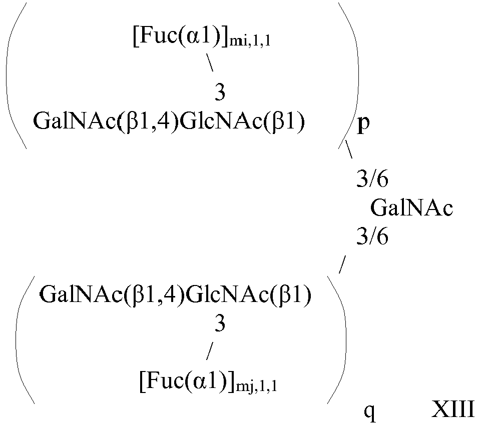 wherein p, q, m_{i,1,1} and m_{j,1,1} have the meaning indicated above.

In an advantageous embodiment, the present invention relates to a pharmaceutical composition comprising at least two glycans of structure VI-2, of the following particular formula XIII-2: 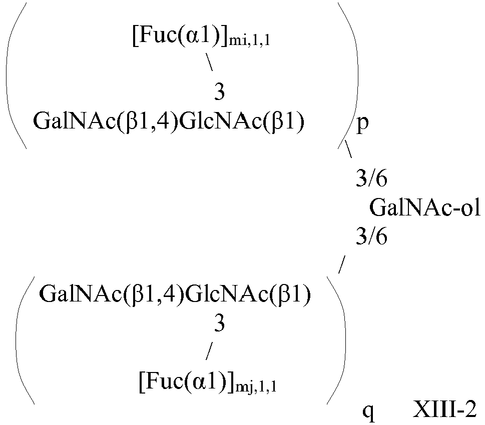 wherein p, q, m_{i,1,1} and m_{j,1,1} have the meaning indicated above.

In an advantageous embodiment, the present invention relates to a pharmaceutical composition comprising at least two glycopeptides comprising independently of each other a motif of structure VI, of the following particular formula XIV: 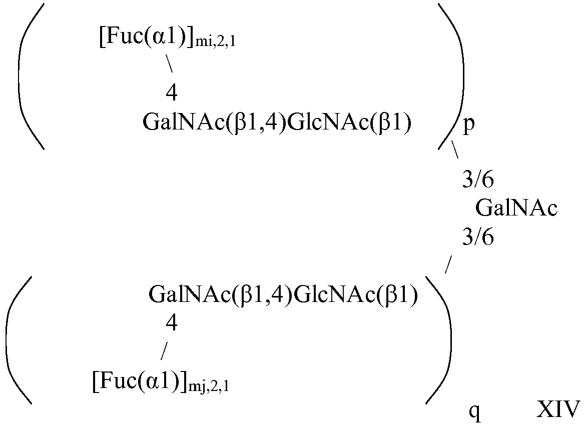 wherein p, q, m_{i,2,1} and m_{j,2,1}, have the meaning indicated above.

In an advantageous embodiment, the present invention relates to a pharmaceutical composition comprising at least two glycans of structure VI-2, of the following particular formula XIV-2: 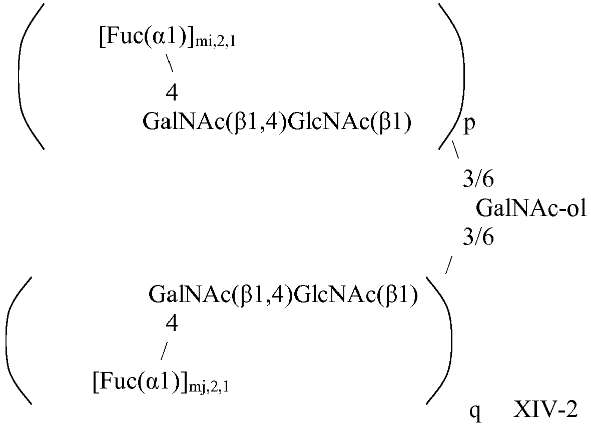 wherein p, q, m_{i,2,1} and m_{j,2,1}, have the meaning indicated above.

In an advantageous embodiment, the present invention relates to a pharmaceutical composition comprising at least two glycans of structure VII or glycopeptides comprising independently of each other a motif of structure XV: 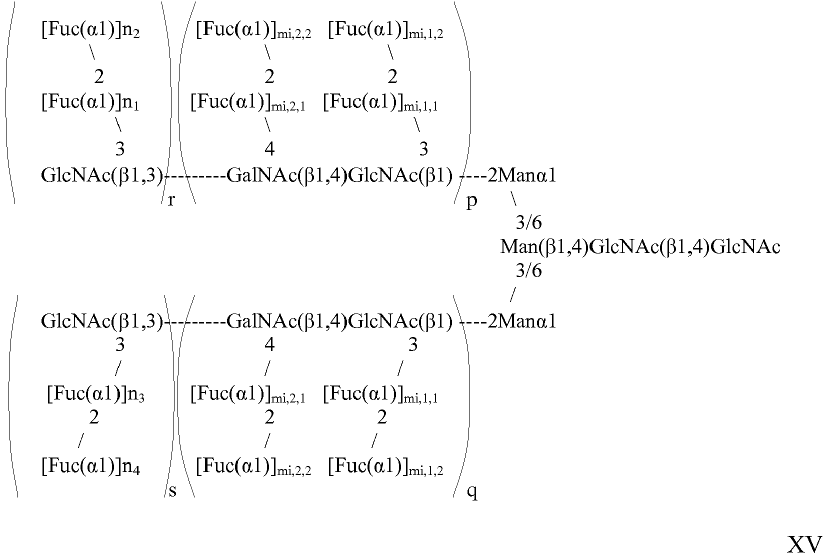 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and m_{i,2,2} is equal to 1, and/or
   - q > 0 and there is at least one j such as at least one of m_{j,1,1}; m_{j,1,2}; m_{j,2,1} and m_{j,2,2} is equal to 1, and/or
   - p > 0, r > 0 and there at least one of m_{i,1,1}; m_{i,1,2}; m_{i,2,1}; m_{i,2,2}; n₁; n₂; n₃ and n₄ is equal to 1, and/or
   - q > 0, s > 0 and there at least one of m_{i,1,1}; m_{i,1,2}; m_{i,2,1}; m_{i,2,2}; n₁; n₂; n₃ and n₄ is equal to 1.

In an advantageous embodiment, the present invention relates to a pharmaceutical composition comprising at least two glycans of structure XV or glycopeptides comprising independently of each other a motif of structure XV, of particular structure XVI: 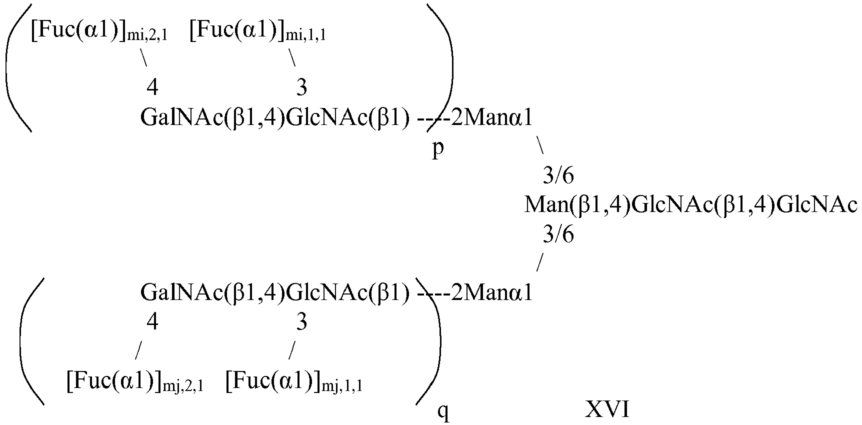 wherein p, q, m_{i,1,1}; m_{i,2,1}; m_{j,1,1} and m_{j,2,1} have the meaning indicated above.

In an advantageous embodiment, the present invention relates to a pharmaceutical composition comprising at least two glycans of structure XV or glycopeptides comprising independently of each other a motif of structure XV, of particular structure XVII: 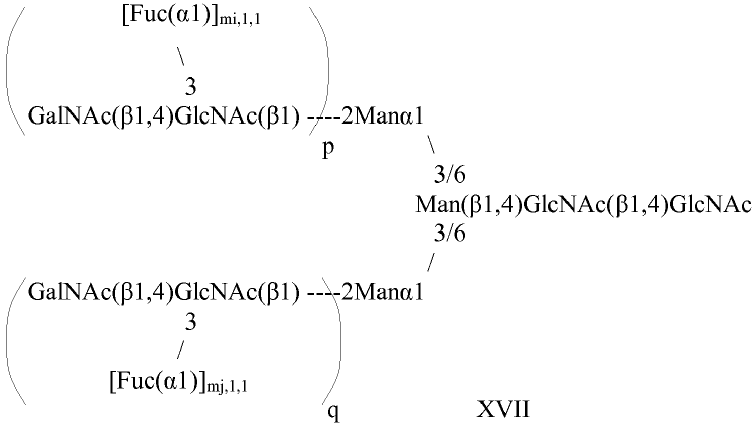 wherein p, q, m_{i,1,1} and m_{j,1,1} have the meaning indicated above.

In an advantageous embodiment, the present invention relates to a pharmaceutical composition comprising at least two glycans of structure XV or glycopeptides comprising independently of each other a motif of structure XV, of particular structure XVIII: 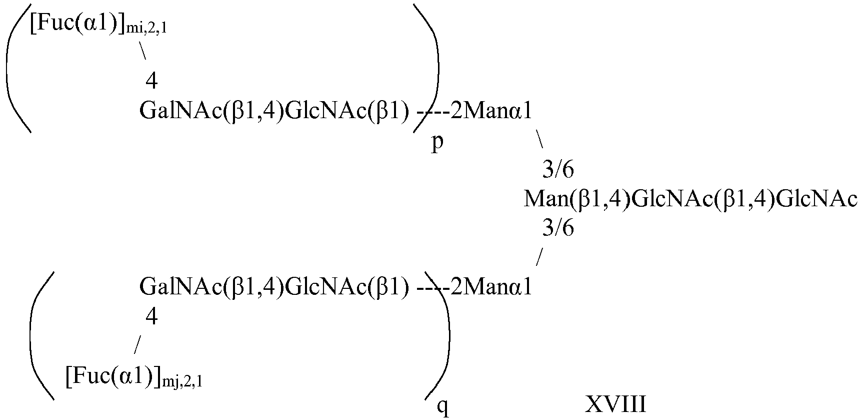 wherein p, q, m_{i,2,1} and m_{j,2,1} have the meaning indicated above.

In an advantageous embodiment, the present invention relates to a pharmaceutical composition wherein the total content of Fuc, GalNAc and GlcNAc in aforesaid active substance are, in molar percentages, as follows:
Fuc: from 5 to 25, in particular from 10 to 25, more particularly from 18 to 25;
GalNac: from 5 to 15;
GlcNAc: from 30 to 50.

In an advantageous embodiment, the present invention relates to a pharmaceutical composition wherein said glycans are isolated from *Halocynthia roretzi.*

The expression "pharmaceutically acceptable vehicle" denotes in particular cellulose, starch, benzyl alcohol, polyethylene glycol, gelatin, lactose, polysorbate, magnesium or calcium stearate, xanthan gum, guar, alginate, colloidal silica.

The compositions according to the invention can be used by oral, parenteral, topic, or rectal route or in aerosols.

As solid compositions for oral administration, tablets, pills, gelatin capsules, powders or granules can be used. In these compositions, the active ingredient according to the invention is mixed with one or more inert diluents or adjuvants, such as saccharose, lactose or starch. These compositions can comprise substances other than the diluents, for example a lubricant such as magnesium stearate or a coating intended for controlled release.

As liquid compositions for oral administration, pharmaceutically acceptable solutions, suspensions, emulsions, syrups and elixirs containing inert diluents such as water or paraffin oil can be used. These compositions can also comprise substances other than the diluents, for example wetting products, sweeteners or flavourings.

The compositions for parenteral administration can be sterile solutions or emulsions. As solvent or vehicle, water, propylene glycol, a polyethylene glycol, vegetable oils, in particular olive oil, injectable organic esters, for example ethyl oleate can be used. These compositions can also contain adjuvants, in particular wetting agents, isotoning agents, emulsifiers, dispersants and stabilizers.

The sterilization can be carried out in several ways, for example using a bacteriological filter, by irradiation or by heating. They can also be prepared in the form of sterile solid compositions which can be dissolved at the moment of use in sterile water or any other injectable sterile medium.

The compositions for topical administration can be for example creams, ointments, lotions or aerosols.

The compositions for rectal administration are suppositories or rectal capsules, which, in addition to the active ingredient, contain excipients such as cocoa butter, semi-synthetic glycerides or polyethylene glycols.

The compositions can also be aerosols.

For use in the form of liquid aerosols, the compositions can be stable sterile solutions or solid compositions dissolved at the moment of use in pyrogen-free sterile water, in serum or any other pharmaceutically acceptable vehicle. For use in the form of dry aerosols intended to be directly inhaled, the active ingredient is finely divided and combined with a diluent or hydrosoluble solid vehicle, for example dextran, mannitol or lactose.

In an advantageous embodiment, the present invention relates to a pharmaceutical composition administrable by oral route at a dose comprised from about 10 mg/kg to about 200 mg/kg of body weight.

In an advantageous embodiment, the present invention relates to a pharmaceutical composition defined above, under a form liable to be administrable by oral route, under the form of a unit dose comprised from 100 mg to 1,500 mg, in particular from 100 mg to 1,000 mg, in particular from 100 to 500 mg.

Said pharmaceutical composition can be administered 1 to 4 times per day, preferably 2 or 3 times per day.

In an advantageous embodiment, the present invention relates to a pharmaceutical composition administrable by intravenous route at a dose comprised from about 5 µg/kg to about 50 mg/kg.

In an advantageous embodiment, the present invention relates to a pharmaceutical composition defined above, under a form liable to be administrable by intraveinous, under the form of a unit dose comprised from 0.1 mg to 1000 mg, in particular from 10 mg to 1,000 mg, in particular from 10 to 500 mg, in particular from 10 to 100 mg.

Said pharmaceutical composition can be administered 1 to 4 times per day, preferably 2 or 3 times per day.

In an advantageous embodiment, the present invention relates to a pharmaceutical composition comprising at least two glycopeptides comprising independently of each other a motif, the structure of which being chosen in the group comprising:

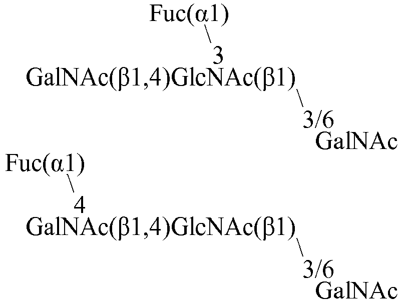

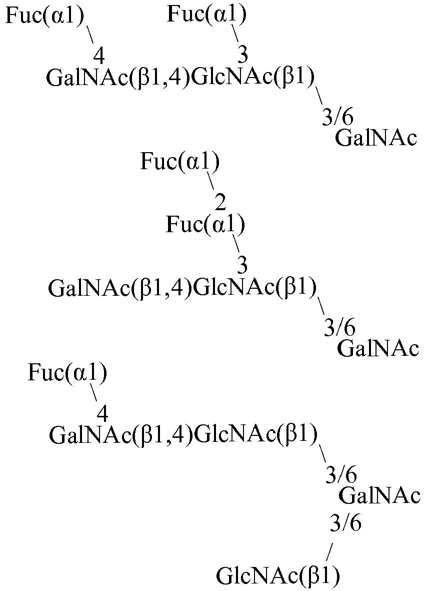

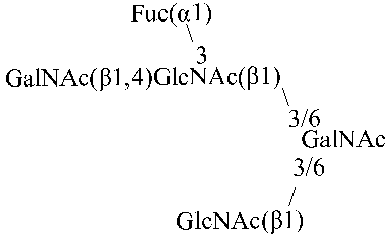

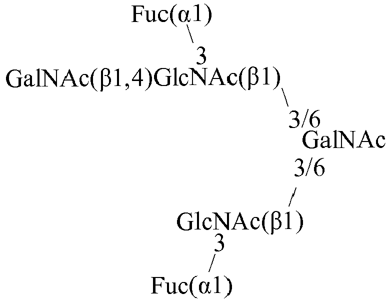

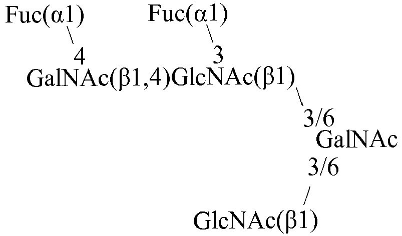

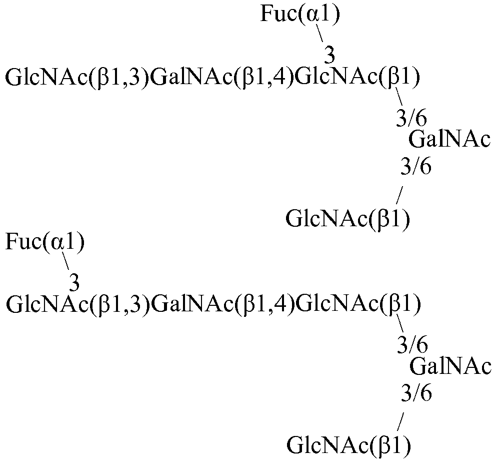

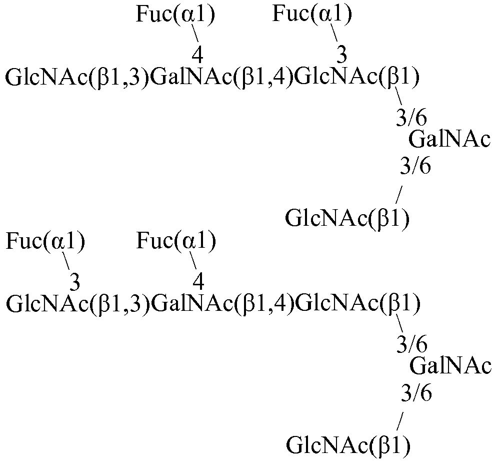

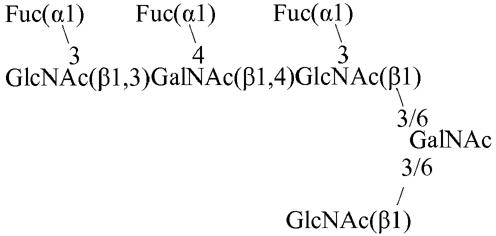

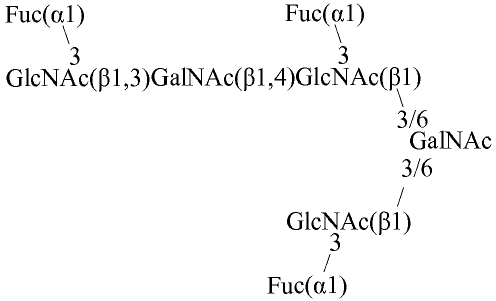

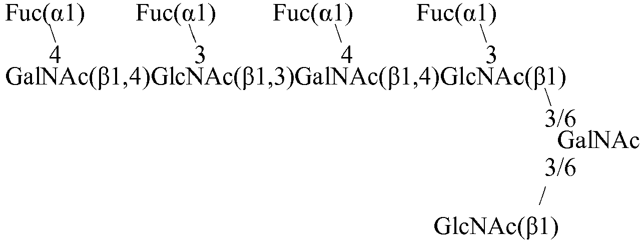

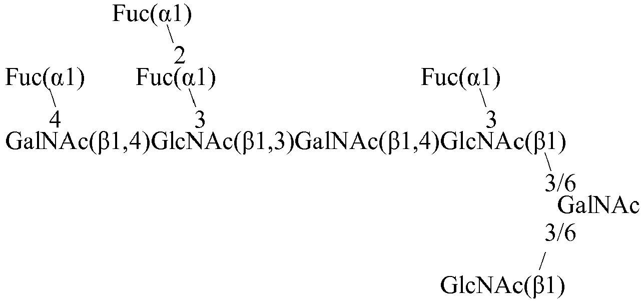

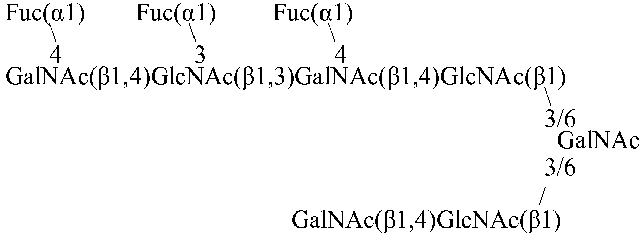

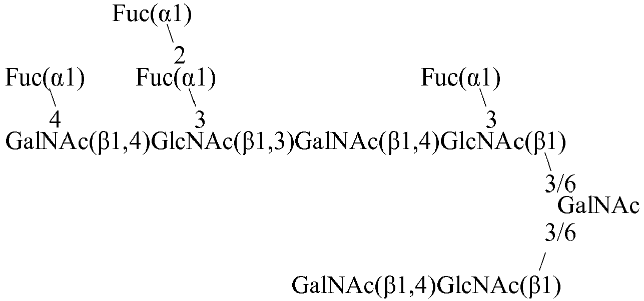

In an advantageous embodiment, the present invention relates to a pharmaceutical composition comprising at least two glycans, said glycans being chosen in the group comprising:

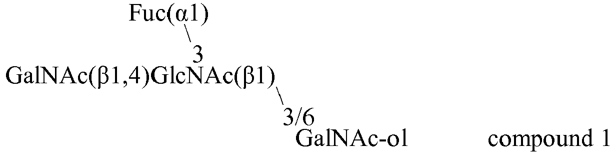

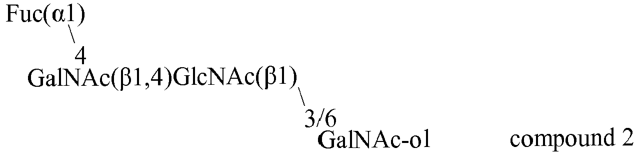

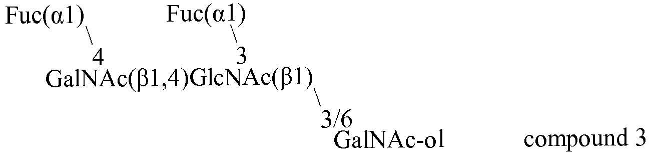

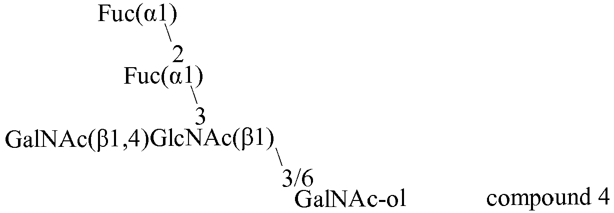

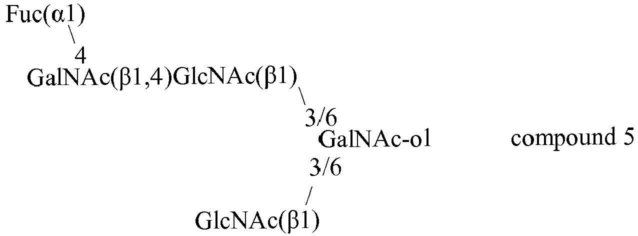

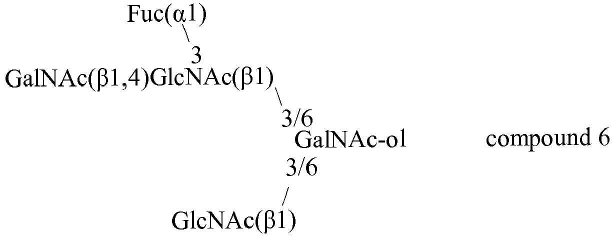

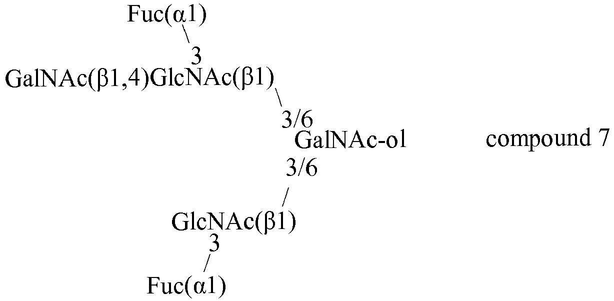

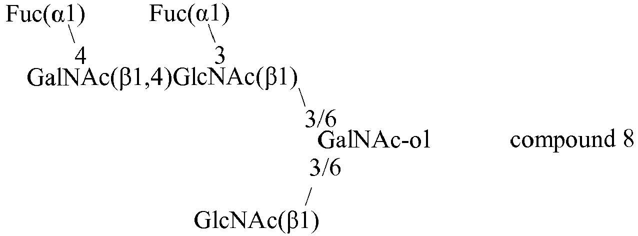

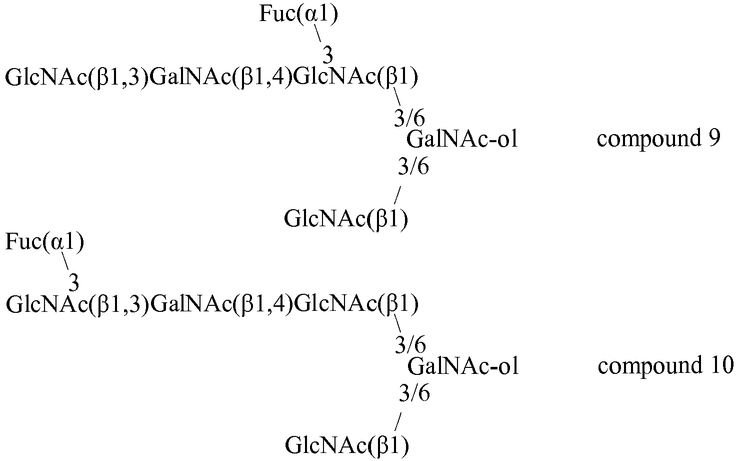

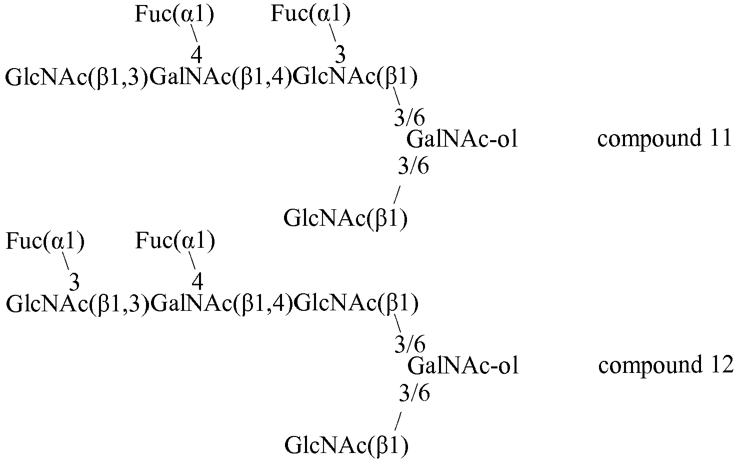

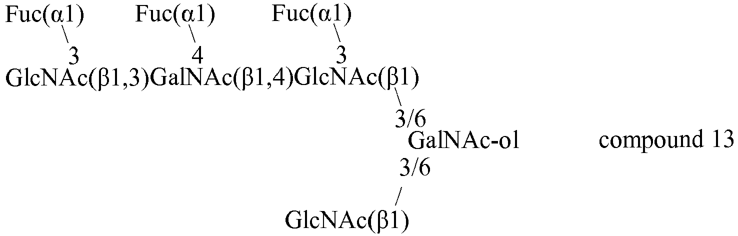

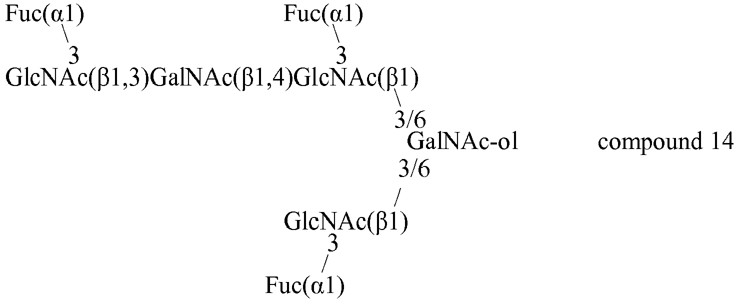

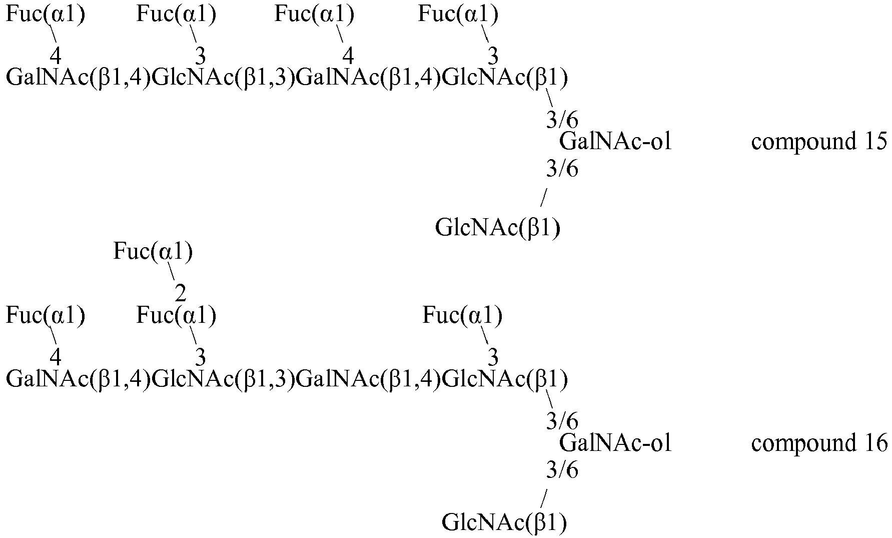

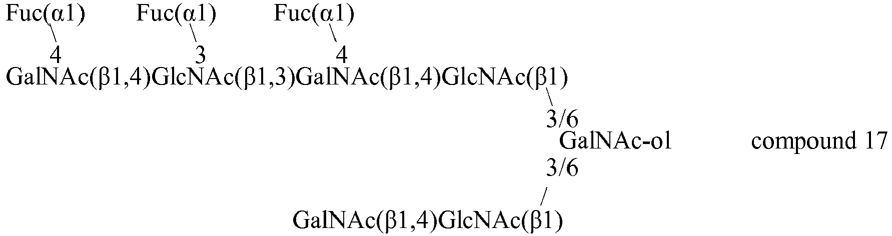

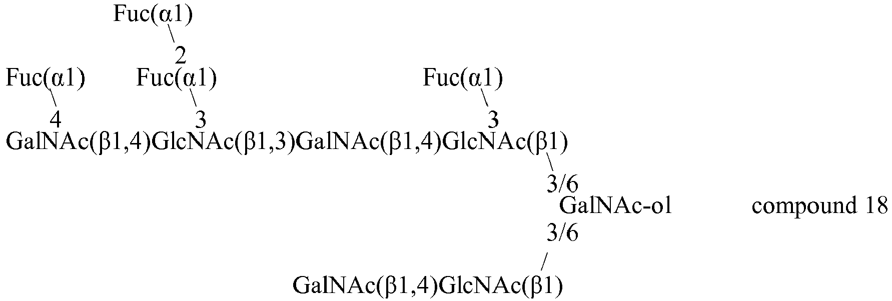

In an advantageous embodiment, the present invention relates to a pharmaceutical composition consisting in or comprising 2, 3, 4, 5 or all of the following compounds:

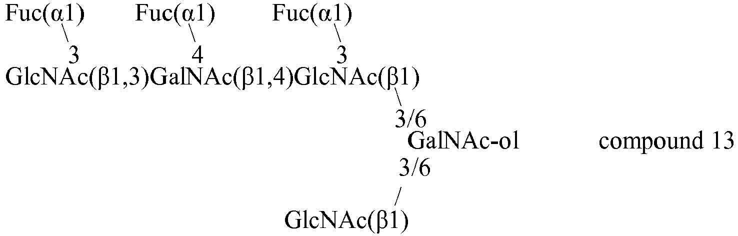

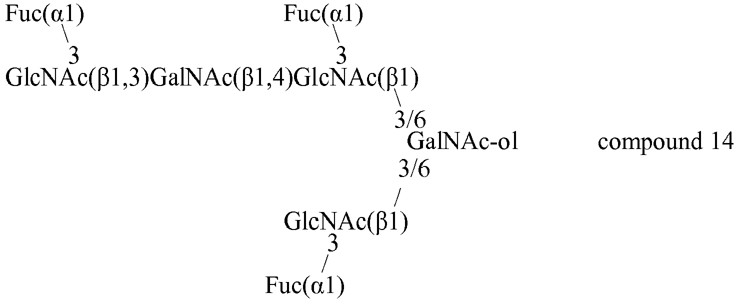

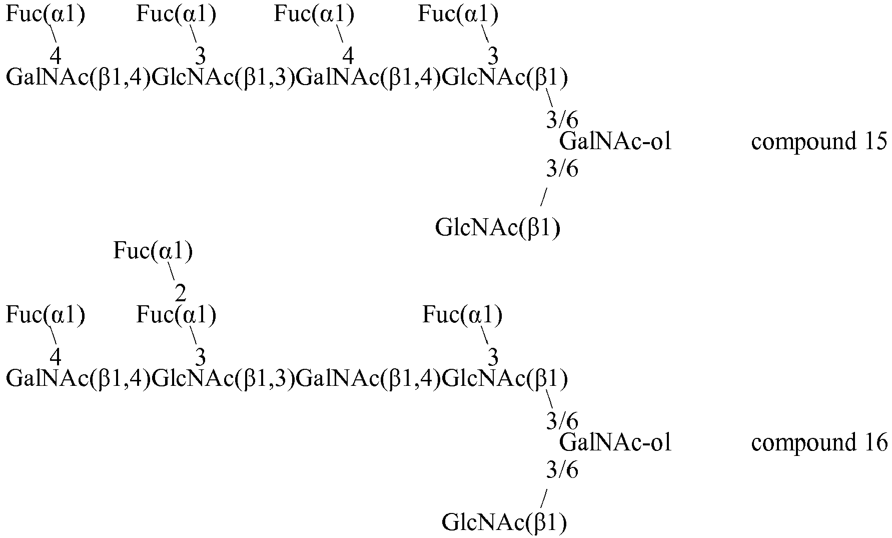

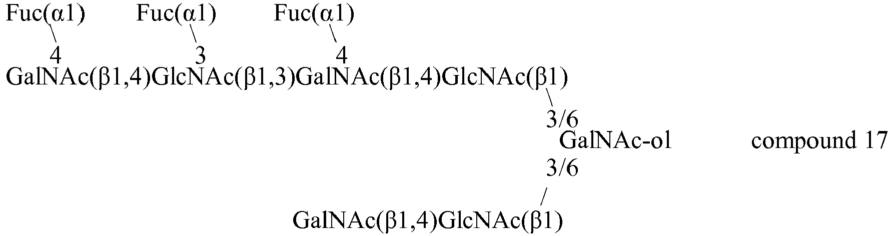

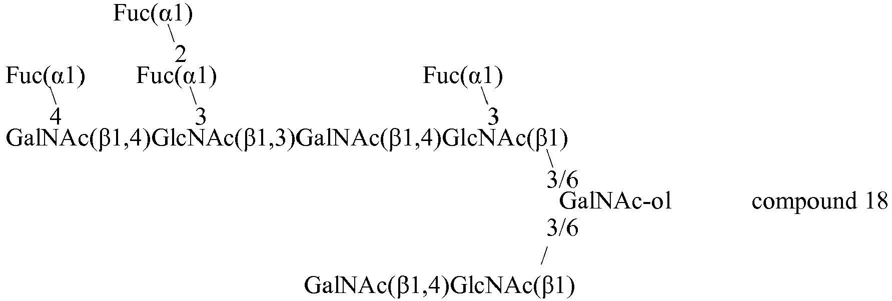

In an advantageous embodiment, the present invention relates to a pharmaceutical composition comprising at least two glycopeptides comprising independently of each other a motif chosen in the group comprising:

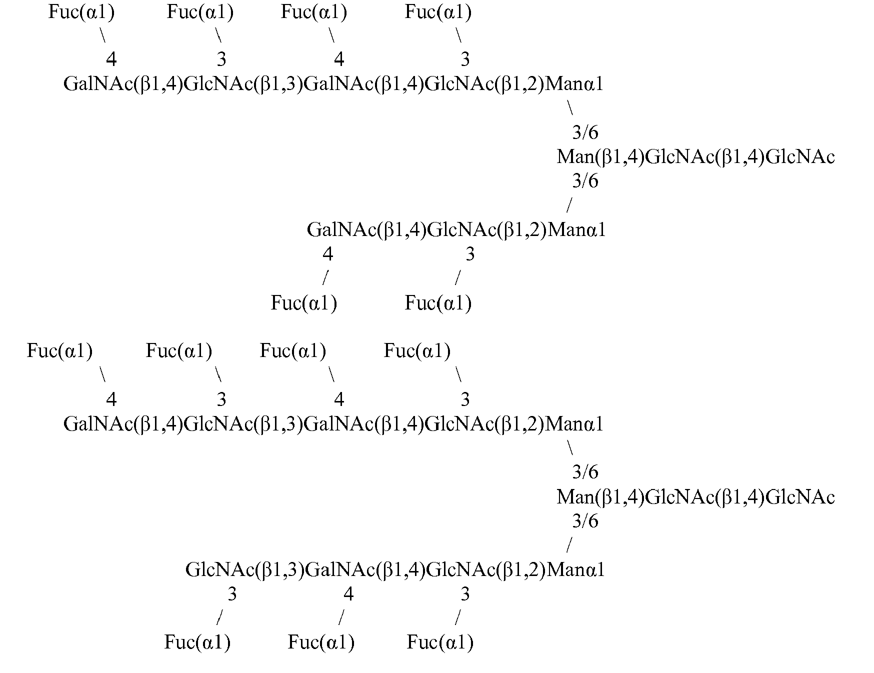

In an advantageous embodiment, the present invention relates to a pharmaceutical composition comprising at least two glycans, said glycans being of the following formulae:

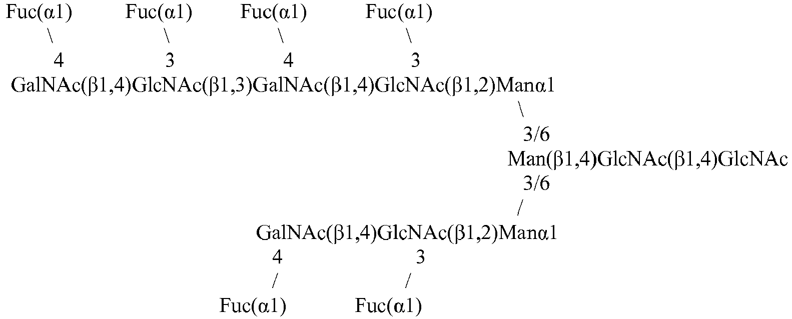

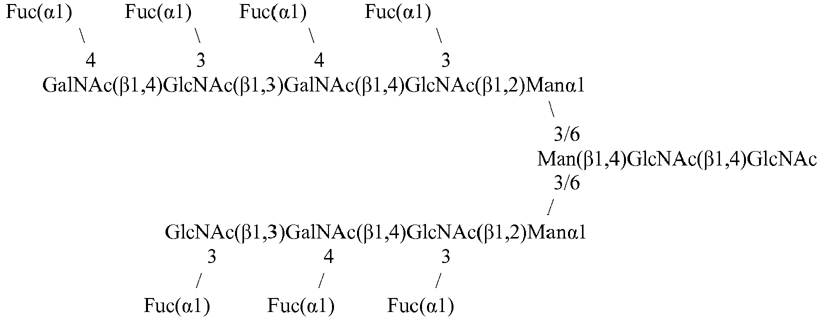

In another aspect, the present invention relates to a composition comprising at least two glycans or glycopeptides comprising independently of each other at least one motif of structure I-3: 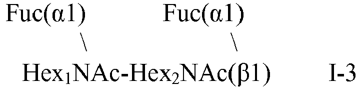 wherein Hex₁ and Hex₂ represent Glc or Gal,
provided that:
- when Hex₁ is Glc, Hex₂ is Gal,
- and when Hex₁ is Gal, Hex₂ is Glc.

Compositions comprising at least two glycans or glycopeptides comprising at least one motif of structure I-3 are new.

In an advantageous embodiment, the present invention relates to a composition, for use as anti-adherence drug to treat infectious disease(s), comprising at least two glycans or glycopeptides comprising at least one motif of structure IV:

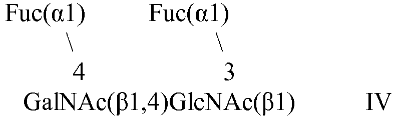

In an advantageous embodiment, the present invention relates to a composition, for use as anti-adherence drug to treat infectious disease(s), comprising at least two glycans or glycopeptides comprising at least one motif of structure IV-2:

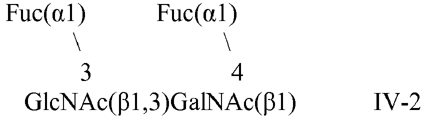

In an advantageous embodiment, the present invention relates to a composition comprising at least two glycans or glycopeptides comprising at least one motif of structure V: 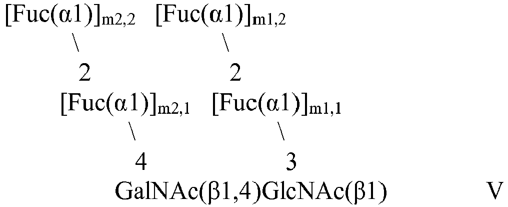 wherein:
m_{1,1}; m_{1,2}; m_{2,1} and m_{2,2} are integers equal to 0 or 1,
provided that:
   - at least one of m_{1,1} and m_{1,2} is equal to 1, and
   - at least one of m_{2,1} and m_{2,2} is equal to 1.

In an advantageous embodiment, the present invention relates to a composition comprising at least two glycopeptides comprising independently of each other a motif of structure VI: 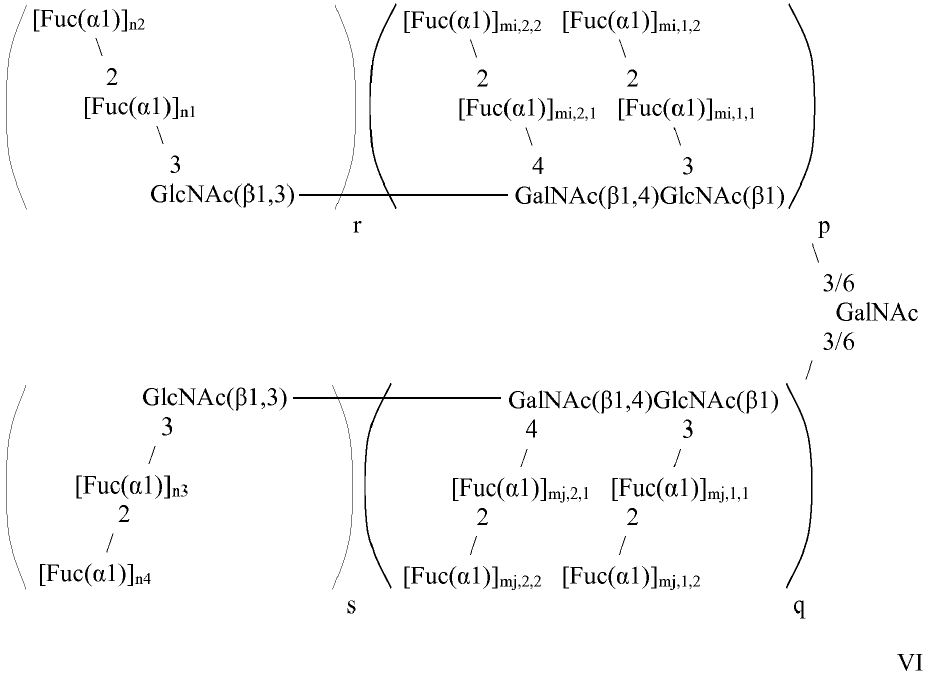 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2} ; n₁; n₂; n₃ and n₄ are integers equal to 0 or 1,
provided that p + q > 0, and:
   - p > 0 and there is at least one i such as:
      - at least one of m_{i,1,1} and m_{i,1,2} is equal to 1, and
      - at least one of m_{i,2,1} and m_{i,2,2} is equal to 1,
and/or
- q > 0 and there is at least one j such as:
   - at least one of m_{j,1,1} and m_{j,1,2} is equal to 1, and
   - at least one of m_{j,2,2} and m_{j,2,2} is equal to 1,
   and/or
- p>0, r> 0 and:
   - at least one of n₁ and n₂ is equal to 1, and
   - there is at least one i such as there at least one of m_{i,2,1} and m_{i,2,2} is equal to 1,
   and/or
- q > 0, s > 0 and:
   - at least one of n₃ and n₄ is equal to 1, and
   - there is at least one i such as there at least one of m_{j,2,1} and m_{j,2,2} is equal to 1.

In an advantageous embodiment, the present invention relates to a composition comprising at least two glycopeptides comprising independently of each other a motif of structure VI: 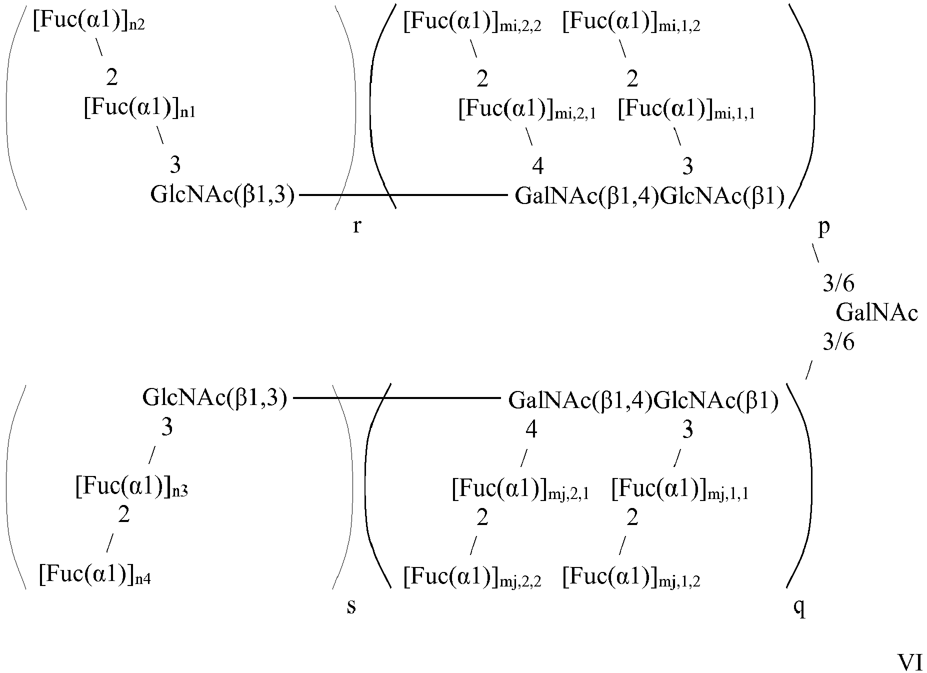 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
   - p > 0 and there is at least one i such as:
      - at least one of m_{i,1,1} and m_{i,1,2} is equal to 1, and
      - at least one of m_{i,2,1} and m_{i,2,2} is equal to 1,
      and/or
   - q > 0 and there is at least one j such as:
      - at least one of m_{j,1,1} and m_{j,1,2} is equal to 1, and
      - at least one of m_{j,2,1} and m_{j,2,2} is equal to 1.

In an advantageous embodiment, the present invention relates to a composition comprising at least two glycopeptides comprising independently of each other a motif of structure VII: 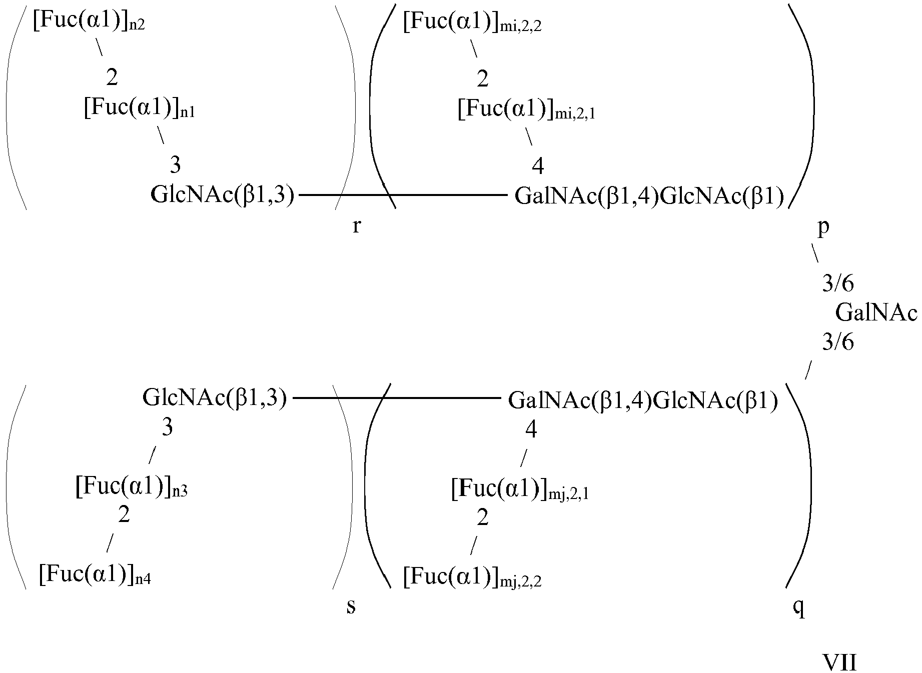 wherein r, p, q, s, m_{i,2,1}; m_{i,2,2}; m_{j,2,1}; m_{j,2,2}; n₁; n₂; n₃ and n₄ have the meaning indicated above.

In an advantageous embodiment, the present invention relates to a composition comprising at least two glycopeptides comprising independently of each other a motif of structure IX: 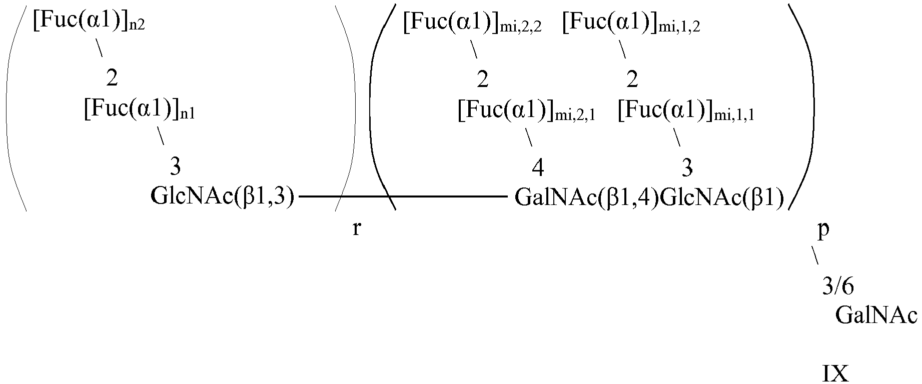 wherein r, p, m_{i,1,1}; m_{i,1,2}; m_{i,2,1}; m_{i,2,2}; n₁ and n₂ have the meaning indicated above.

In an advantageous embodiment, the present invention relates to a composition comprising at least two glycopeptides comprising independently of each other a motif of structure X: 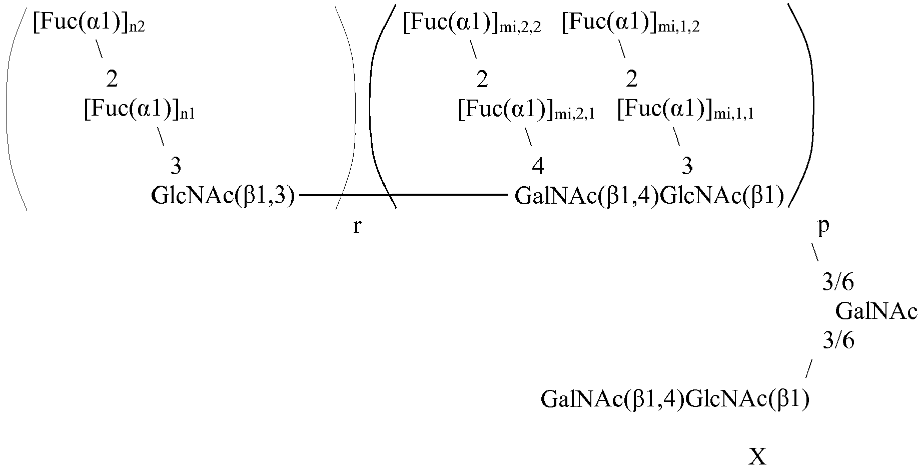 wherein r, p, m_{i,1,1}; m_{i,1,2}; m_{i,2,1}; m_{i,2,2}; n₁ and n₂ have the meaning indicated above.

In an advantageous embodiment, the present invention relates to a composition comprising at least two glycopeptides comprising independently of each other a motif of structure XI: 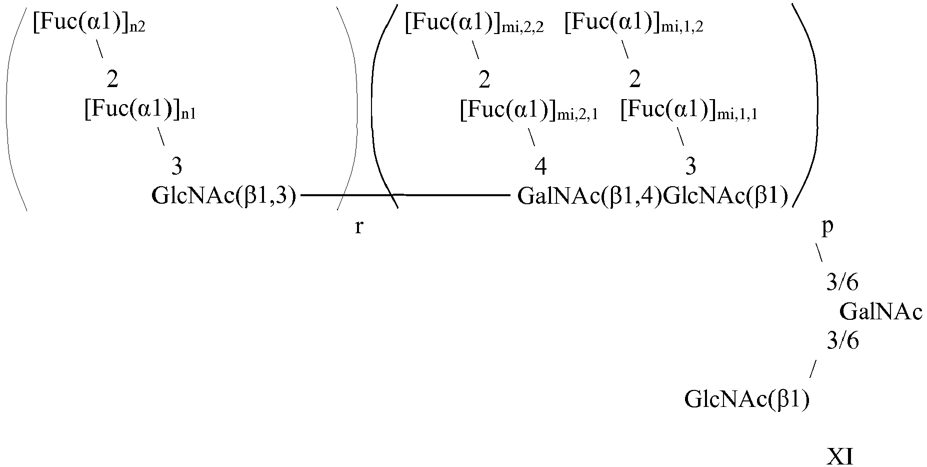 wherein r, p, m_{i,1,1}; m_{i,1,2}; m_{i,2,1}; m_{i,2,2}; n₁ and n₂ have the meaning indicated above.

In an advantageous embodiment, the present invention relates to a composition comprising at least two glycans of structure VI-2: 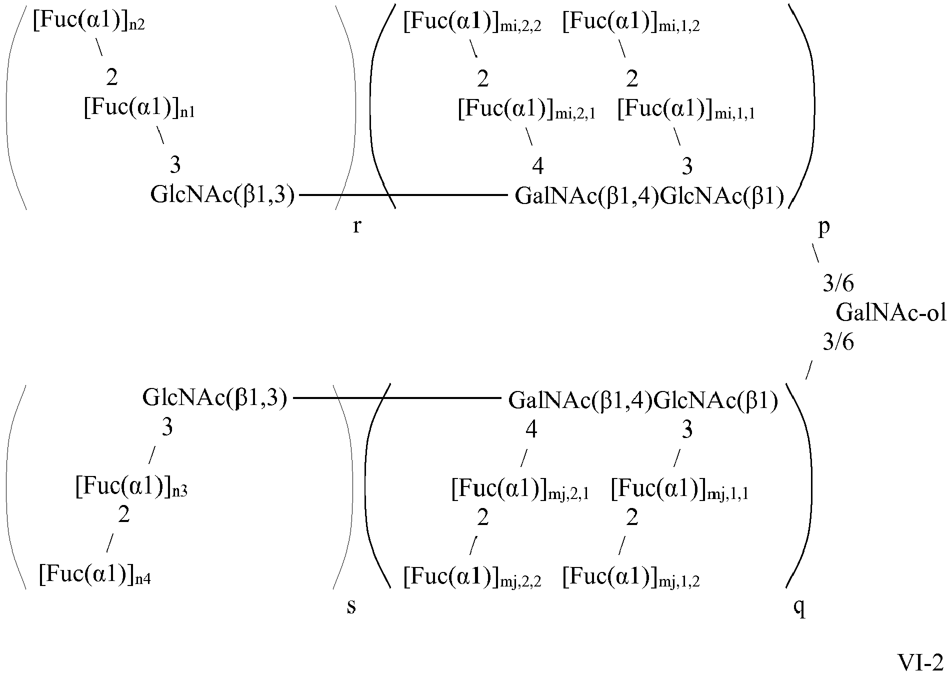 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
   - p > 0 and there is at least one i such as:
      - at least one of m_{i,1,1} and m_{i,1,2} is equal to 1, and
      - at least one of m_{i,2,1} and m_{i,2,2} is equal to 1,
and/or
   - q > 0 and there is at least one j such as:
      - at least one of m_{j,1,1} and m_{j,1,2} is equal to 1, and
      - at least one of m_{j,2,1} and m_{j,2,2} is equal to 1,
and/or
   - p>0, r> 0 and:
      - at least one of n₁ and n₂ is equal to 1, and
      - there is at least one i such as there at least one of m_{i,2,1} and m_{i,2,2} is equal to 1,
and/or
   - q > 0, s > 0 and:
      - at least one of n₃ and n₄ is equal to 1, and
there is at least one i such as there at least one of m_{j,1,2} and m_{j,2,2} is equal to 1.

The abbreviation "GalNAc-ol" refers to N-acetylgalactosaminitol.

In an advantageous embodiment, the present invention relates to a composition comprising at least two glycopeptides comprising independently of each other a motif of structure VI-2: 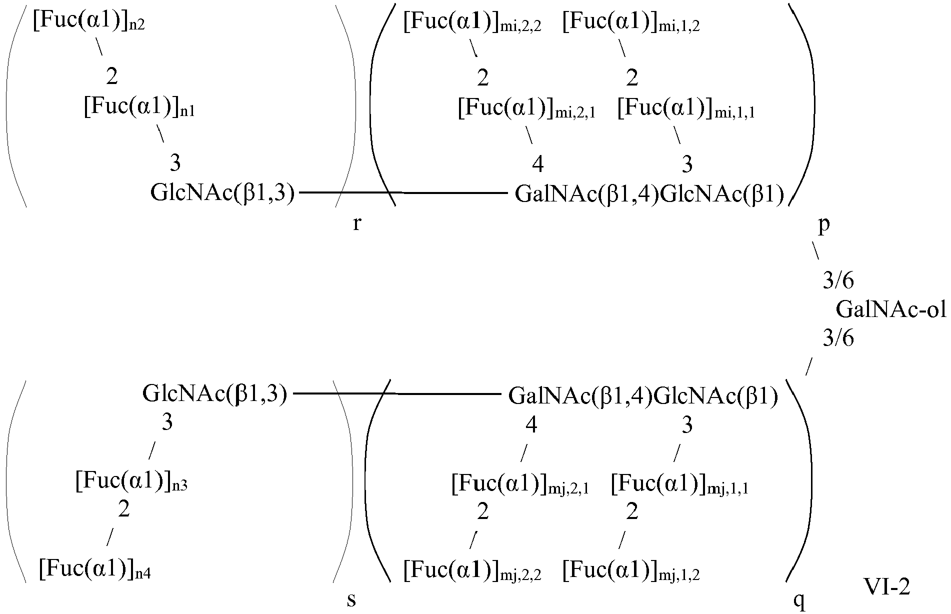 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2} ; n₁; n₂; n₃ and n₄ are integers equal to 0 or 1,
provided that p + q > 0, and:
   - p > 0 and there is at least one i such as:
      - at least one of m_{i,1,1} and m_{i,1,2} is equal to 1, and
      - at least one of m_{i,2,1} and m_{i,2,2} is equal to 1,
and/or
   - q > 0 and there is at least one j such as:
      - at least one of m_{j,1,1} and m_{j,1,2} is equal to 1, and
      - at least one of m_{j,2,1} and m_{j,2,2} is equal to 1.

In an advantageous embodiment, the present invention relates to a composition comprising at least two glycans of structure VII-2: 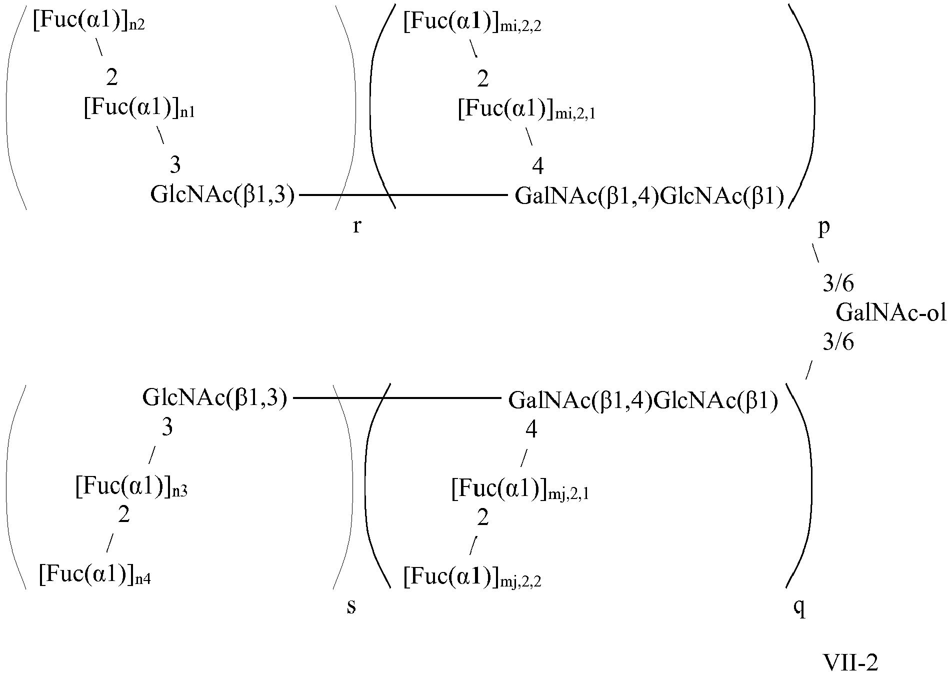 wherein r, p, s, q, m_{i,2,1} ; m_{i,2,2}; m_{j,2,1}; m_{j,2,2}; n₁; n₂; n₃ and n₄ have the meaning indicated above.

In an advantageous embodiment, the present invention relates to a composition comprising at least two glycans of structure IX-2: 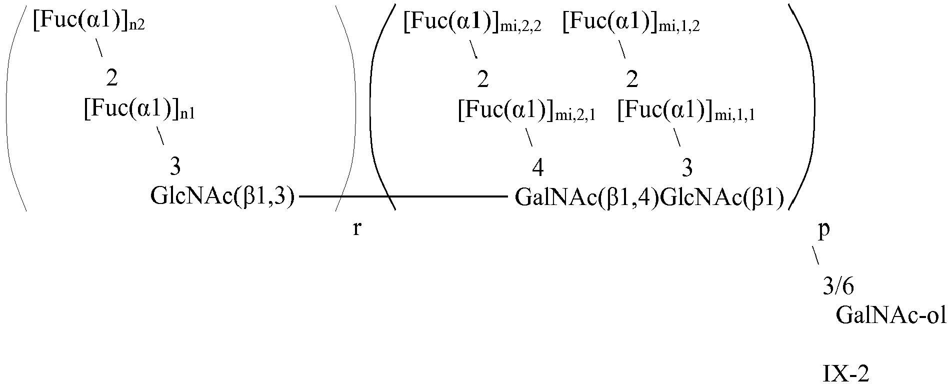 wherein r, p, m_{i,1,1}; m_{i,1,2}; m_{i,2,1}; m_{i,2,2}; n₁ and n₂ have the meaning indicated above.

In an advantageous embodiment, the present invention relates to a composition comprising at least two glycans of structure X-2: 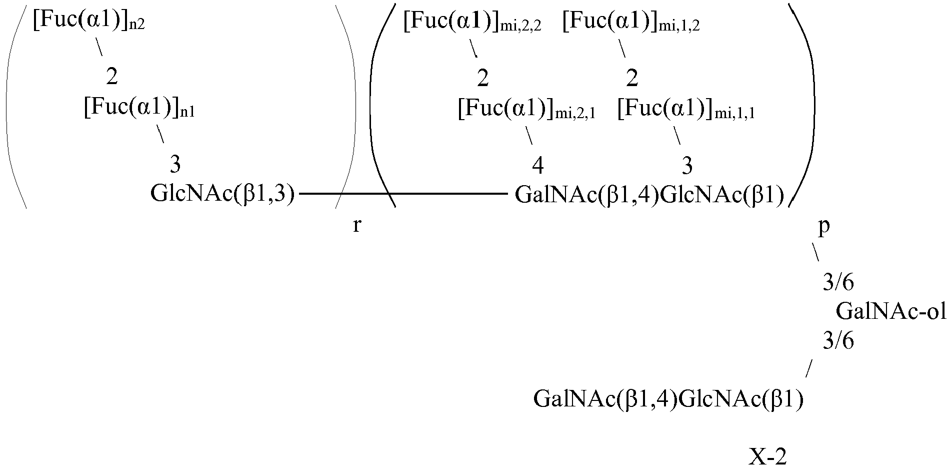 wherein r, p, m_{i,1,1}; m_{i,1,2}; m_{i,2,1}; m_{i,2,2}; n₁ and n₂ have the meaning indicated above.

In an advantageous embodiment, the present invention relates to a composition comprising at least two glycans of structure XI-2: 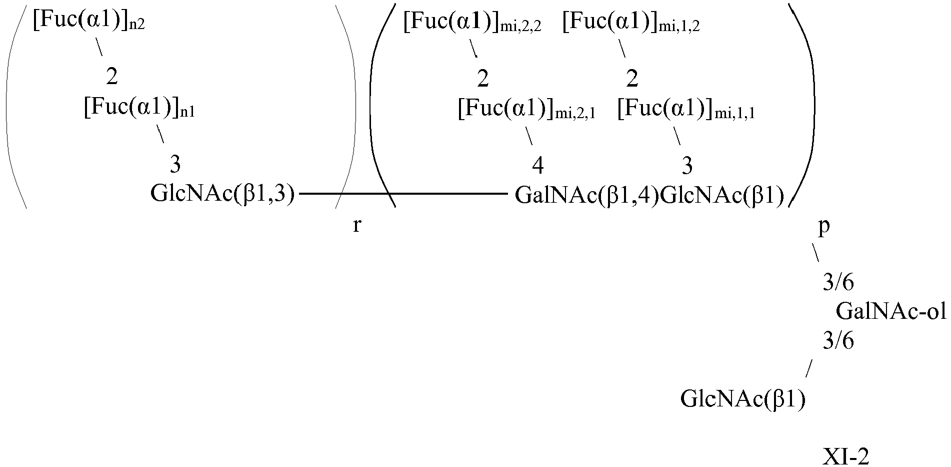 wherein r, p, m_{i,1,1}; m_{i,1,2}; m_{i,2,1}; m_{i,2,2}; n₁ and n₂ have the meaning indicated above.

In an advantageous embodiment, the present invention relates to a composition comprising at least two glycopeptides comprising independently of each other a motif of structure VI, of the following particular formula XII: 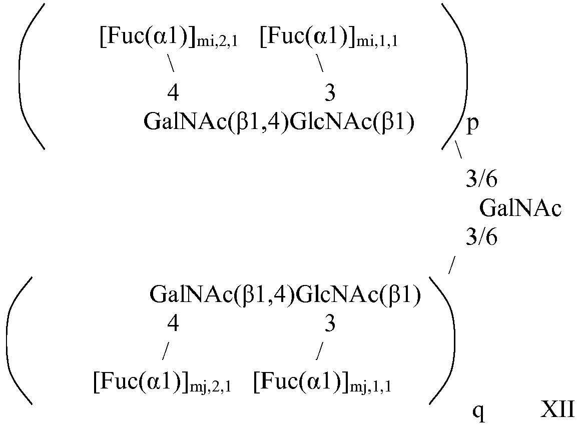 wherein p, q, m_{i,1,1}; m_{i,2,1}; m_{j,1,1} and m_{j,2,1} have the meaning indicated above.

In an advantageous embodiment, the present invention relates to a composition comprising at least two glycans of structure VI-2, of the following particular formula XII-2: 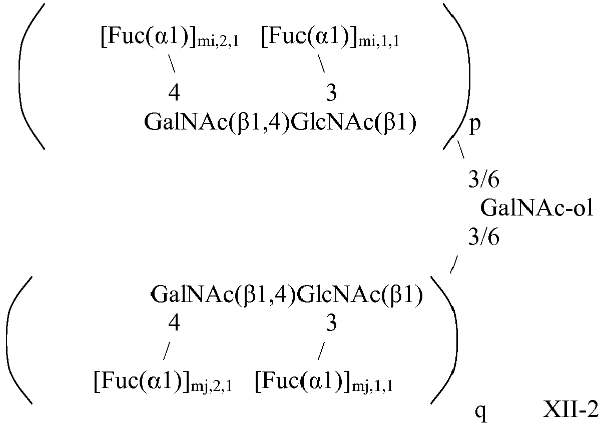 wherein p, q, m_{i,1,1}; m_{i,2,1}; m_{j,1,1} and m_{j,2,1} have the meaning indicated above.

In an advantageous embodiment, the present invention relates to a composition comprising at least two glycans of structure VII or glycopeptides comprising independently of each other a motif of structure XV: 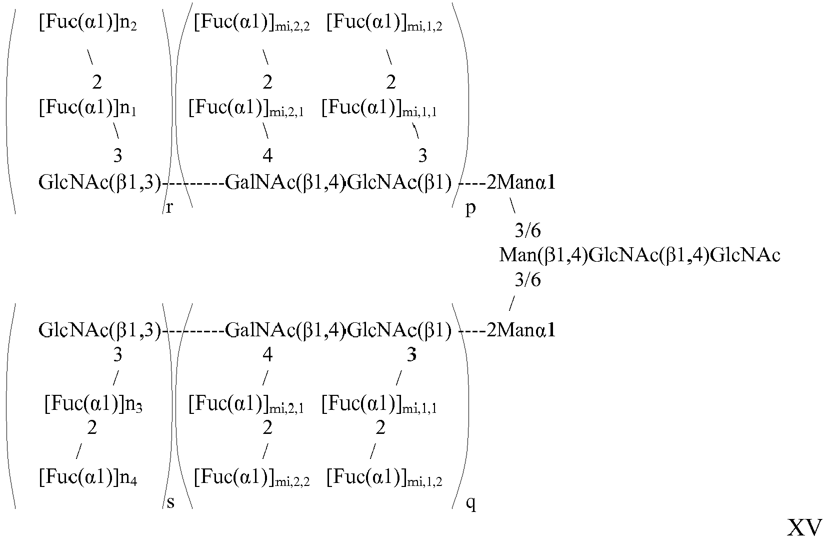 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
   - p > 0 and there is at least one i such as:
      - at least one of m_{i,1,1} and m_{i,1,2} is equal to 1, and
      - at least one of m_{i,2,1} and m_{i,2,2} is equal to 1,
and/or
   - q > 0 and there is at least one j such as:
      - at least one of m_{j,1,1} and m_{i,1,2} is equal to 1, and
      - at least one of m_{j,2,1} and m_{j,2,2} is equal to 1,
and/or
   - p>0, r> 0 and:
      - at least one of n₁ and n₂ is equal to 1, and
      - there is at least one i such as there at least one of m_{i,2,1} and m_{i,2,2} is equal to 1,
and/or
   - q > 0, s > 0 and:
      - at least one of n₃ and n₄ is equal to 1, and
      - there is at least one i such as there at least one of m_{j,2,1} and m_{j,2,2} is equal to 1.

In an advantageous embodiment, the present invention relates to a composition comprising at least two glycans of structure VII or glycopeptides comprising independently of each other a motif of structure XV: 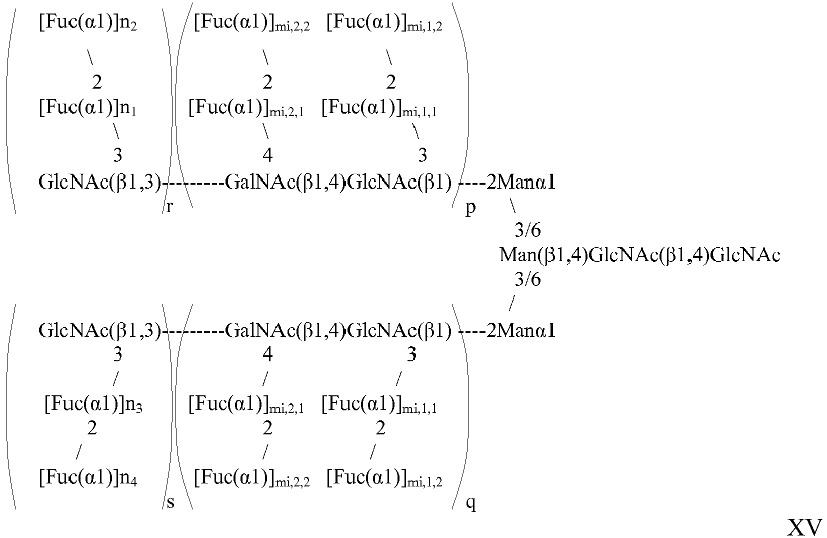 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
   - p > 0 and there is at least one i such as:
      - at least one of m_{i,1,1} and m_{i,1,2} is equal to 1, and
      - at least one of m_{i,2,1} and m_{i,2,2} is equal to 1,
and/or
   - q > 0 and there is at least one j such as:
      - at least one of m_{j,1,1} and m_{j,1,2} is equal to 1, and
      - at least one of m_{j,2,1} and m_{j,2,2} is equal to 1.

In an advantageous embodiment, the present invention relates to a composition comprising at least two glycans of structure XV or glycopeptides comprising independently of each other a motif of structure XV, of particular structure XVI: 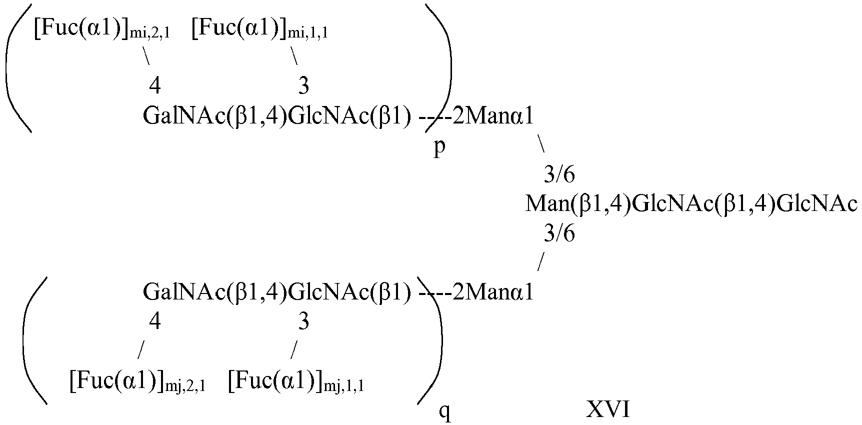 wherein p, q, m_{i,1,1}; m_{i,2,1}; m_{j,1,1} and m_{j,2,1} have the meaning indicated above.

In an advantageous embodiment, the present invention relates to a composition wherein the total content of Fuc, GalNAc and GlcNAc are, in molar percentages, as follows:
Fuc: from 5 to 25, in particular from 10 to 25, more particularly from 18 to 25;
GalNac: from 5 to 15;
GlcNAc: from 30 to 50.

In an advantageous embodiment, the present invention relates to a composition wherein said glycans are isolated from *Halocynthia roretzi.*

In an advantageous embodiment, the present invention relates to a composition comprising at least two glycopeptides comprising independently of each other a motif, the structure of which being chosen in the group comprising:

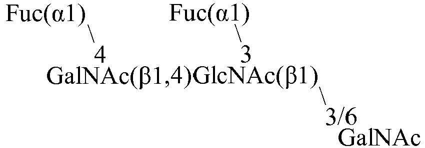

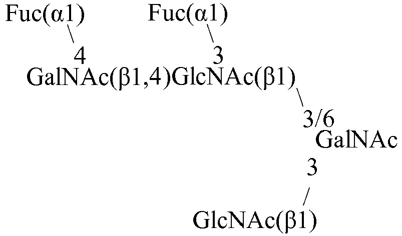

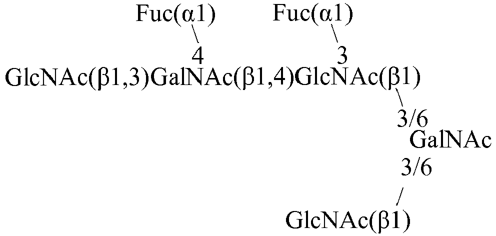

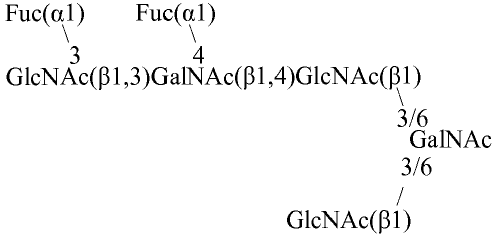

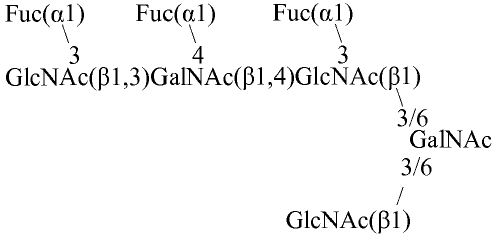

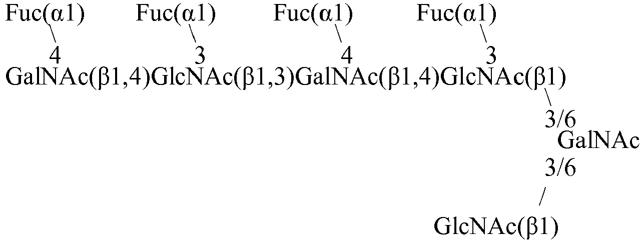

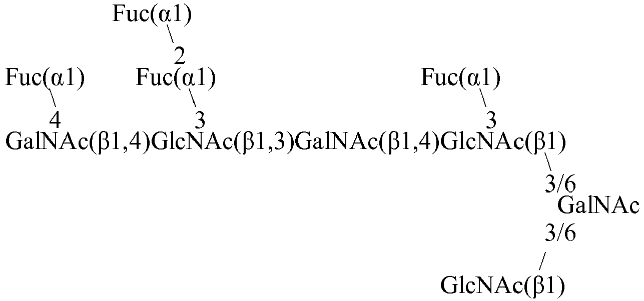

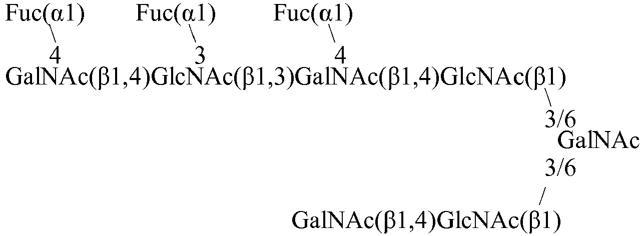

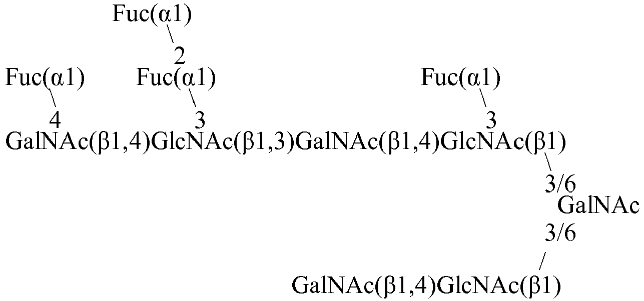

In an advantageous embodiment, the present invention relates to a composition, for use as anti-adherence drug to treat infectious disease(s), comprising at least two glycans, said glycans being chosen in the group comprising:

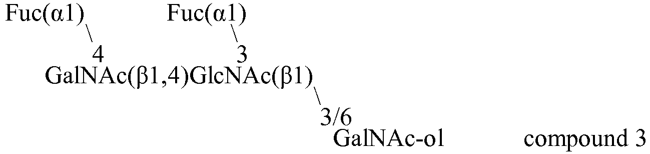

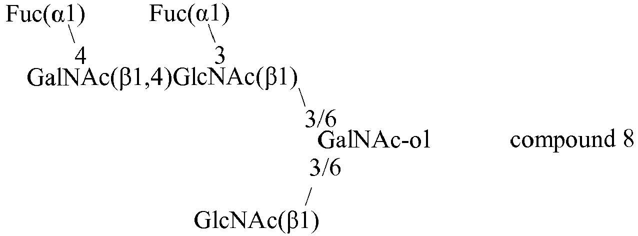

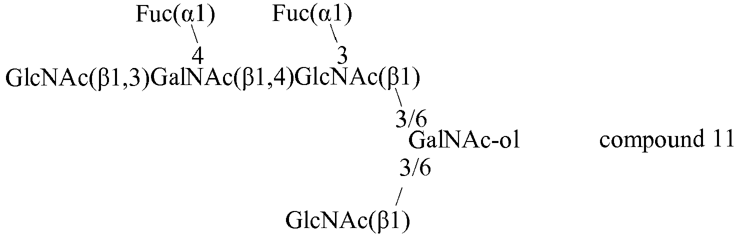

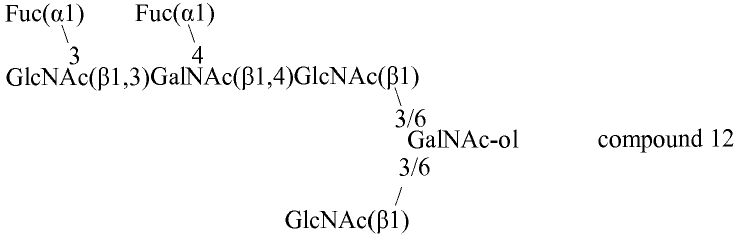

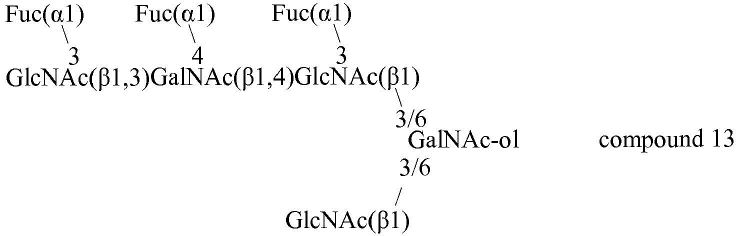

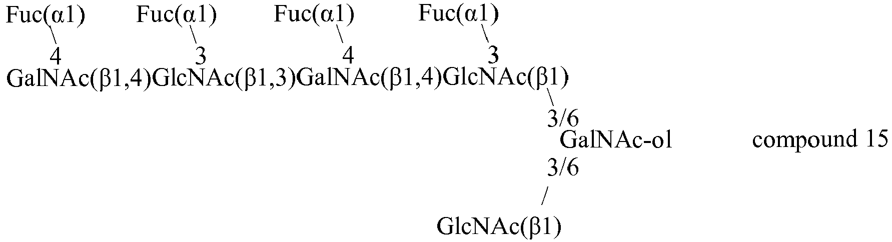

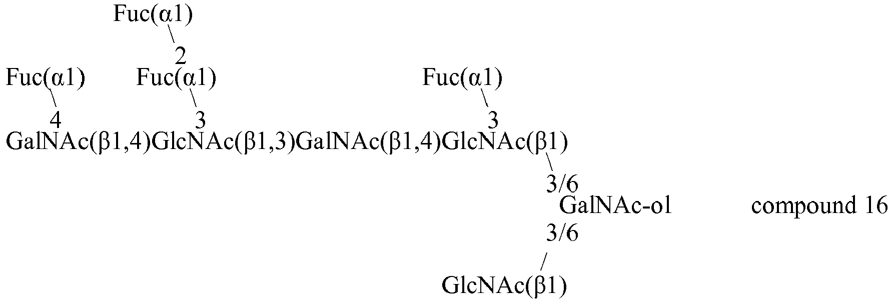

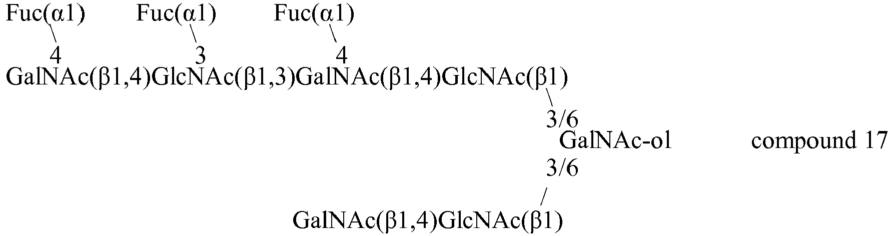

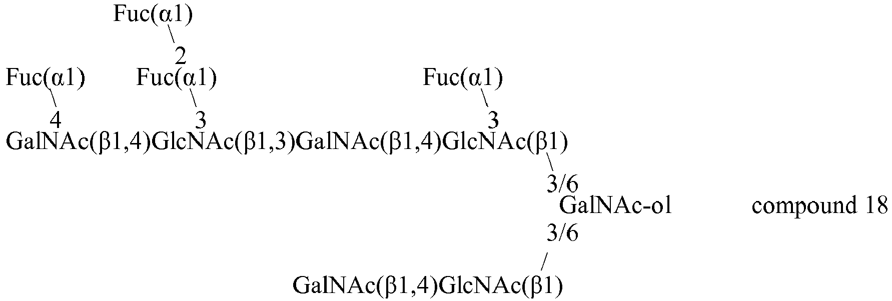

In an advantageous embodiment, the present invention relates to a composition, for use as anti-adherence drug to treat infectious disease(s), consisting in or comprising 2, 3, 4 or all of the following compounds:

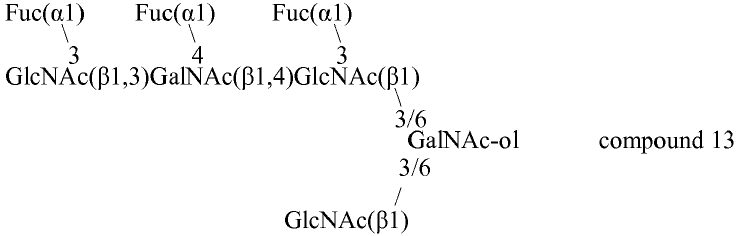

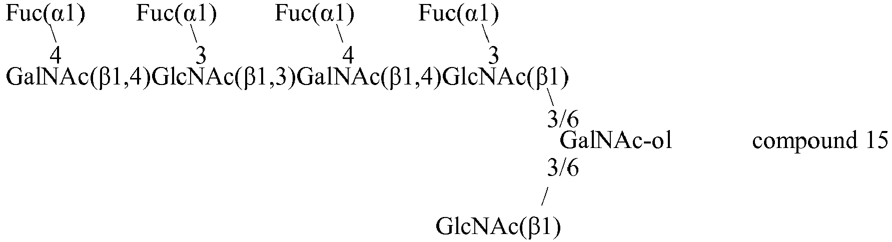

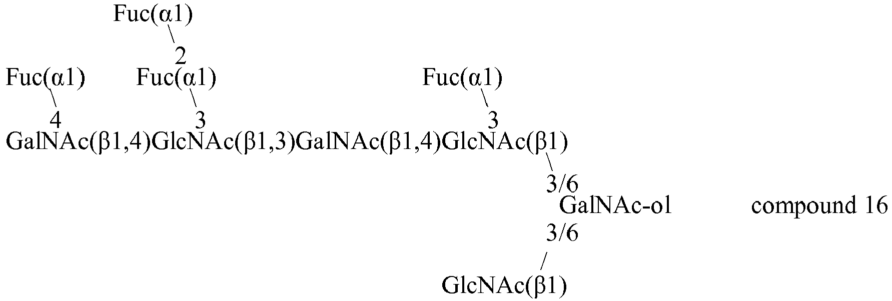

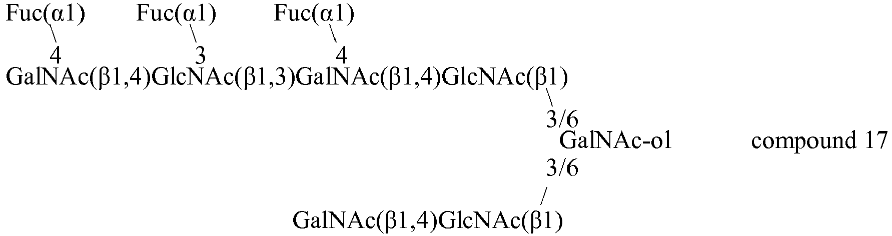

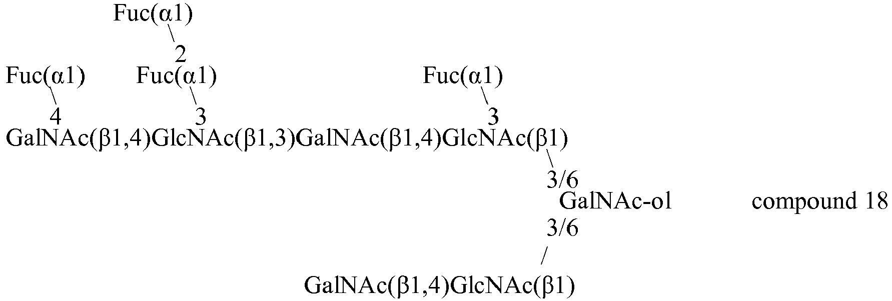

In an advantageous embodiment, the present invention relates to a composition, for use as anti-adherence drug to treat infectious disease(s), comprising at least two glycopeptides comprising independently of each other a motif chosen in the group comprising:

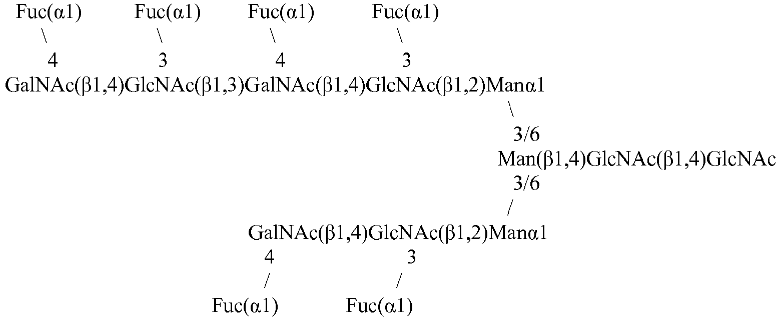

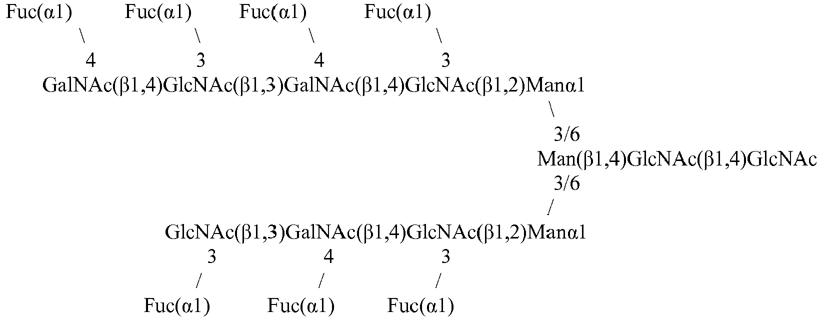

In an advantageous embodiment, the present invention relates to a composition, for use as anti-adherence drug to treat infectious disease(s), comprising at least two glycans, said glycans being of the following formulae:

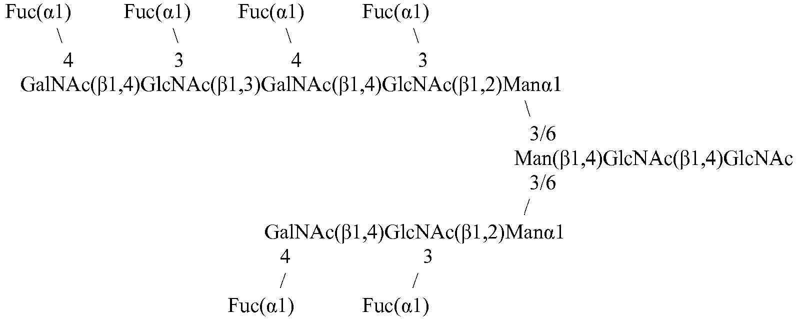

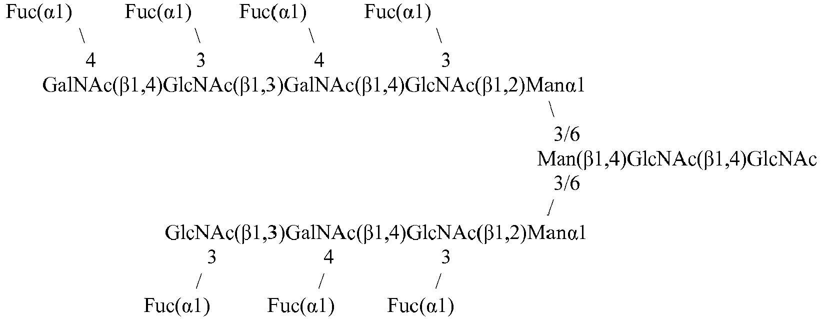

The present invention also relates to a process of preparation of a composition comprising at least two glycans or glycopeptides comprising independently of each other at least one motif of structure I: 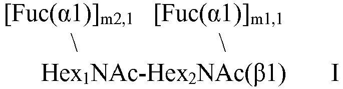 wherein:
m_{1,1} and m_{2,1} are integers equal to 0 or 1,
Hex₁ and Hex₂ represent Glc or Gal,
provided that:
   - at least one of m_{1,1} and m_{2,1} is equal to 1, and
   - when Hex₁ is Glc, Hex₂ is Gal,
   - and when Hex₁ is Gal, Hex₂ is Glc,
said process of preparation comprising the following steps:
   - extraction of glycoproteins, in particular from gonads of *Halocynthia rortezi,* to obtain a glycoprotein composition;
   - digestion of said glycoprotein composition by (a) protease(s) to obtain a digested protein composition;
   - purification of said digested protein composition to obtain said composition comprising at least two glycopeptides comprising at least one motif of structure I.

Extraction of glycoproteins, in particular from gonads of *Halocynthia rortezi* may be performed by any technique known by those skilled in the art.

For example, gonads are minced and suspended in a buffered aqueous solution. Soluble compounds dissolved in aforesaid solution are separated from insoluble compounds, for instance by a first centrifugation. Soluble glycoproteins are precipitated from aforesaid solution by adding an organic solvent or a mixture containing at least one organic solvent. Aforesaid soluble glycoproteins are separated from the supernatant containing free glycans, for instance by a second centrifugation. The insoluble compounds obtained after mincing and suspension are extracted at least once with an organic solvent or a mixture containing at least one organic solvent. Obtained organic extract(s) contain(s) glycolipids and are consequently discarded. Remaining solid contains insoluble glycoproteins. The total glycoprotein fraction corresponds to the soluble and the insoluble glycoproteins.

By "digestion by (a) protease(s)" is meant obtention of water soluble polypeptides linked to one or several oligosaccharides or single amino-acids linked to a single oligosaccharide. The oligosaccharides may be covalently linked to the carrying amino-acid backbone either through a N-glycosidic bond between an N-acetylglucosamine and an asparagine or through a o-glycosidic bond between a N-acetylgalactosamine and a threonine or a serine.

Examples of protease are Pronase^{®} from *Streptomyces griseus* (Roche Applied Science) and Proteinase K from *Tritirachium album* (Sigma-Aldrich).

Aforesaid purification enables separation of aforesaid glycopeptides from aforesaid digested protein composition containing, in addition to glycopeptides and protease(s), amino acids and/or peptides.

Aforesaid purification may be performed by any technique known by those skilled in the art, in particular ethanol precipitation, gel filtration chromatography and anion exchange chromatography.

In an advantageous embodiment, the present invention relates to a process of preparation of a composition comprising at least two glycans or glycopeptides comprising at least one motif of structure I-1: 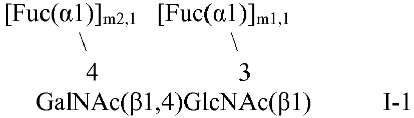 wherein m_{1,1} and m_{2,1} have the meaning indicated above.

In an advantageous embodiment, the present invention relates to a process of preparation of a composition comprising at least two glycans or glycopeptides comprising at least one motif of structure I-2: 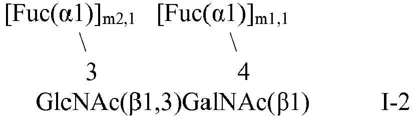 wherein m_{1,1} and m_{2,1} have the meaning indicated above.

In an advantageous embodiment, the present invention relates to a process of preparation of a composition comprising at least two glycans of structure VI-1: 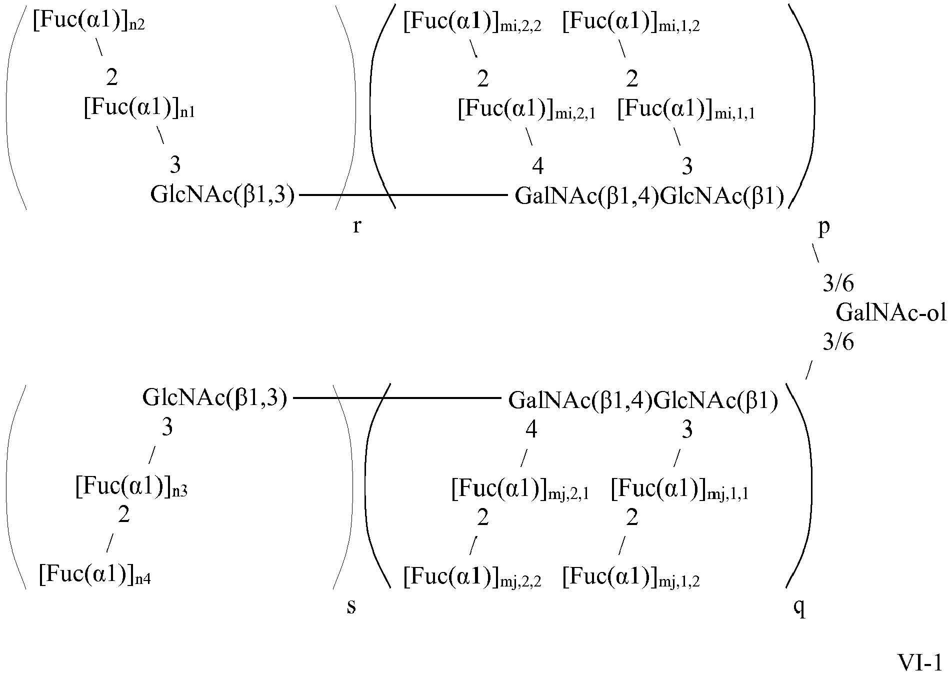 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and m_{i,2,2} is equal to 1, and/or
   - q > 0 and there is at least one j such as at least one of m_{j,1,1}; m_{j,1,2}; m_{j,2,1} and m_{j,2,2} is equal to 1, and/or
   - p > 0, r > 0 and there at least one of m_{i,1,1}; m_{i,1,2}; m_{i,2,1}; m_{i,2,2}; n₁; n₂; n₃ and n₄ is equal to 1, and/or
   - q > 0, s > 0 and there at least one of m_{i,1,1}; m_{i,1,2}; m_{i,2,1}; m_{i,2,2}; n₁; n₂; n₃ and n₄ is equal to 1,
said process of preparation comprising after said extraction, digestion and purification the following step:
   - releasing said composition comprising at least two glycans of structure VI from said composition comprising at least two glycopeptides comprising at least one motif of structure I, in particular by hydrazinolysis followed by N-reacetylation of said composition comprising at least two glycopeptides.

Said glycans of structure VI are O-glycans: they are released from glycopeptides wherein the carbohydrate part is linked to the peptide by a bond involving an atom of oxygen.

By "releasing" is meant cleavage of the covalent bond linking the glycan part(s) and the peptide side chain(s) of aforesaid glycopeptides.

N-glycans may be released from corresponding glycopeptides by any technique known by those skilled in the art, in particular hydrazinolysis followed by N-reacetylation of N-acetylhexosamine residues , alkalynolysis, enzymatic digestion using several enzymes including peptide-N(4)-(N-acetyl-beta-glucosaminyl)asparagine amidase (PNGase F and PNgas A, EC 3.5.1.52) from *Elizabethkingia meningoseptica, Elizabethkingia miricola,* and almond and endoglycosidase (EC 3.2.96) from *Streptomyces plicatus* and *Streptomyces griseus.*

Hydrazinolysis may be performed by treating said composition comprising at least two glycopeptides comprising at least one motif of structure I with anhydrous hydrazine at a temperature comprised from 80 to 120°C, preferably from 90 to 110°C, more preferably from 95 to 105°C.

In an advantageous embodiment, the present invention relates to a process of preparation of a composition comprising at least two glycans of structure VII: 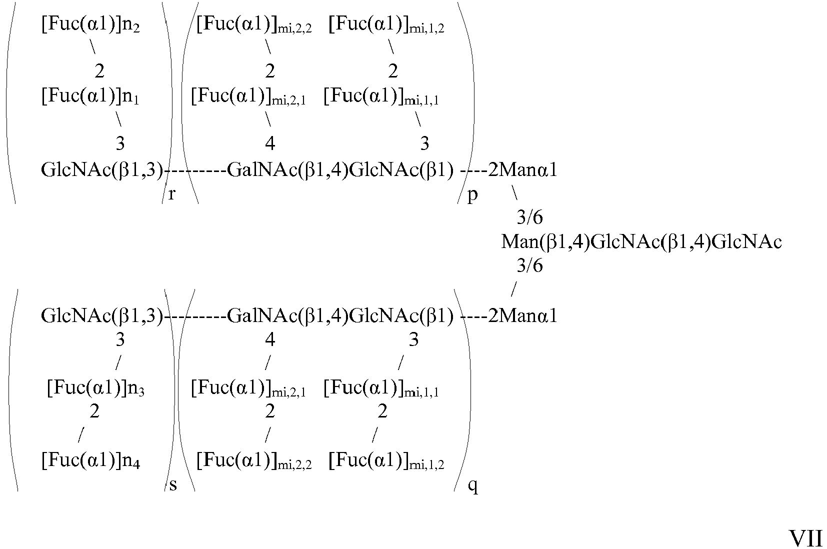 wherein:
p and q are integers equal to 0, 1, 2 or 3,
r and s are integers equal to 0 or 1,
i is an integer:
   - equal to 0 when p is equal to 0,
   - equal to 1 when p is equal to 1,
   - and varying from 1 to 2 when p is equal to 2,
j is an integer:
   - equal to 0 when q is equal to 0,
   - equal to 1 when q is equal to 1,
   - and varying from 1 to 2 when q is equal to 2,
m_{i,1,1}; m_{i,1,2}; m_{i,2,1} ; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
   - p > 0 and there is at least one i such as at least one of m_{i,1,1}; m_{i,1,2}; m_{i,2,1} and m_{i,2,2} is equal to 1, and/or
   - q > 0 and there is at least one j such as at least one of m_{j,1,1}; m_{j,1,2}; m_{j,2,1} and m_{j,2,2} is equal to 1, and/or
   - p > 0, r > 0 and there at least one of m_{i,1,1}; m_{i,1,2}; m_{i,2,1}; m_{i,2,2}; n₁; n₂; n₃ and n₄ is equal to 1, and/or
   - q > 0, s > 0 and there at least one of m_{i,1,1}; m_{i,1,2}; m_{i,2,1}; m_{i,2,2}; n₁; n₂; n₃ and n₄ is equal to 1, said process of preparation comprising after said extraction and purification the following step:
   - releasing said composition comprising at least two glycans of structure VII from said composition comprising at least glycopeptides comprising at least one motif of structure I, in particular by reductive β-elimination of said composition comprising at least two glycopeptides.

Said glycans of structure VII are N-glycans: they are isolated from glycopeptides wherein the carbohydrate part is linked to the peptide by a bond involving an atom of nitrogen.

O-glycans may be isolated from corresponding glycopeptides by any technique known by those skilled in the art, in particular reductive β-elimination or mild hydrazinolysis.

Reductive β-elimination may be performed by treating said composition comprising at least two glycopeptides comprising at least one motif of structure I with a reducing agent, for example sodium borohydride or potassium borohydride at a temperature comprised from 10 to 60°C, preferably from 20 to 50°C, more preferably from 37 to 45°C.

Said reducing agent may be used after adding an alkaline solution, for example a sodium hydroxide aqueous solution, to said composition comprising at least two glycopeptides.

### DESCRIPTION OF THE DRAWINGS

**Figure 1** presents the general scheme of purification of glycoconjugates from *H*. *roretzi* gonads.
**Figure 2** presents the separation of O-glycans liberated from gonads by reductive β-elimination on a gel filtration column (Biogel P6) analysed by thin layer chromatography and orcinol-sulfuric staining.

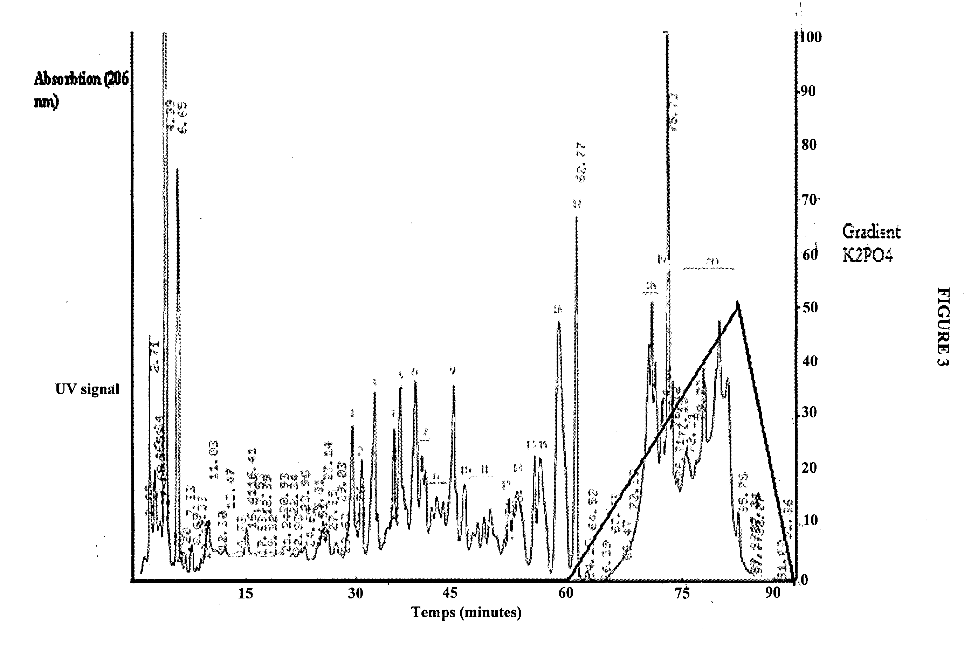

**Figure 3** presents the separation of O-glycans liberated from gonads by reductive β-elimination prior to NMR analysis by normal phase column HPLC (Supercosil LC-NH2 25x4,6cm 5µm ) in a gradient of water and potassium phosphate in acetonitrile (ACN/H₂O/H₂PO₄K 80:20:0 to ACN/H₂O/H₂PO₄K 50:0:50 in 60 min).

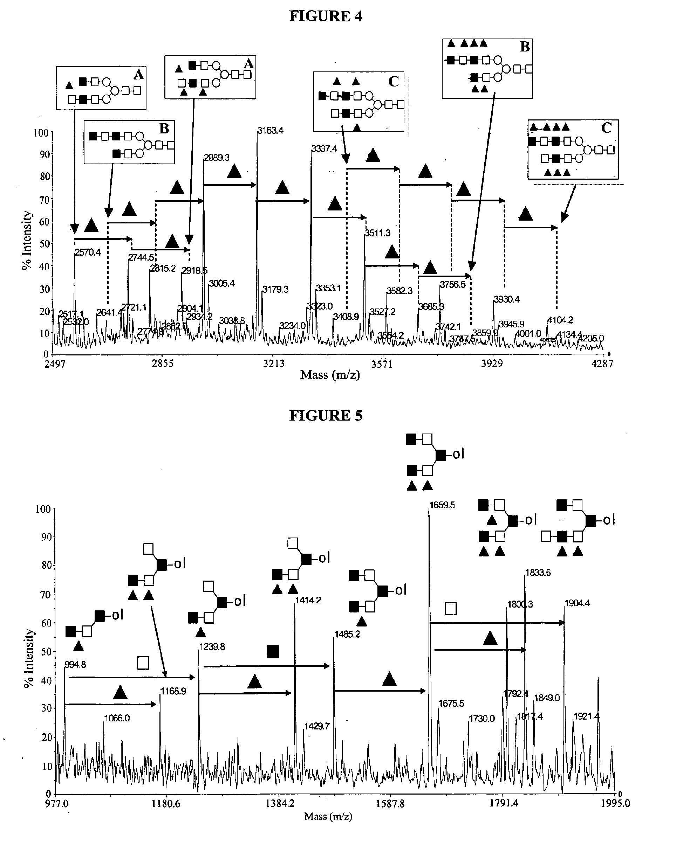

**Figure 4** presents an example of MALDI-TOF MS analysis of permethylated N-glycans isolated from glycoprotein fraction of *H. roretzi* gonads. White square, N-acetylglucosamine; black square, N-acetylgalactosamine; white circle, mannose; black triangle, fucose.
**Figure 5** presents an example of MALDI-TOF MS analysis of total O-glycans liberated from gonads by reductive β-elimination. White square, N-acetylglucosamine; black square, N-acetylgalactosamine; black triangle, fucose.

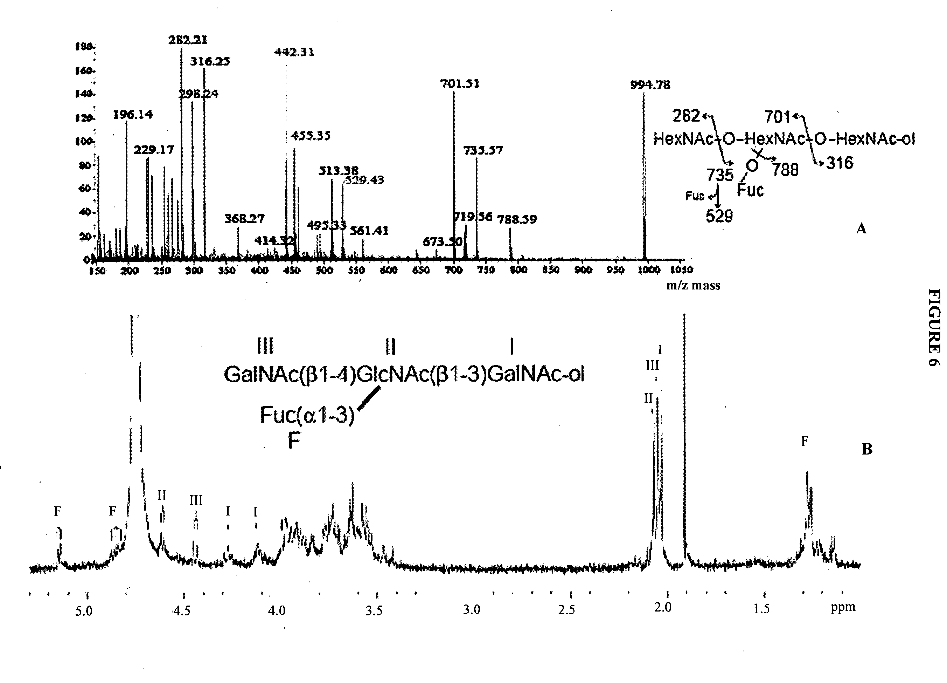

**Figure 6** presents an example of MS/MS (**A**) and ¹H 1D NMR (**B**) analysis of a monofucosylated O-glycan containing the motif GalNAc(β1-4)[Fuc(α1-3)]GlcNAc motif, liberated from gonads by reductive β-elimination after separation by normal phase HPLC. Oligosaccharide was analyzed by MS/MS as permethylated derivative.

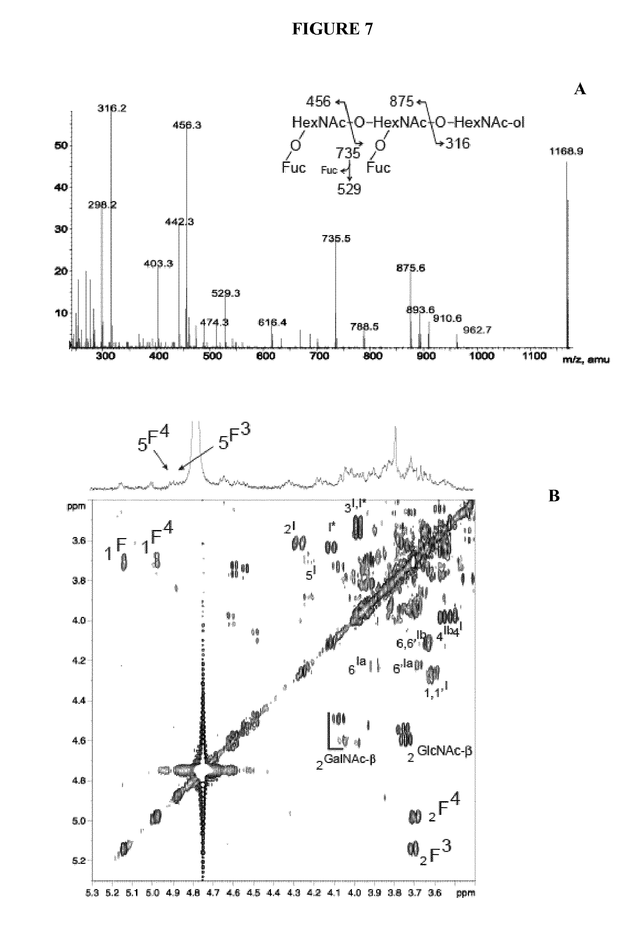

**Figure 7** presents an example of MS/MS (**A**) and ¹H/¹H COSY NMR (**B**) analysis of a difucosylated O-glycan containing the motif Fuc(α1-4)GalNAc(β1-4)[Fuc(α1-3)]GlcNAc, liberated from gonads by reductive β-elimination after separation by normal phase HPLC. Oligosaccharide was analyzed by MS/MS as permethylated derivative.
**Figure 8** presents a FSC vs SSC dot plot. Cellular aggregates or debris, with very high or low FSC or SSC characteristics were gated out. Fluorescence of PKH2 dye, emitted in the FITC channel, was measured for each single-cell.
**Figure 9** presents mean fluorescence intensity of infected-single cells. The solid line, representing cells infected with non-labelled bacteria, indicates the basal fluorescence of cells. The dotted line, representing cells infected with labelled bacteria in absence of *H. roretzi* extracts, is used as a positive control of adherence (100%). The filled line, representative infected cells in presence of *H. roretzi* extracts, displays a decrease in fluorescence.
**Figure 10** presents the separation of O-glycans liberated from *Halocynthia roretzi* gonads by reductive β-elimination on a gel filtration column (Biogel P6) irrigated by water, and analyzed by thin layer chromatography (SilicaGe160 run in butanol/acetic acid/water 2:1:1.5) and orcinol-sulfuric staining, and the O-glycan fraction "OG P6-2" used for inhibition assays.
**Figure 11** presents the relative adherence of labeled bacteria (in %) to human gastric epithelial cells incubated with increasing concentrations of fucosylated O-glycans compared to untreated cells.
**Figure 12** presents the monosaccharide composition (%) of glycopeptide fraction generated and purified from *Halocynthia roretzi* gonads proteins.
**Figure 13** presents the relative adherence of labeled bacteria (in %) to human gastric epithelial cells incubated with increasing concentrations of total glycopeptides compared to cells incubated with O-glycans and untreated cells.
**Figure 14** presents the relative adherence of labeled bacteria (in %) to human gastric epithelial cells incubated with increasing concentrations of defucosylated (defuc) O-glycans and defucosylated glycopeptides compared to cells incubated with their respective fucosylated counterparts and to untreated cells.

### EXAMPLES

### Example 1: preparation of a composition comprising glycopeptides

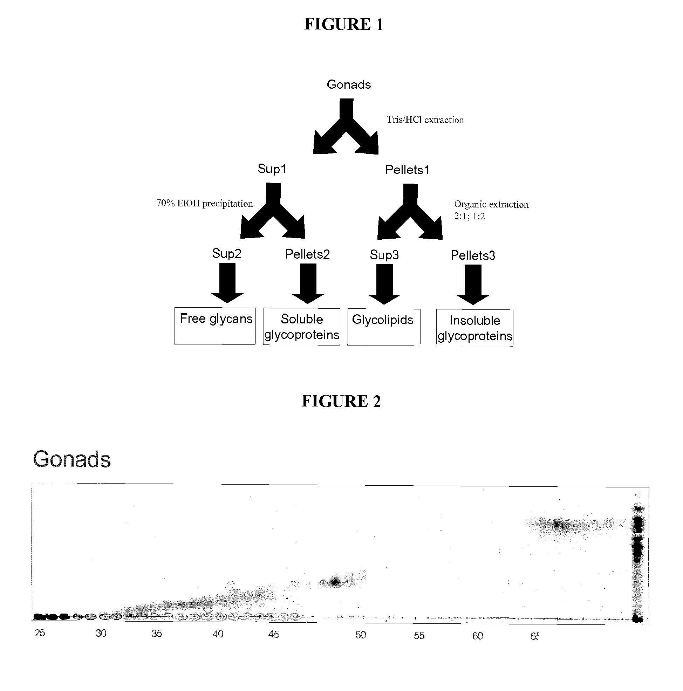

Gonads were collected from freshly collected *Halocynthia roretzi.* Gonads from one or several animals were cut into pieces, suspended in Tris/HCl 10 mM pH8 and minced with polytron. Suspension was centrifuged three times at 35000 RPM to obtain a supernatant **(Sup1)** and pellets (**Pellets1**)**. Sup1** was precipitated with 70% cold ethanol overnight and centrifuged to obtain **Sup2** and **Pellets2. Pellets1** were sequentially extracted with Chloroform/Methanol (2:1) and Chloroform/Methanol (1:2) and centrifuged. Organic fractions were pooled (**Sup3**) and pellets (**Pellets3**) dried. **Pellets2** and **pellets3** were pooled together and correspond to the total protein fraction. The procole is summarized in **figure 1****.**

Total proteins were dissolved in calcium acetate 0.01 M, the pH was adjusted to 8 with a diluted NaOH solution. Dried pronase was added to the solution with a ratio pronase /protein of 1:50. The solution was incubated at 37 °C under stirring. Pronase (1:50 w/w) was added after 6h and 12h and the pH adjusted. Proteolysis was stopped by addition of acetic acid until pH4. Solution was concentrated by rotary evaporator and centrifuged. The supernatant was mixed with 10 volumes of cold ethanol and let to precipitate overnight and the solution centrifuged. The insoluble glycopeptides were collected in the pellets. The pellets were dissolved in 5% trichloroacetic acid in water and centrifuged. The supernatant was collected and the glycopeptides separated on a Biogel P6 (Biorad) gel filtration column irrigated by water. Eluted glycopeptides were visualized by sulfuric/orcinol staining, collected together and dried by lyophilisation.

### Example 2: preparation of a composition comprising N-glycans

Total protein extract was subjected to hydrazinolysis. In a Teflon capped vessel, the freeze-dried protein powder was covered by anhydrous hydrazine and heated at 100°C for 14 h. The excess hydrazine was eliminated by repetitive evaporation in the presence of toluene under a stream of nitrogen, then totally removed by vacuum desiccation over H₂SO₄. The released N-glycans were purified on a Biogel P4 (Biorad) gel filtration column irrigated by water. Eluted N-glycans were visualized by sulfuric/orcinol staining, collected together and dried by lyophilisation.

### Example 3: preparation of a composition comprising O-glycans

O-glycans were released from total protein extract by reductive β-elimination. Dried proteins were incubated in 100 mM NaOH containing 1.0 M sodium borohydride at 37°C for 72 h. The reaction was stopped by addition of Dowex 50×8 cation-exchange resin (25-50 mesh, H⁺ form) at 4°C until pH 6.5 was reached and, after evaporation to dryness, boric acid was distilled as methyl ester in the presence of methanol. Total material was then submitted to cation-exchange chromatography on a Dowex 50×2 column (200-400 mesh, H⁺ form) to remove residual peptides. The released O-glycans were purified on a Biogel P2 (Biorad) gel filtration column irrigated by water. Eluted O-glycans were visualized by sulfuric/orcinol staining, collected together and dried by lyophilisation (**figure 2**).

### Example 4: Analysis of compositions comprising O-glycans or N-glycans

### 4.1 Preparation of N- and O-glycans for the analysis of total glycoproteins

Aliquots (50 mg) of protein fraction were re-suspended in a solution of 6 M guanidinium chloride and 5 mM EDTA in 0.1 M Tris/HCl, pH 8, and agitated for 4 h at 4 °C. Dithiothreitol was then added to a final concentration of 20 mM and incubated for 5 h at 37 °C, followed by addition of iodoacetamide to a final concentration of 50 mM and further incubated overnight in the dark at room temperature. Reduced/alkylated sample was dialysed against water at 4°C for three days and lyophilized. The recovered protein samples were then digested by TPCK treated trypsin overnight at 37°C, in 50 mM ammonium bicarbonate buffer, pH 8.4. Crude peptide fraction was separated from hydrophilic components on a C18 Sep-Pak cartridge (Waters) equilibrated in 5% acetic acid by extensive washing in the same solvent and eluted with a step gradient of 20, 40 and 60% propan-1-ol in 5% acetic acid. Pooled propan-1-ol fraction was dried and subjected to N-glycosidase F (Roche) digestion in 50 mM ammonium bicarbonate buffer pH 8.4, overnight at 37°C. Released N-glycans were separated from peptides using the same C-18 Sep-Pak procedure as described above. To liberate O-glycans, retained peptide fraction from C18 Sep-Pak was submitted to alkaline reductive β-elimination in 100 mM NaOH containing 1.0 M sodium borohydride at 37°C for 72h. The reaction was stopped by addition of Dowex 50×8 cation-exchange resin (25-50 mesh, H⁺ form) at 4°C until pH 6.5 and, after evaporation to dryness, boric acid was distilled as methyl ester in the presence of methanol. Total material was then submitted to cation-exchange chromatography on a Dowex 50×2 column (200-400 mesh, H⁺ form) to remove residual peptides.

### 4.2 Mass spectrometry analyses

For mass spectrometry analyses, the glycan samples were analysed as native and permethylated derivatives. Glycans were permethylated using the NaOH/dimethyl sulfoxide method. Dried glycan were solubilized in a suspension of NaOH in DMSO (10 mg/ml) to which was added 10% (v/v) ICH₃. After 20 minutes sonication, the reaction was stopped by addition of 3 volumes of water on ice. The permethylated derivatives were then extracted in chloroform and repeatedly washed with water. The permethyl derivatives in acetonitrile were mixed 1:1 with 2,5-dihydroxybenzoic acid (DHB) matrix (10 mg/ml in acetonitrile), spotted on the target plate, air-dried and recrystallized on-plate with ethanol whenever necessary. For MS analysis, glycans were analysed on a voyager Elite DE-STR mass spectrometer (Perspective Biosystems, Framingham, MA). Sequence analyses of permethylated and native oligosaccharides by MS/MS were performed on a MALDI-TOF/TOF 4800 Proteomics Analyzer (Applied Biosystems, Framingham, MA, USA) mass spectrometer and a Q-Tof Ultima MALDI instrument (Micromass).

### 4.3 Monosaccharidic assays

Tables 1, 2 and 3 presents respectively the monosaccharidic composition of respectively a composition comprising glycoproteins, a composition comprising O-glycans and a composition comprising N-glycans (Fuc, fucose ; Gal, Galactose ; Man, Mannose; Glc, Glucose; GalNAc, *N-*acétylglucosamine; GalNAc, *N-*acétylgalactosamine, Xyl, Xylose; Rha, Rhamnose).

**Table 1 : monosaccharidic composition of a composition comprising glycoproteins.**

| *Monosaccharides* | *Molar %* |
|---|---|
| Fuc | 20,49 |
| Gal | 6,38 |
| Man | 16,35 |
| GalNAc | 9,71 |
| GlcNAc | 47,08 |
| | 100 |

**Table 2 : monosaccharidic composition of a composition comprising O-glycans.**

| *Monosaccharides* | *Molar %* |
|---|---|
| Fuc | 11,97 |
| Gal | 5,91 |
| Man | 28,48 |
| Glc | 1,73 |
| GalNAc | 7,03 |
| GlcNAc | 43,55 |
| Xyl | 1,33 |
| Rha | 0,00 |
| | 100,00 |

**Table 3 : monosaccharidic composition of a composition comprising N-glycans.**

| *Monosaccharides* | *Molar %* |
|---|---|
| Fuc | 7,85 |
| Gal | 0,00 |
| Man | 41,38 |
| Glc | 0,00 |
| GalNAc | 10,29 |
| GlcNAc | 36,32 |
| Xyl | 0,00 |
| Rha | 4,16 |
| | 100,00 |

### 4.4 Monosaccharide composition analysis

Monosaccharide compositions were determined by GC-MS analysis as per-heptafluorobutyrylated derivatives. Samples were methanolysated in 500 µl of 0.5 M MetOH/HCl at 80°C for 16 h. Reagent was removed under a stream of nitrogen and methyglycosides were per-heptafluorobutyrylated by the addition of 200 µl of dried acetonitrile and 25 µl of heptafluobutyryl-anhydride and the incubation at 100°C for 30 min. After cooling, reagents were removed under a stream of nitrogen and the compounds solubilized in 200 µl of acetonitrile before injection in GC/MS. The GC separation was performed on a Carlo Erba gas chromatograph equipped with a 25 m x 0.32 mm CP-Si15 CB Low bleed/MS capillary column, 0.25 m film phase (Chrompack France, Les Ullis, France). The temperature of the injector was 280 °C and the samples were analyzed using the following temperature program: 90 °C for 3 min then 5°C/min until 260°C. The column was coupled to a Finnigan Automass II mass spectrometer for routine analysis.

Table 4 summaries MS-based identified N-glycans liberated from gonads hydrazinolysis, giving Permeth. *m*/*z* values of [M+Na]⁺ adducts of permethylated oligosaccharides; HN, N-acetlyhexosamine; F, fucose, M, mannose.

**Table 4**

| **Permeth (m/z)** | **HexNAc** | **Fuc** | **Hex** | **Core** |
|---|---|---|---|---|
| 2153,1 | 4 | | | M3GN2 |
| 2327,2 | 4 | 1 | | M3GN2 |
| 2501,2 | 4 | 2 | | M3GN2 |
| 2675,40 | 4 | 3 | | M3GN2 |
| 2851,5 | 4 | 4 | | M3GN2 |
| 2398,1 | 5 | | | M3GN2 |
| 2572,3 | 5 | 1 | | M3GN2 |
| 2746,4 | 5 | 2 | | M3GN2 |
| 2920,5 | 5 | 3 | | M3GN2 |
| 3094,5 | 5 | 4 | | M3GN2 |
| 3268,6 | 5 | 5 | | M3GN2 |
| 2817,4 | 6 | 1 | | M3GN2 |
| 2991,5 | 6 | 2 | | M3GN2 |
| 3165,6 | 6 | 3 | | M3GN2 |
| 3339,7 | 6 | 4 | | M3GN2 |
| 3513,7 | 6 | 5 | | M3GN2 |
| 3687,7 | 6 | 6 | | M3GN2 |
| 3861,7 | 6 | 7 | | M3GN2 |
| 3410,6 | 7 | 3 | | M3GN2 |
| 3584,6 | 7 | 4 | | M3GN2 |
| 3758,6 | 7 | 5 | | M3GN2 |
| 3932,7 | 7 | 6 | | M3GN2 |
| 4106,7 | 7 | 7 | | M3GN2 |
| 1784 | | | 3 | M3GN2 |
| 1988 | | | 4 | M3GN2 |
| 2192 | | | 5 | M3GN2 |
| 2396 | | | 6 | M3GN2 |

Table 5 summaries the MS-based identified O-glycans liberated from gonads by reductive β-elimination, giving natif, *m*/*z* values of [M+Na]⁺ adducts of native oligosaccharides and permethylated *m*/*z* values of [M+Na]⁺ adducts of permethylated oligosaccharides; GlcN, N-acetlyglucosamine; GN, N-acetylgalactosamine; F, fucose.

**Table 5**

| **Natif** | **Permethylated** | **composition-ol** | **Séquences** |
|---|---|---|---|
| 652,1 | 820,6 | HN3 | 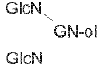 |
| 652,1 | 820,6 | HN3 | 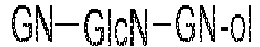 |
| 798,2 | 994,7 | HN3F | 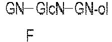 |
| 798,2 | 994,7 | HN3F | 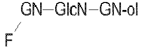 |
| 855,2 | 1065,8 | HN4 | 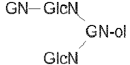 |
| 944,3 | 1168,8 | HN3F2 | 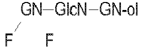 |
| 944,3 | 1168,8 | HN3F2 | 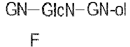 |
| 1001,3 | 1239,9 | HN4F | 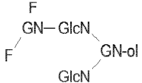 |
| 1001,3 | 1239,9 | HN4F | 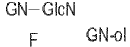 |
| 1147,3 | 1414,1 | HN4F2 | 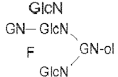 |
| 1147,3 | 1414,1 | HN4F2 | 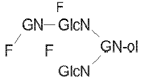 |
| 1204,3 | 1485,1 | HN5F | 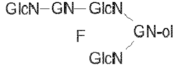 |
| 1204,3 | 1485,1 | HN5F | 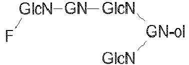 |
| 1350,3 | 1659,2 | HN5F2 | 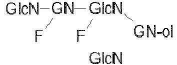 |
| 1350,3 | 1659,2 | HN5F2 | 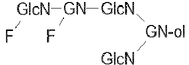 |
| 1496,9 | 1833,7 | HN5F3 | 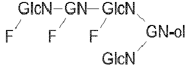 |
| 1496,9 | 1833,7 | HN5F3 | 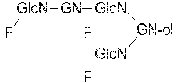 |
| 1643,9 | 2007,7 | HN5F4 | 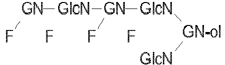 |
| 1643,9 | 2007,7 | HN5F4 | 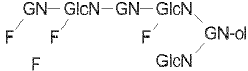 |
| 1700 | 2078,7 | HN6F3 | 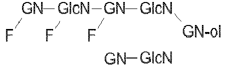 |
| 1846,2 | 2251,7 | HN6F4 | 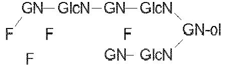 |

### 4.5 Nuclear magnetic resonance analyses

Liquid NMR experiments were performed on a 9.4 T Avance Bruker® spectrometer where ¹H resonates at 400.33 MHz. The experiments were acquired with a Broad Band Inverse self-shielded z-gradient probehead. Spectra were recorded at 300K in D₂O after two chemical exchanges with ²H₂O (Euriso-top, Gif-sur-Yvette, France). Durations and power levels were optimized for each experiment. Spectra were recorded without sample spinning. The chemical shifts were expressed in ppm downfield from the signals of internal acetone ¹H at 2.225 ppm.

### Example 5 : O-Glycans from Halocynthia roretzi inhibit the adherence of Helicobacter pylori to epithelial cells

### 5.1 Bacterial culture and labelling

*Helicobacter pylori* J99 strain was cultured on Wilkins-Chalgren agar plates supplemented with human blood (10% v/v) and antibiotics (10 µg/ml of vancomycin, 10 µg/ml of cefsulodin, 5 µg/ml of trimethoprim, and 10 µg/ml of amphothericin B) under microaerobic conditions. For coculture experiments, *H. pylori* strains were grown at 37°C for 24 h, resuspended in PBS and adjusted to an OD₆₀₀ₙₘ = 1 (corresponding to 2x10⁸ CFU/ml) in PBS before infection. 500 µl of bacterial suspension were spun at 4,000 rpm for 10 min. The bacterial pellet was resuspended in 500 µl of Diluent A and 1 µl of PKH2 dye was added (PKH2 green fluorescent kit, Sigma). After 2 minutes and 30 seconds, the dye solution was diluted with 1 ml of fetal calf serum (FCS). After 2 minutes, 2 ml of F12K medium were added. The suspension was spun at 4,000 rpm for 10 min. The pellet was washed twice with 1 ml of PBS and spun again. After the last wash, the pellet was resuspended in 500 µl of F12K medium.

### 5.2 Human cell culture

The human gastric epithelial cell line AGS was cultured in F12K medium supplemented with 10% FCS, 2mM glutamine, 100U/ml of penicillin and 100 µg/ml of streptomycin. For coculture experiments, cells were grown in absence of antibiotics. The day before the infection, 40,000 cells in 100 µl were plated in 96 wells plate.

### 5.3 Infection and stimulation

Before infection, cells were washed twice with PBS and fresh medium was added. Cells were stimulated with a mix of 10 µl of bacterial suspension (corresponding to 2.10⁶ bacteria) and the corresponding volume of *Halcynthi roretzi* extracts, both of them preincubated for 10 minutes. 75 min post infection, cells were washed twice with PBS to discard non-adherent bacteria and harvested using a trypsin solution. Cells were spun at 2,000 rpm for 5 min and the resulting pellet was resuspended in 150 µl of PBS.

### 5.4 Fluorescence analysis

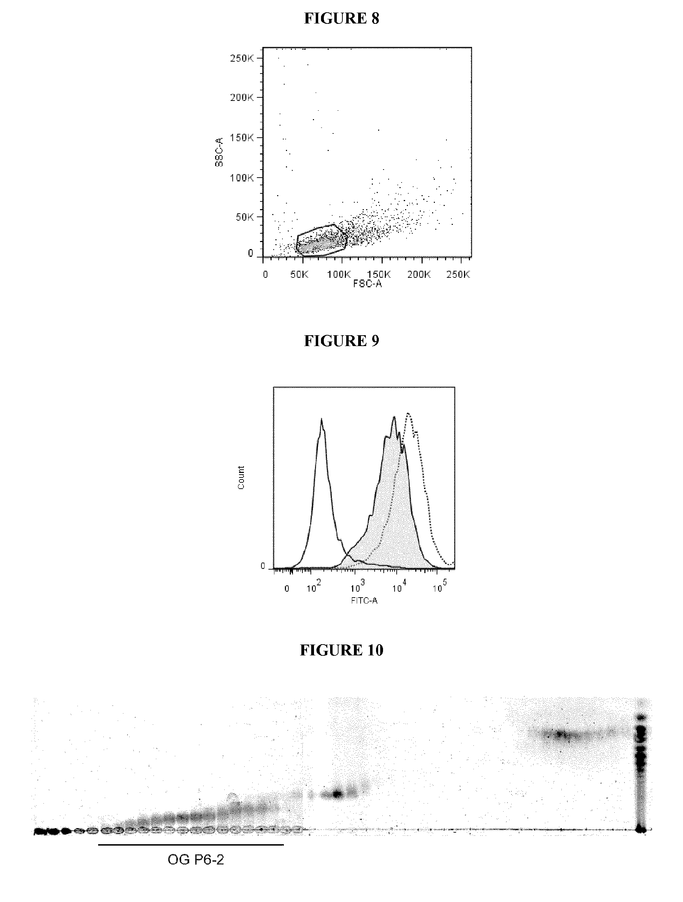

Fluorescence was analysed using a FacsCANTO flow cytometer. The cells were first analysed using light scattering to exclude debris and aggregates. The light scattering correlates with the cell volume (Forward Scatter or FSC) and the internal structure (Side Scatter or SSC), allowing to gate a population of single-cells (Figure 8).

5,000 cells were counted for each condition and results were represented in a histogram (Figure 9). The mean fluorescence intensity (MFI) is correlated with the number of adherent bacteria. As control, fluorescence of non-infected cells, cells infected with unlabelled-*H*. *pylori* or *H. pylori* alone were also measured. Three replicates were analysed for each conditions and results were displayed as the relative MFI of *H. pylori* in presence of *H. roretzi* extracts compared to the MFI of *H. pylori* in absence of other compounds.

### 5.5 Composition analysis

The O-glycans used for the inhibition assays were isolated from *Halocynthia roretzi* gonads by β-elimination of the total protein faction. Isolated O-glycans were separated on a biogel P-6 (Biorad) column irrigated by water and assayed on thin layer chromatography (SilicaGe160 run in butanol/acetic acid/water 2:1:1.5) by sulfuric/orcinol staining. O-glycans collected in the fraction called "OG P6-2" (figure 10) were used for inhibition assays.

The composition of aforesaid fraction was established by a combination of MALDI-MS analyses and MALDI-Q/TOF MS/MS fragmentation analyses on native and permethylated oligosaccharides as a mixture of highly fucosylated O-glycans substituted by the epitope Fuc(α1-4)GalNAc(β1-4)[(Fuc)₀₋₁Fuc(α 1-3)]GlcNAc. The sequences of the major components were established and are summarized in table 6 (HN, N-acetylated hexosamin; F, fucose).

**Table 6**

| *m*/*z* natif | *m*/*z* permethylated | Composition | Sequences | |
|---|---|---|---|---|
| 1496,9 | 1833,7 | HN5F3 | 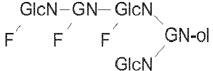 | 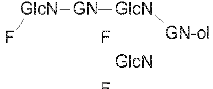 |
| 1643,9 | 2007,7 | HN5F4 | 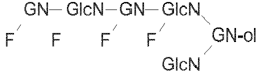 | 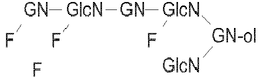 |
| 1700 | 2078,7 | HN6F3 | 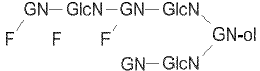 | |
| 1846,2 | 2251,7 | HN6F4 | 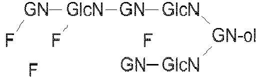 | |

### 5.6 Results

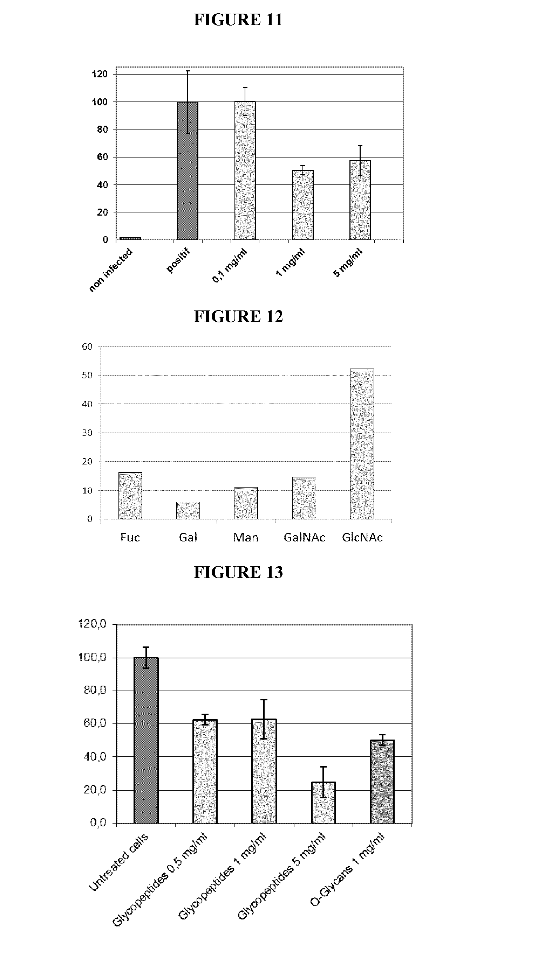

Purified O-glycans released from total proteins by reductive β-elimination exert a strong inhibitory activity toward the adherence of *Helicobacter pylori* to human gastric epithelial cells cultivated in 96 wells plates (40000 cells per well). As observed in figure 11, co-incubation of cells with labeled *Helicobacter pylori* (strain J99) and increasing concentrations of reduced oligosaccharides induced a significant reduction of adhered bacteria compared to untreated cells (positif). Bacteria and oligosaccharides are incubated 10 minutes prior to infection of cells. Cells are then incubated with bacteria and oligosaccharides for 75 minutes, washed with PBS prior to counting of cell-adhered bacteria by FACS. From 1 mg/ml oligosaccharide a 40% reduction of bacterial adherence is observed.

### Example 6 : Total glycopeptides from Halocynthia roretzi inhibit the adherence of Helicobacter pylori to epithelial cells

Paragraphs 5.1-5.4 apply here.

Total protein fraction extracted from gonads of *Halocynthia roretzi* were digested by pronase proteolysis. Glycopeptides were separated from remaining peptides and amino-acids by Biogel P6 (Biorad) gel filtration in water. Monosaccharide analysis of glycopeptide fraction established that it was constituted by 40% of carbohydrate (w/w) including fucose, mannose, galactose, glcNAc and galNAc (figure 12), in accordance with the composition of constituting N-glycans and O-glycans.

Glycopeptides co-incubated with human gastric epithelial cells and labeled *Helicobacter pylori* exerted an inhibitory effect toward bacterial adherence very similar to O-glycans purified from *Halocynthia roretzi* gonads. At identical concentrations (1 mg/ml), total glycopeptide and O-glycan fractions showed inhibitory effect on bacterial adherence between 40 and 50%, compared to untreated cells (figure 13).

### Example 7: The fucosylation of glycopeptides and O-glycans from Halocynthia roretzi is required for the inhibition of the adherence of Helicobacter pylori to epithelial cells

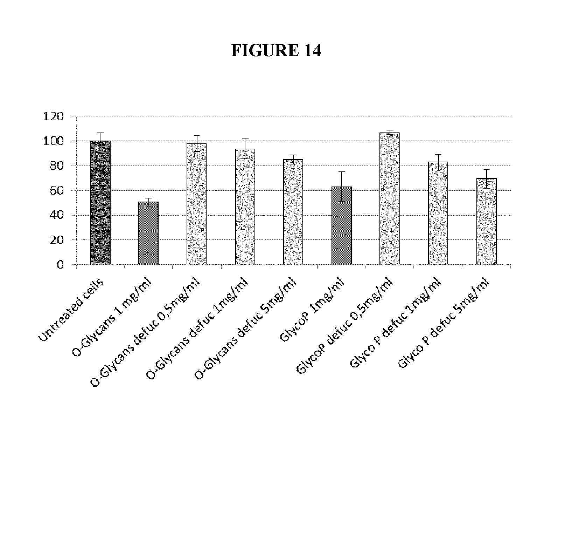

Glycopeptides and O-glycans were chemically defucosylated (Met/HCl 0.05M, 65°C, 45 minutes) and purified by solid phase extraction on a C18 Sepak-cartridge (Waters) washed by water and eluted by acetonitrile/water (25:75) and gel filtration on a Biogel P2 column (Biorad), respectively. The extend of defucosylation was assessed by mass spectrometry and composition analyses. The inhibitory capacities of the resulting defucosylated glycopeptides and O-glycans toward adherence of bacteria to human gastric epithelial cells was compared to their fucosylated counterparts. As shown in figure 14, defucosylation induced a decreased of inhibitory capacities of both O-glycans (by 40% at 1 mg/ml) and glycopeptides (by 20% at 1 mg/ml).

## Claims

1. Pharmaceutical composition comprising, as active substance, at least two glycans or glycopeptides comprising independently of each other at least one motif of structure I: 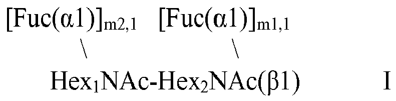 wherein:
m_{1,1} and m_{2,1} are integers equal to 0 or 1,
Hex₁ and Hex₂ represent Glc or Gal,
provided that:
- at least one of m_{1,1} and m_{2,1} is equal to 1, and
- when Hex₁ is Glc, Hex₂ is Gal,
- and when Hex₁ is Gal, Hex₂ is Glc,
in association with a pharmaceutically acceptable vehicle,
in particular a pharmaceutical composition, comprising at least two glycans or glycopeptides comprising at least one motif of structure I-1: 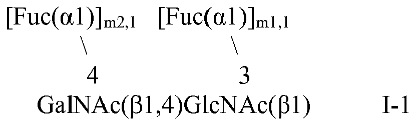
wherein m_{1,1} and m_{2,1} have the meaning indicated above,
or
in particular a pharmaceutical composition comprising at least two glycans or glycopeptides comprising at least one motif of structure I-2: 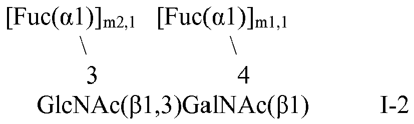
wherein m_{1,1} and m_{2,1} have the meaning indicated above,
or
in particular a pharmaceutical composition comprising at least two glycans or glycopeptides comprising at least one motif of structure I, wherein m_{1,1} is equal to 1 and m_{2,1} is equal to 0, of particular formula II: 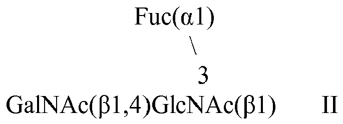 or
in particular a pharmaceutical composition comprising at least two glycans or glycopeptides comprising at least one motif of structure I, wherein m_{1,1} is equal to 0 and m_{2,1} is equal to 1, of particular formula III: 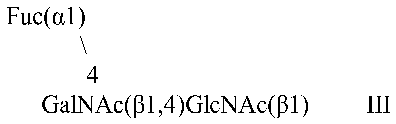 or
in particular a pharmaceutical composition comprising at least two glycans or glycopeptides comprising at least one motif of structure I, wherein m_{1,1} is equal to 0 and m_{2,1} is equal to 1, of particular formula III-2: 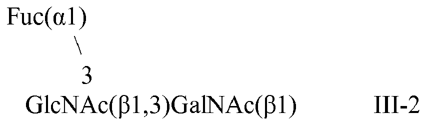 or
in particular a pharmaceutical composition comprising at least two glycans or glycopeptides comprising at least one motif of structure I, wherein m_{1,1} and m_{2,1} are equal to 1, of particular formula IV: 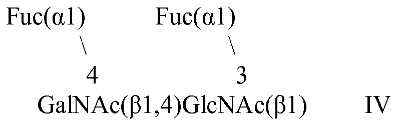 or
in particular a pharmaceutical composition comprising at least two glycans or glycopeptides comprising at least one motif of structure I, wherein m_{1,1} and m_{2,1} are equal to 1, of particular formula IV-2: 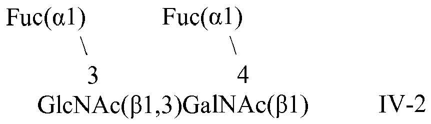 or
in particular a pharmaceutical composition comprising at least two glycans or glycopeptides comprising at least one motif of structure V: 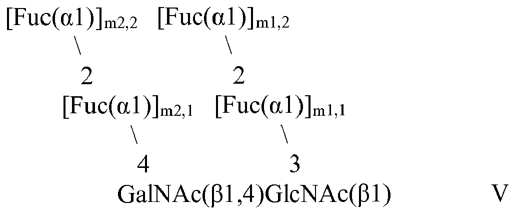 wherein:
m_{1,1}; m_{1,2}; m_{2,1} and m_{2,2} are integers equal to 0 or 1,
provided that at least one of m_{1,1}; m_{1,2}; m_{2,1} and m_{2,2} is equal to 1.

2. Pharmaceutical composition according to claim 1, comprising at least two glycopeptides comprising independently of each other a motif of structure VI: 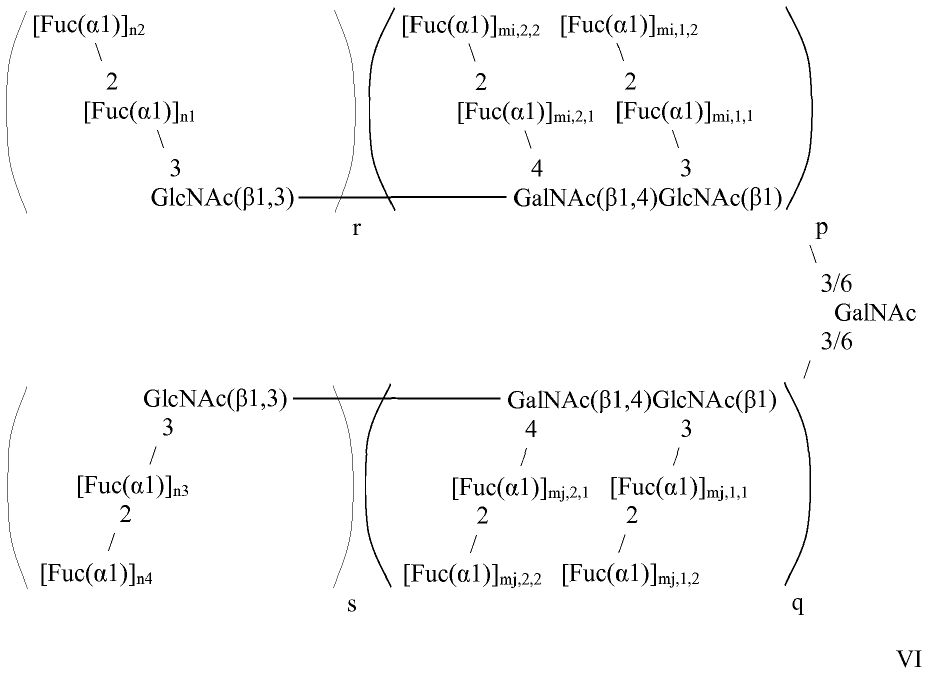 wherein:
p and q are integers equal to 0, 1, 2 or 3,
r and s are integers equal to 0 or 1,
i is an integer:
- equal to 0 when p is equal to 0,
- equal to 1 when p is equal to 1,
- and varying from 1 to 2 when p is equal to 2,
j is an integer:
- equal to 0 when q is equal to 0,
- equal to 1 when q is equal to 1,
- and varying from 1 to 2 when q is equal to 2,
m_{i,1,1}; m_{i,1,2}; m_{i,2,1}; m_{i,2,2}; m_{j,1,1}; m_{j,1,2}; m_{j,2,1}; m_{j,2,2}; n₁; n₂; n₃ and n₄ are integers equal to 0 or 1,
provided that p + q > 0, and:
- p > 0 and there is at least one i such as at least one of m_{i,1,1}; m_{i,1,2}; m_{i,2,1} and m_{i,2,2} is equal to 1, and/or
- q > 0 and there is at least one j such as at least one of m_{j,1,1}; m_{j,1,2}; m_{j,2,1} and m_{j,2,2} is equal to 1, and/or
- p > 0, r > 0 and there at least one of m_{i,1,1}; m_{i,1,2}; m_{i,2,1}; m_{i,2,2}; n₁; n₂; n₃ and n₄ is equal to 1, and/or
- q > 0, s > 0 and there at least one of m_{i,1,1}; m_{i,1,2}; m_{i,2,1}; m_{i,2,2}; n₁; n₂; n₃ and n₄ is equal to 1,
in particular a pharmaceutical composition comprising at least two glycopeptides comprising independently of each other a motif of structure VII: 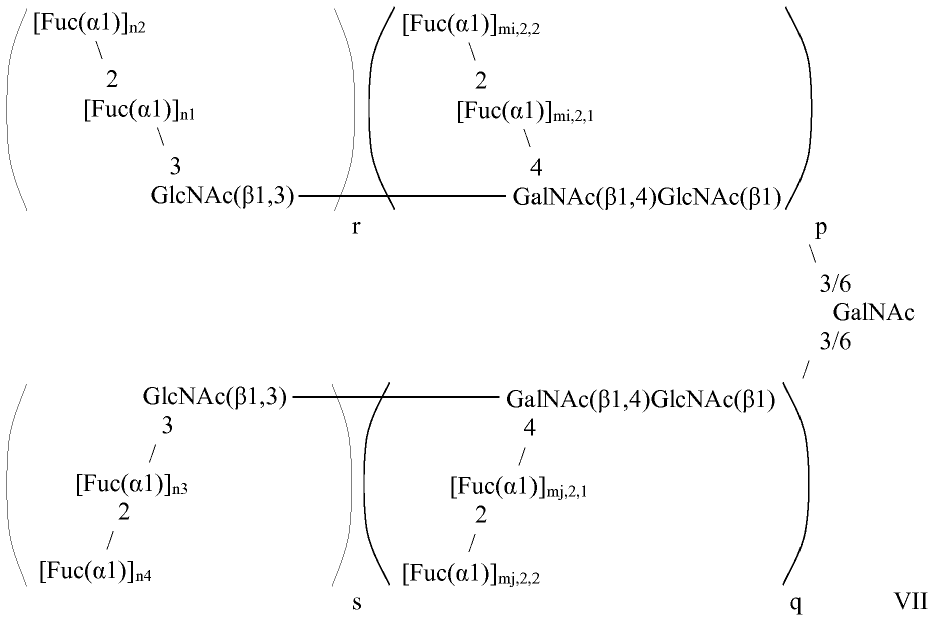
wherein r, p, q, s, m_{i,2,1}; m_{i,2,2}; m_{j,2,1}; m_{j,2,2}; n₁; n₂; n₃ and n₄ have the meaning indicated above,
or
in particular a pharmaceutical composition comprising at least two glycopeptides comprising independently of each other a motif of structure VIII: 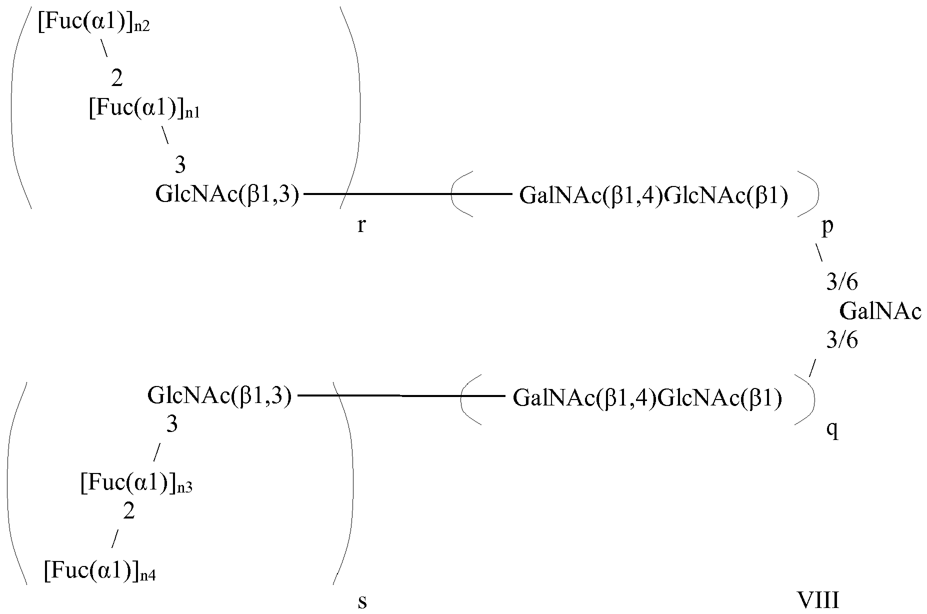
wherein r, p, s, q, n₁; n₂; n₃ and n₄ have the meaning indicated above,
or
in particular a pharmaceutical composition comprising at least two glycopeptides comprising independently of each other a motif of structure IX: 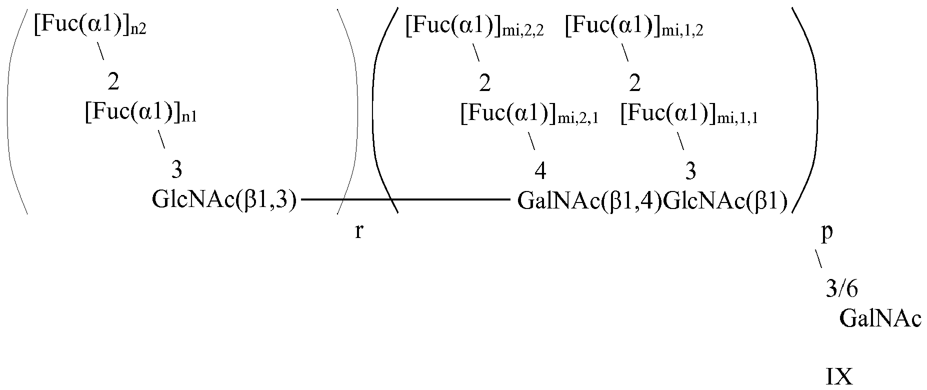
wherein r, p, m_{i,1,1}; m_{i,1,2}; m_{i,2,1}; m_{i,2,2}; n₁ and n₂ have the meaning indicated above,
or
in particular a pharmaceutical composition comprising at least two glycopeptides comprising independently of each other a motif of structure X: 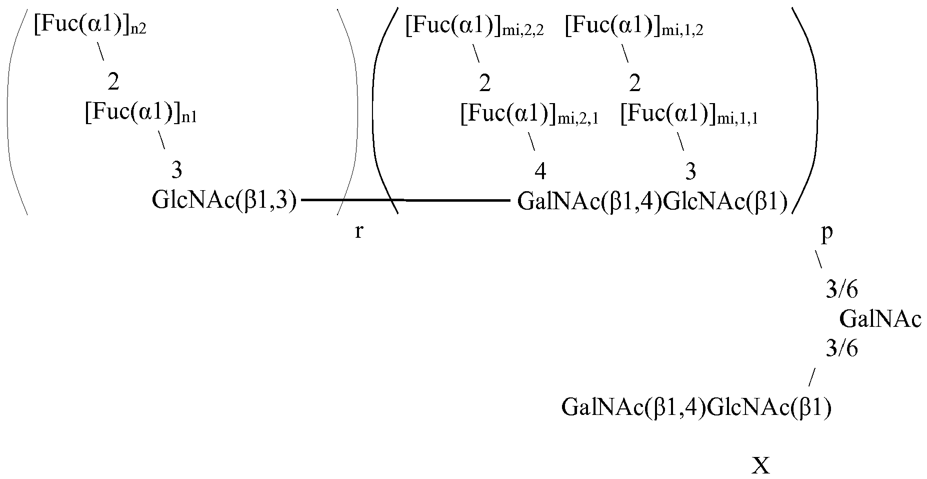
wherein r, p, m_{i,1,1}; m_{i,1,2}; m_{i,2,1}; m_{i,2,2}; n₁ and n₂ have the meaning indicated above,
or
in particular a pharmaceutical composition comprising at least two glycopeptides comprising independently of each other a motif of structure XI: 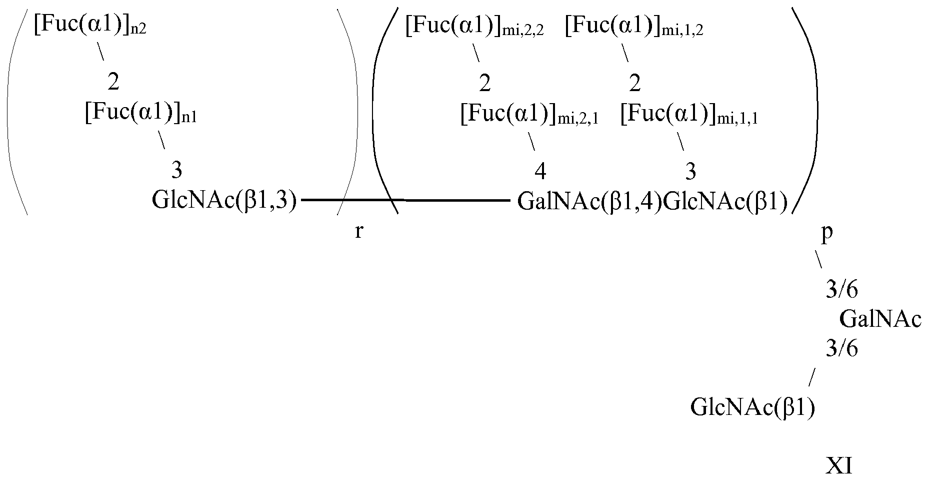
wherein r, p, m_{i,1,1}; m_{i,1,2}; m_{i,2,1}; m_{i,2,2}; n₁ and n₂ have the meaning indicated above,
or
in particular a pharmaceutical composition comprising at least two glycopeptides comprising independently of each other a motif of structure VI, of the following particular formula XII: 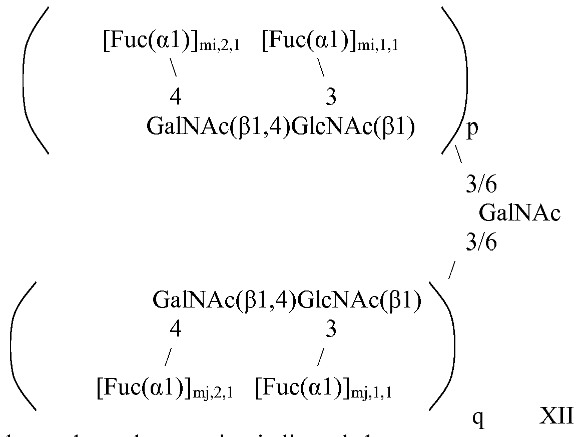
wherein p, q, m_{i,1,1}; m_{i,2,1}; m_{j,1,1} and m_{j,2,1} have the meaning indicated above,
or
in particular a pharmaceutical composition comprising at least two glycopeptides comprising independently of each other a motif of structure VI, of the following particular formula XIII: 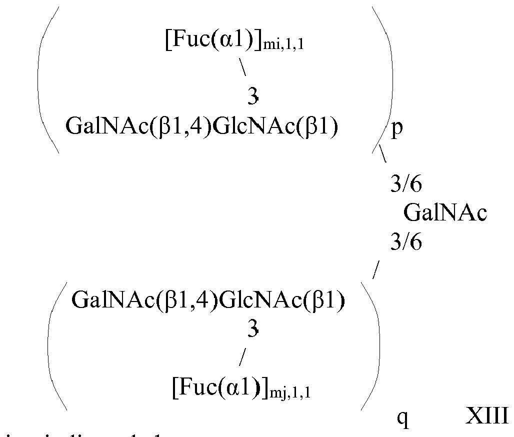
wherein p, q, m_{i,1,1} and m_{j,1,1} have the meaning indicated above,
or
in particular a pharmaceutical composition comprising at least two glycopeptides comprising independently of each other a motif of structure VI, of the following particular formula XIV: 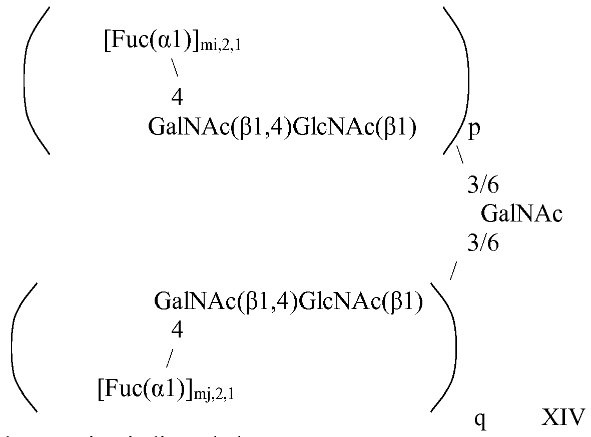
wherein p, q, m_{i,2,1} and m_{j,2,1} have the meaning indicated above.

3. Pharmaceutical composition according to claim 1, comprising at least two glycans of structure VI-2: 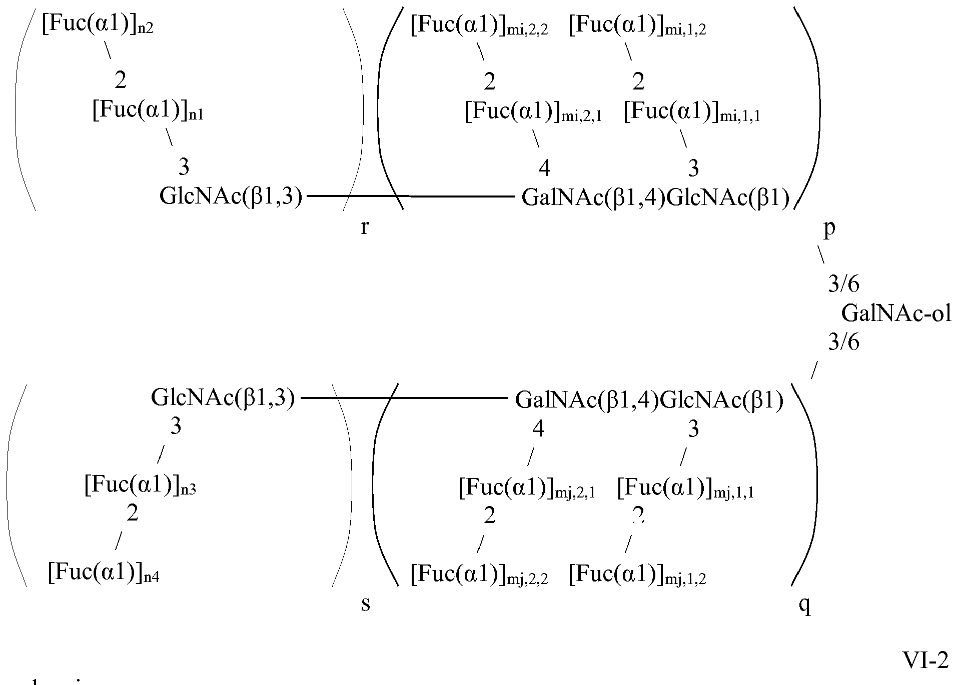 wherein:
p and q are integers equal to 0, 1, 2 or 3,
r and s are integers equal to 0 or 1,
i is an integer:
- equal to 0 when p is equal to 0,
- equal to 1 when p is equal to 1,
- and varying from 1 to 2 when p is equal to 2,
j is an integer:
- equal to 0 when q is equal to 0,
- equal to 1 when q is equal to 1,
- and varying from 1 to 2 when q is equal to 2,
m_{i,1,1}; m_{i,1,2}; m_{i,2,1}; m_{i,2,2}; m_{i,1,1}; m_{j,1,2}; m_{j,2,1}; m_{j,2,2}; n₁; n₂; n₃ and n₄ are integers equal to 0 or 1,
provided that p + q > 0, and:
- p > 0 and there is at least one i such as at least one of m_{i,1,1}; m_{i,1,2}; m_{i,2,1} and m_{i,1,2,2} is equal to 1, and/or
- q > 0 and there is at least one j such as at least one of m_{i,1,1}; m_{j,1,2}; m_{j,2,1} and m_{j,2,2} is equal to 1, and/or
- p > 0, r > 0 and there at least one of m_{i,1,1}; m_{i,1,2}; m_{i,2,1}; m_{i,2,2}; n₁; n₂; n₃ and n₄ is equal to 1, and/or
- q > 0, s > 0 and there at least one of m_{i,1,1}; m_{i,1,2}; m_{i,2,1}; m_{i,2,2}; n₁; n₂; n₃ and n₄ is equal to 1.
in particular a pharmaceutical composition comprising at least two glycans of structure VII-2: 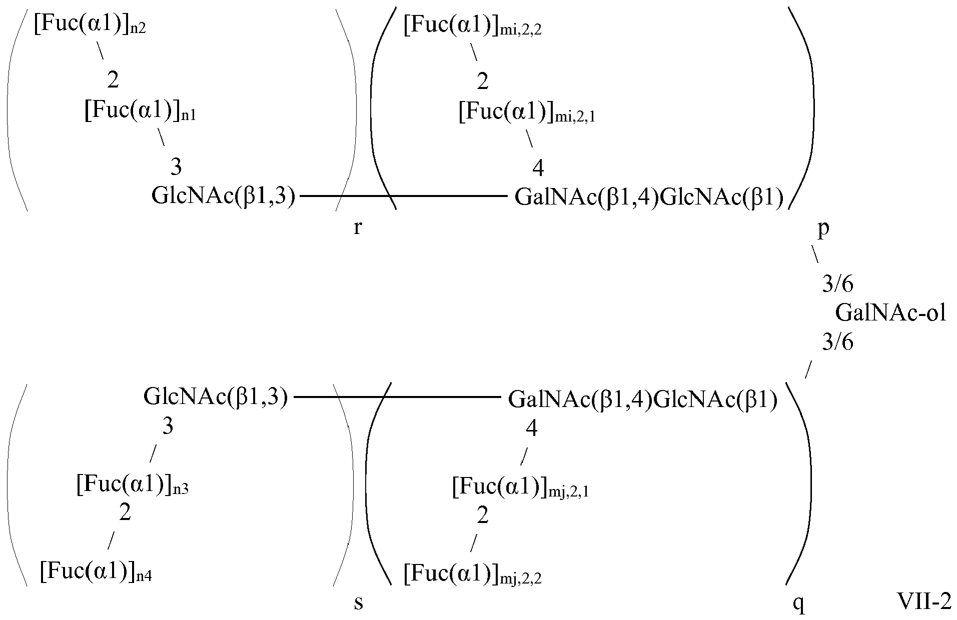
wherein r, p, s, q, m_{i,2,1}; m_{i,2,2}; m_{i,2,1}; m_{j,2,2}; n₁; n₂; n₃ and n₄ have the meaning indicated above,
or
in particular a pharmaceutical composition comprising at least two glycans of structure VIII-2: 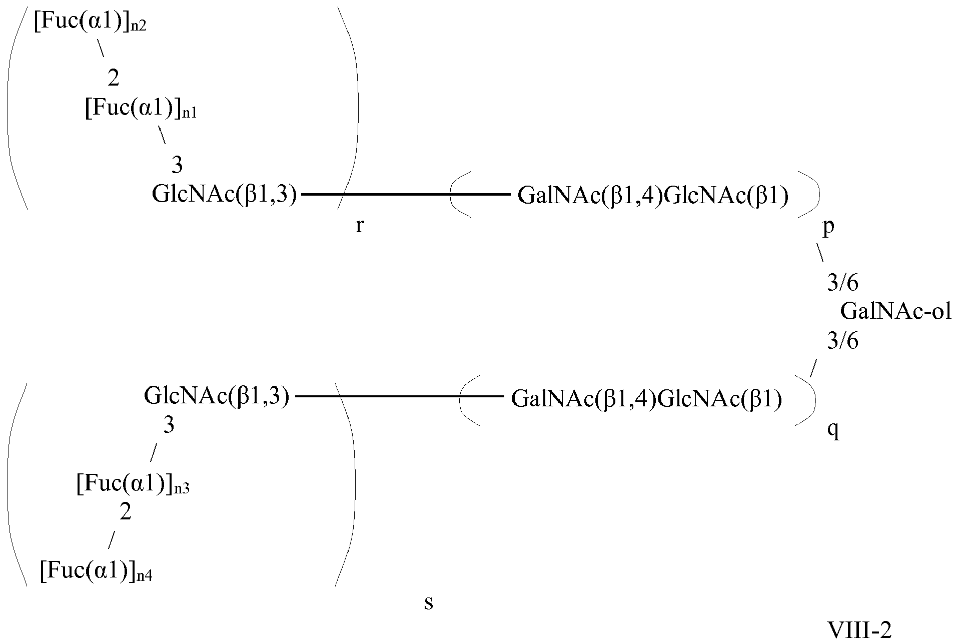
wherein r, p, s, q, n₁; n₂; n₃ and n₄ have the meaning indicated above,
or
in particular a pharmaceutical composition comprising at least two glycans of structure IX-2 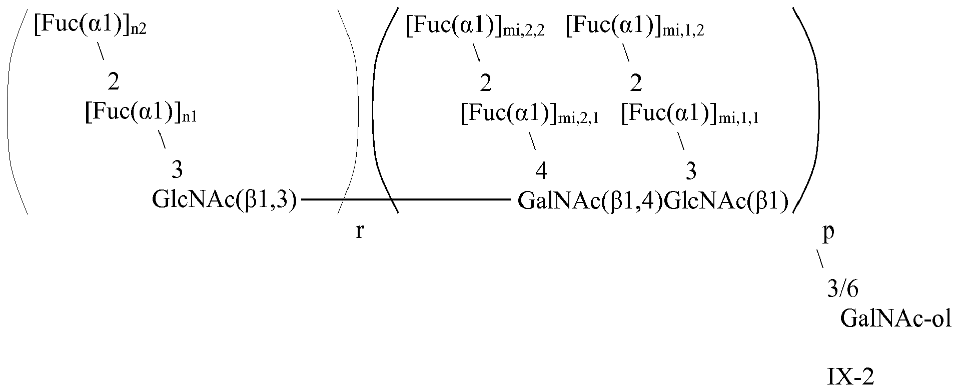
wherein r, p, m_{i,1,1}; m_{i,1,2}; m_{i,2,1}; m_{i,2,2}; n₁ and n₂ have the meaning indicated above,
or
in particular a pharmaceutical composition comprising at least two glycans of structure X-2: 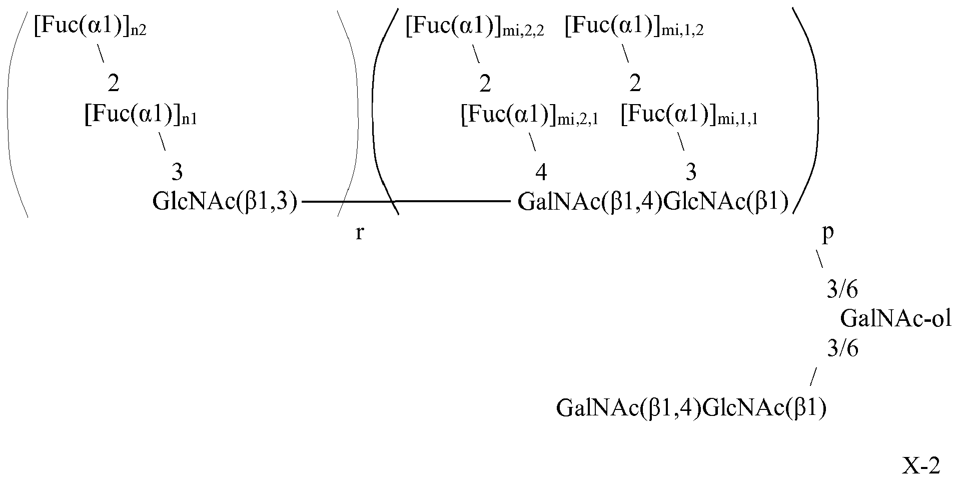
wherein r, p, m_{i,1,1}; m_{i,1,2}; m_{i,1,1} ; m_{i,2,2}; n₁ and n₂ have the meaning indicated above,
or
in particular a pharmaceutical composition comprising at least two glycans of structure XI-2: 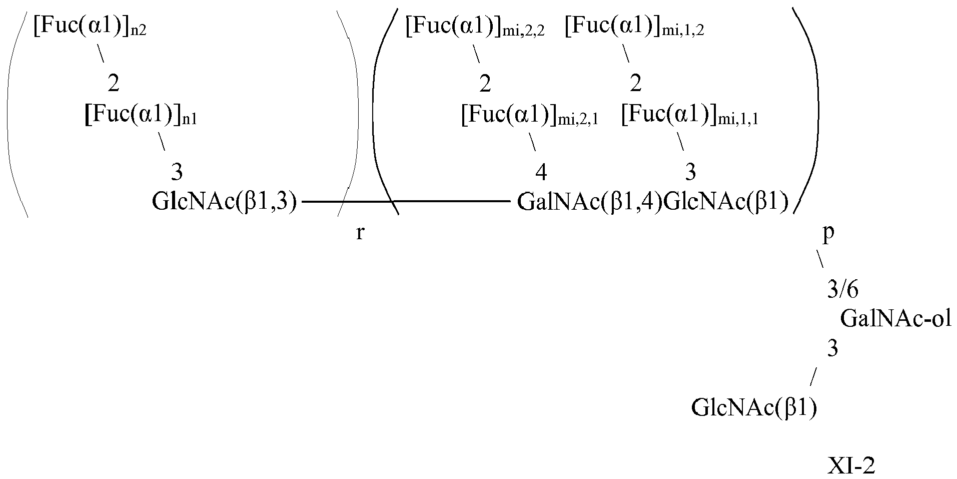
wherein r, p, m_{i,1,1}; m_{i,1,2}; m_{i,1,1} ; m_{i,2,2}; n₁ and n₂ have the meaning indicated above,
or
in particular a pharmaceutical composition comprising at least two glycans of structure VI-2, of the following particular formula XII-2: 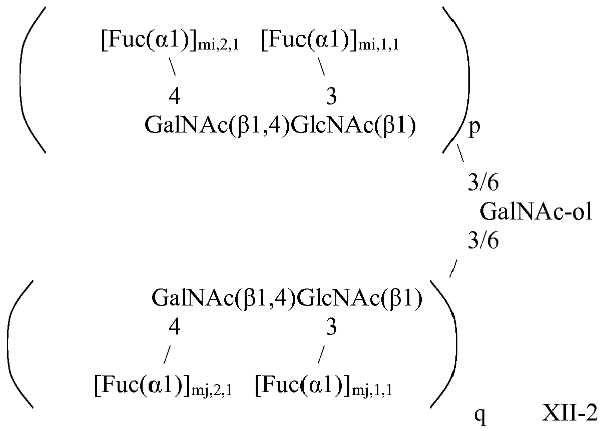
wherein p, q, m_{i,1,1}; m_{i,2,1} ; m_{j,1,1} and m_{j,2,1} have the meaning indicated above,
or
in particular a pharmaceutical composition comprising at least two glycans of structure VI-2, of the following particular formula XIII-2: 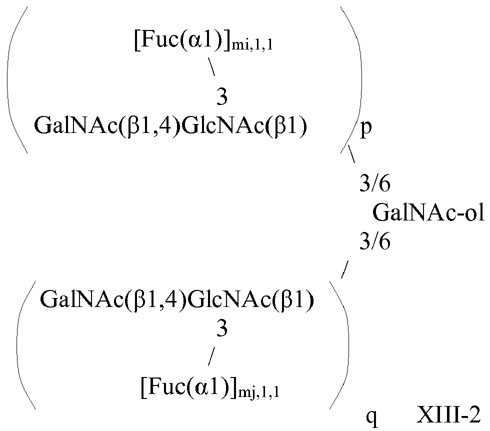
wherein p, q, m_{i,1,1} and m_{j,1,1} have the meaning indicated above,
or
in particular a pharmaceutical composition comprising at least two glycans of structure VI-2, of the following particular formula XIV-2: 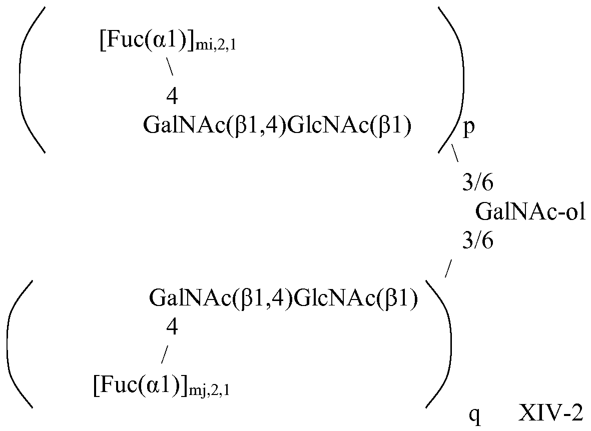
wherein p, q, m_{i,2,1} and m_{j,2,1} have the meaning indicated above.

4. Pharmaceutical composition, according to claim 1 or 3, comprising at least two glycans, said glycans being chosen in the group comprising:

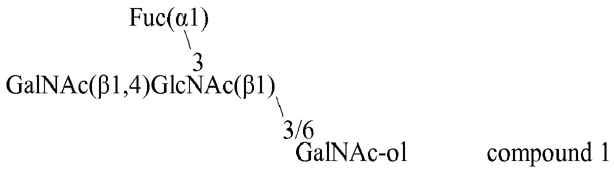

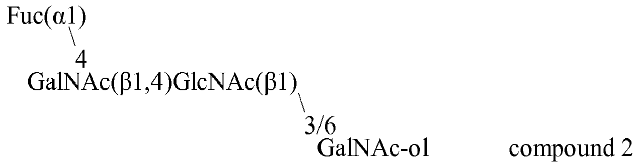

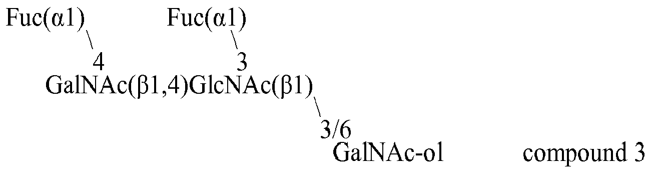

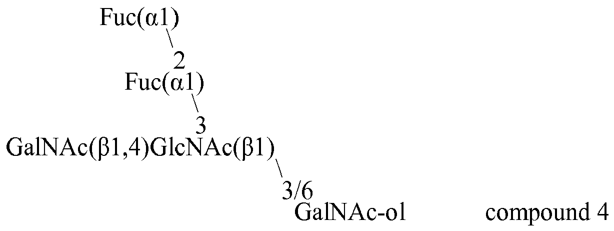

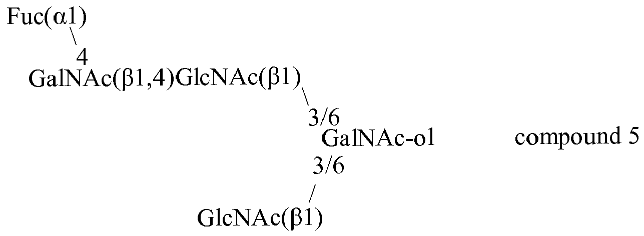

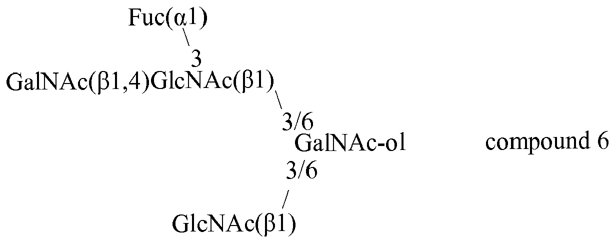

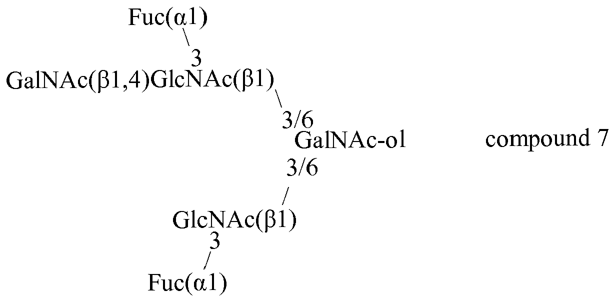

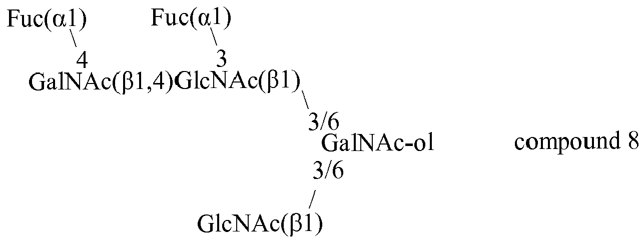

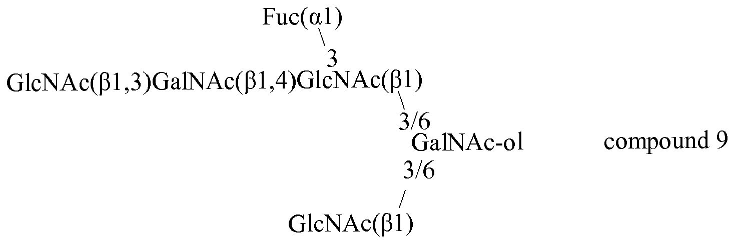

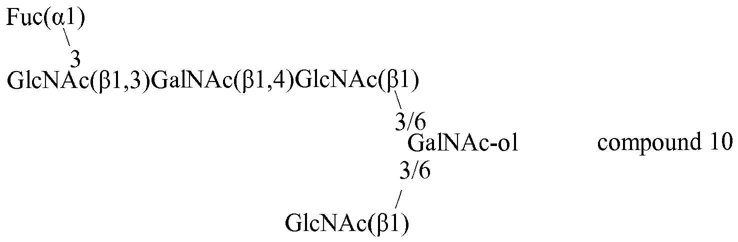

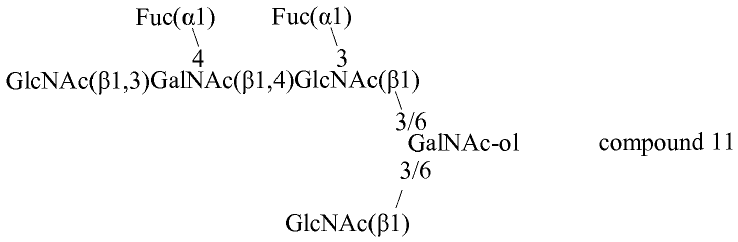

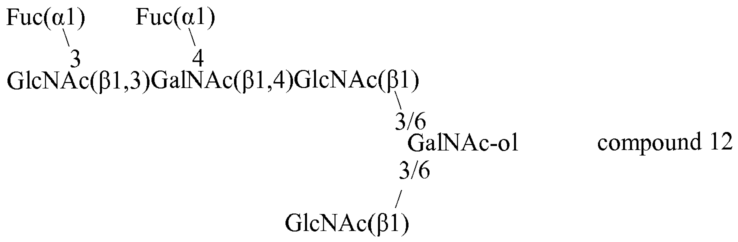

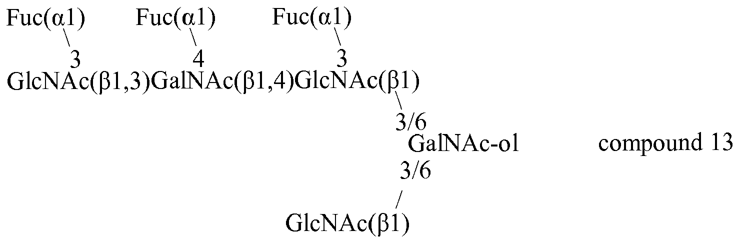

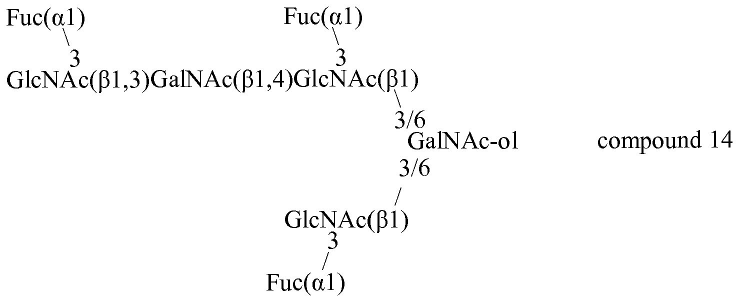

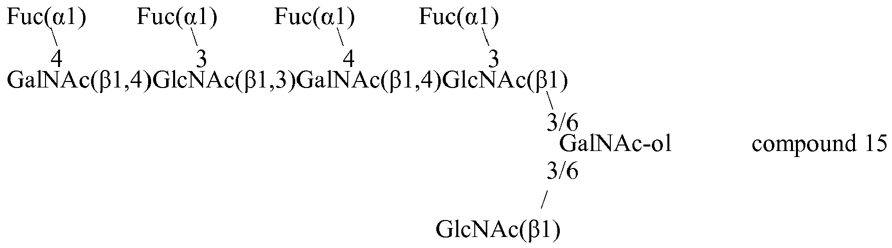

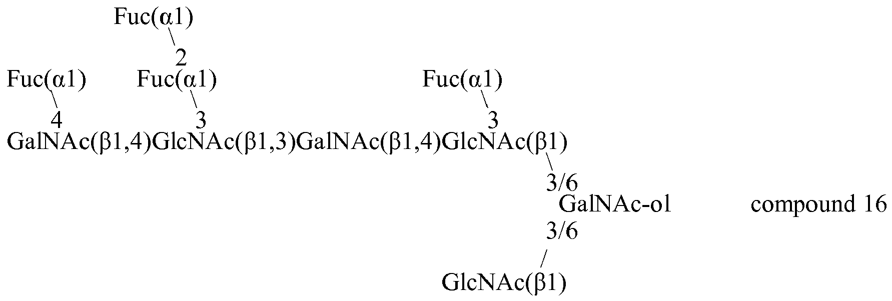

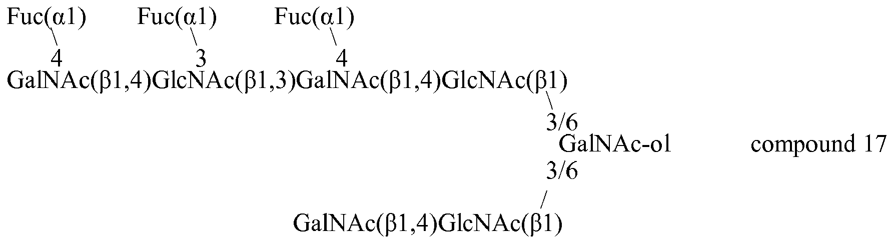

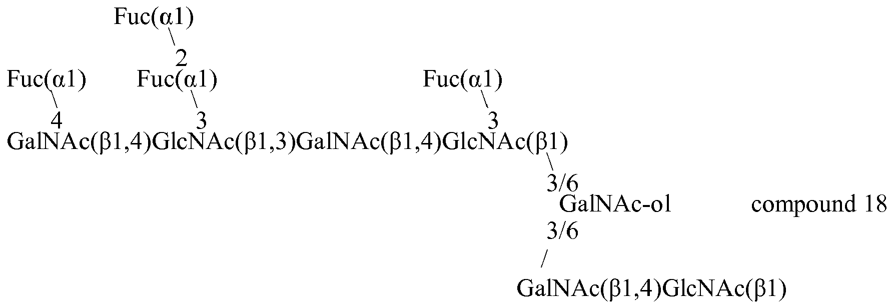

5. Pharmaceutical composition according to claim 1, comprising at least two glycans of structure XV or glycopeptides comprising independently of each other a motif of structure XV: 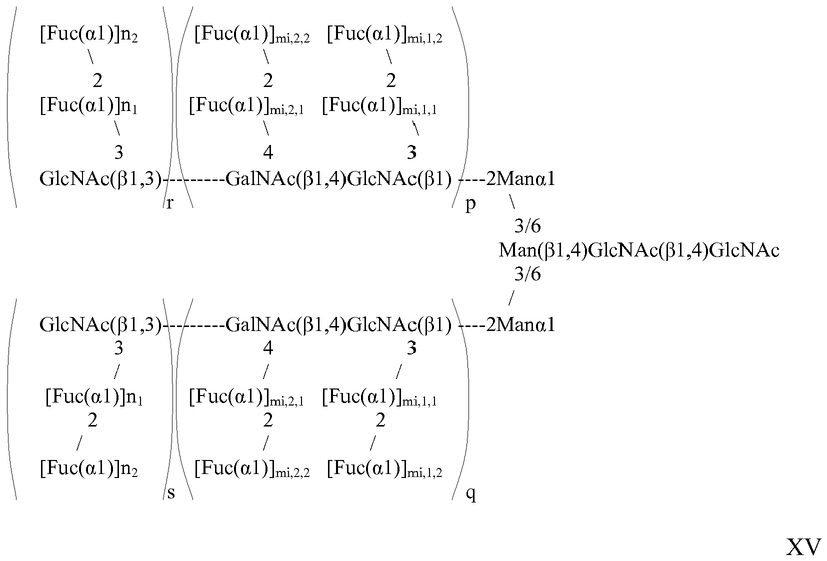 wherein:
p and q are integers equal to 0, 1, 2 or 3,
i is an integer:
- equal to 0 when p is equal to 0,
- equal to 1 when p is equal to 1,
- and varying from 1 to 2 when p is equal to 2,
j is an integer:
- equal to 0 when q is equal to 0,
- equal to 1 when q is equal to 1,
- and varying from 1 to 2 when q is equal to 2,
M_{i,1,1}; M_{i,1,2}; M_{i,2,1} ; M_{i,2,2}; m_{j,1,1}; m_{i,1,2}; m_{j,2,1} and m_{j,2,2} are integers equal to 0 or 1,
provided that:
p + q > 0, and:
- p > 0 and there is one i such as one of m_{i,1,1}; m_{i,1,2}; m_{i,2,1} and m_{i,2,2} is equal to 1, and/or
- q > 0 and there is one j such as one of m_{j,1,1;} m_{j,1,2}; m_{j,2,1} and m_{j,2,2} is equal to 1,
in particular a pharmaceutical comprising at least two glycans of structure XV or glycopeptides comprising independently of each other a motif of structure XV, of particular structure XVI: 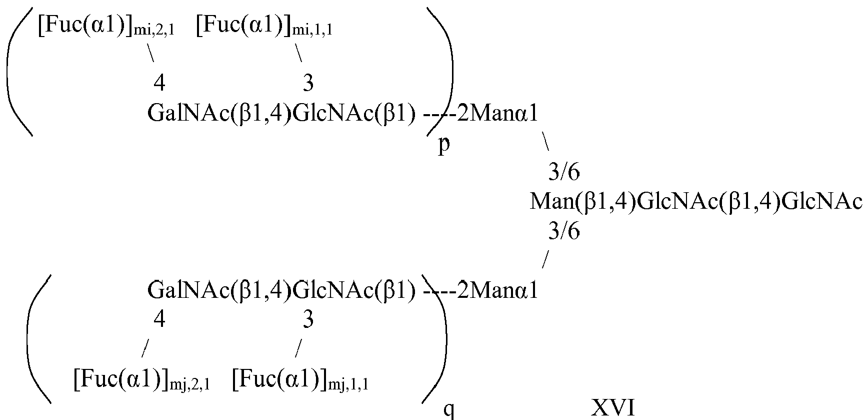
wherein p, q, m_{i,1,1}; m_{i,2,1} ; m_{j,1,1} and m_{j,2,1} have the meaning indicated above,
or
in particular a pharmaceutical composition comprising at least two glycans of structure XV or glycopeptides comprising independently of each other a motif of structure XV, of particular structure XVII: 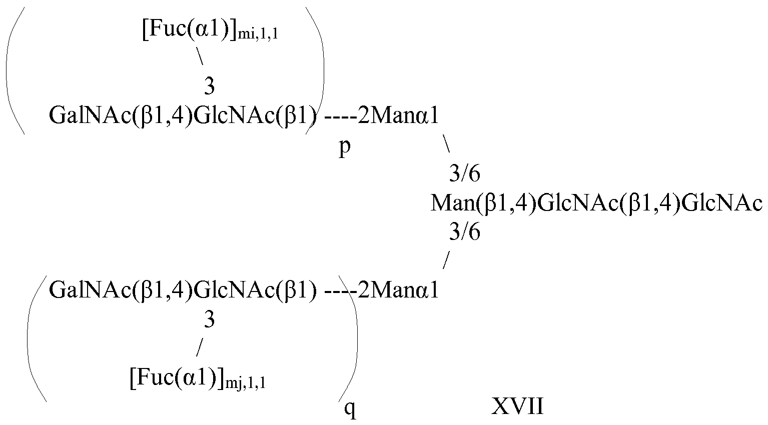
wherein p, q, m_{i,1,1} and m_{j,1,1} have the meaning indicated above,
or
in particular a pharmaceutical composition comprising at least two glycans of structure XV or glycopeptides comprising independently of each other a motif of structure XV, of particular structure XVIII: 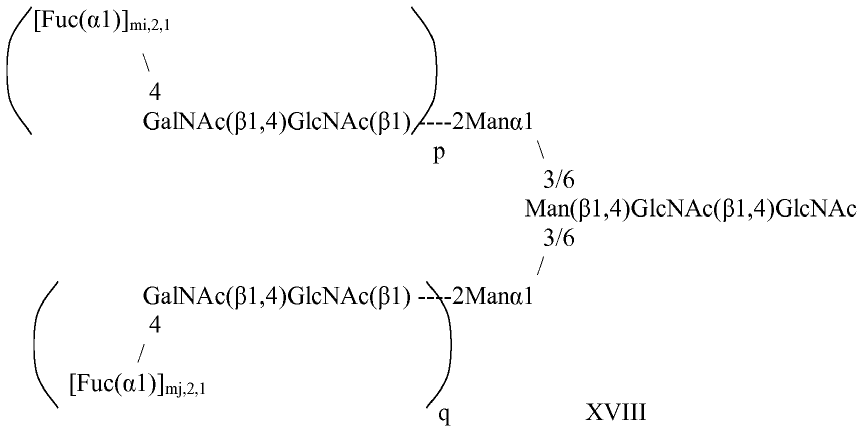
wherein p, q, m_{i,2,1} and m_{j,2,1} have the meaning indicated above.

6. Pharmaceutical composition, according to claim 1 or 5, comprising at least two glycanes of the following formulae:

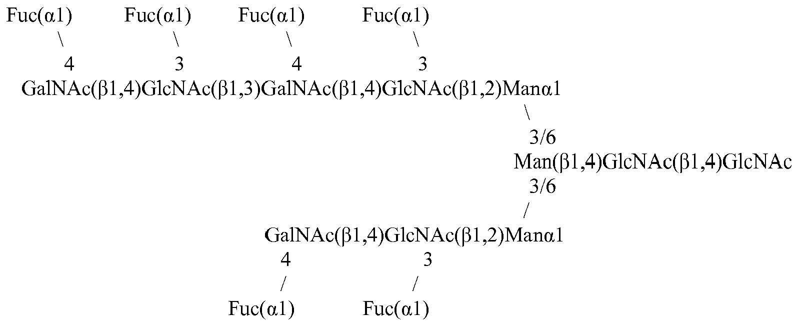

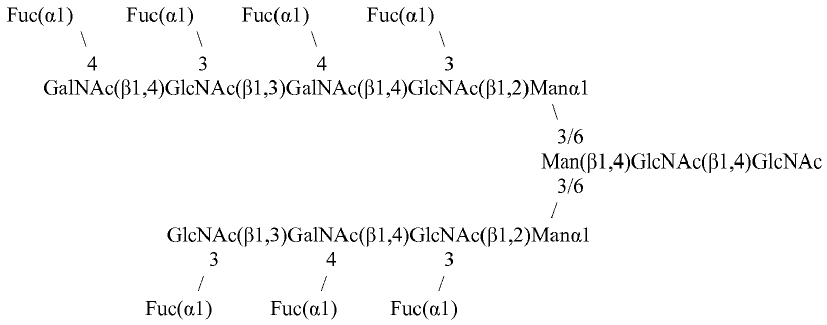

7. Pharmaceutical composition, according to claim 1 or 5, comprising at least two glycopeptides comprising independently of each other a motif, the structure of which being chosen in the group comprising:

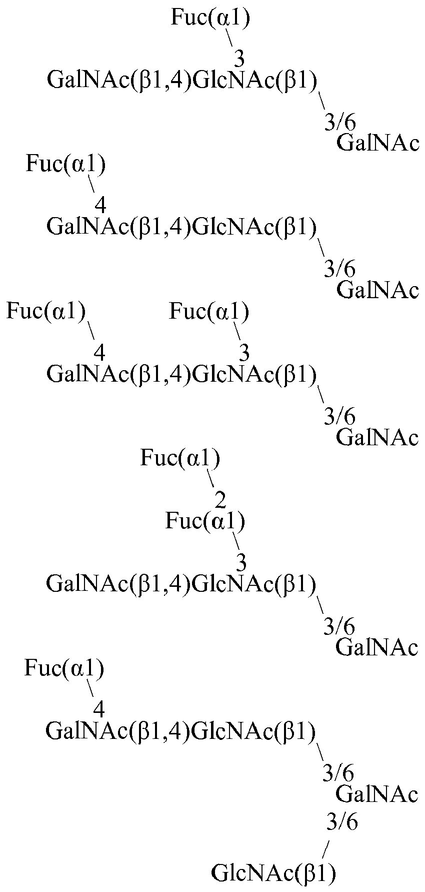

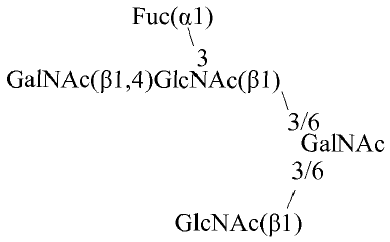

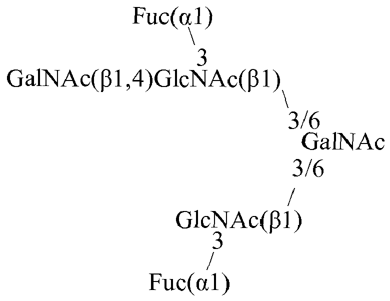

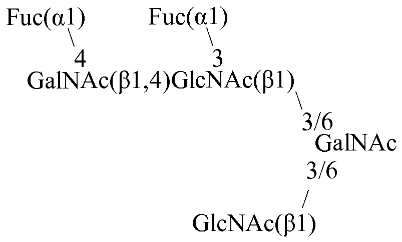

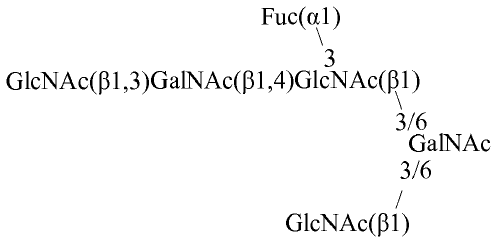

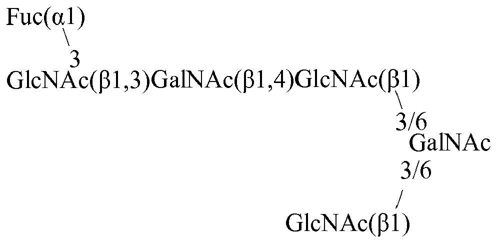

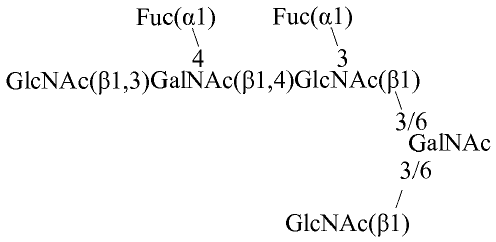

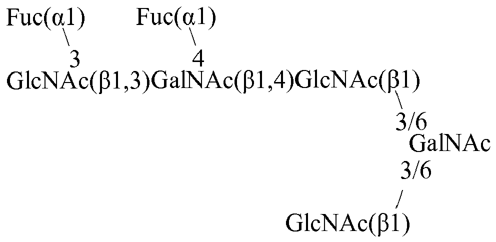

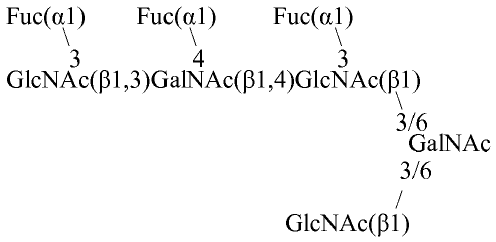

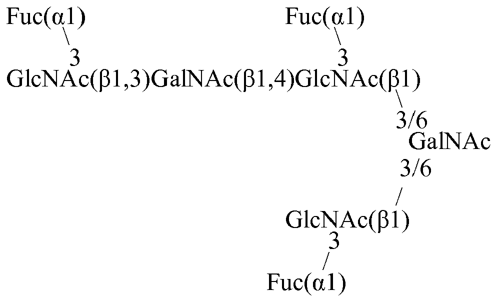

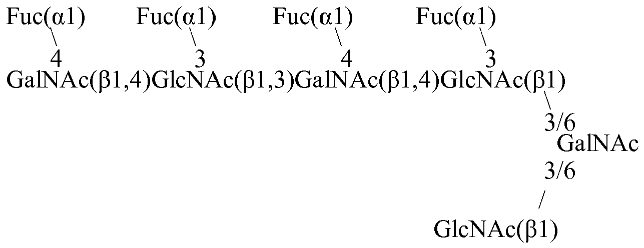

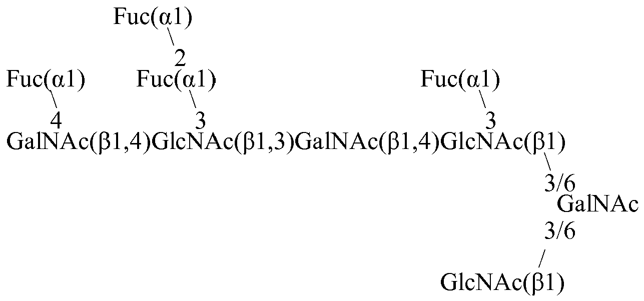

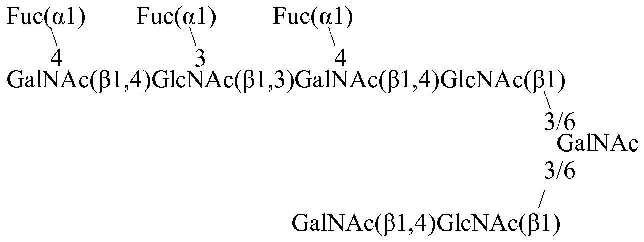

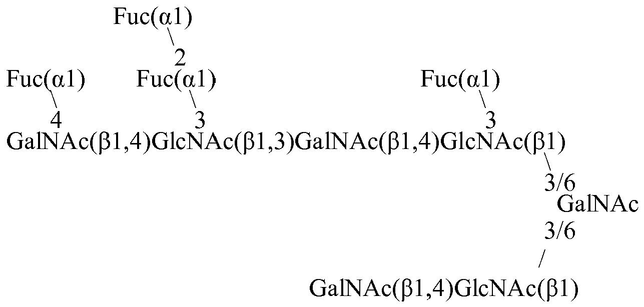

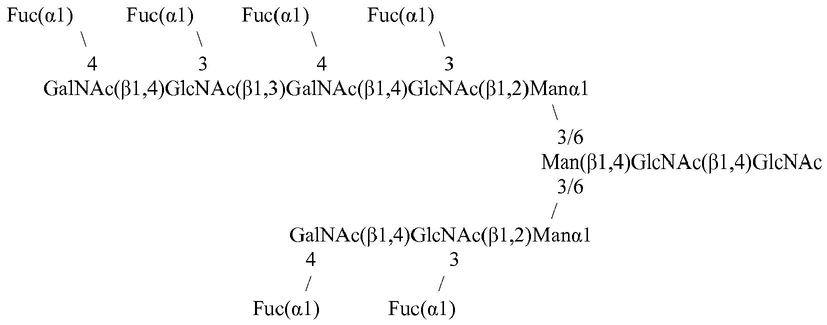

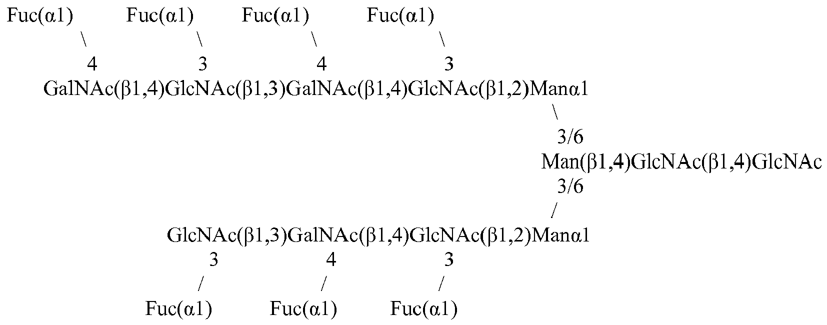

8. Pharmaceutical composition, according to anyone of claims 1 to 7, administrable by oral route at a dose comprised from about 10 mg/kg to about 200 mg/kg of body weight, or administrable by intravenous route at a dose comprised from about 5 µg/kg to about 50 mg/kg.

9. Composition, for use as anti-adherence drug to treat infectious disease(s), comprising at least two glycans or glycopeptides comprising independently of each other at least one motif of structure I, according to anyone of claims 1 to 7.

10. Composition, according to claim 9, for use as anti-adherence drug to treat disease(s) caused by bacteria of the species *Heliobacter pylori,* said diseases belonging to the group comprising:
- infection of the stomach,
- dyspepsia,
- gastritis,
- ulcers, in particular ulcers of the stomach or the duodenum.

11. Composition comprising at least two glycans or glycopeptides comprising independently of each other at least one motif of structure I-3: 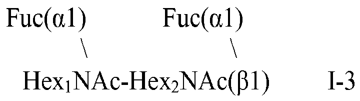 wherein Hex₁ and Hex₂ represent Glc or Gal,
provided that:
- when Hex₁ is Glc, Hex₂ is Gal,
- and when Hex₁ is Gal, Hex₂ is Glc,
in particular a pharmaceutical composition comprising at least two glycans or glycopeptides comprising at least one motif of structure I, wherein m_{1,1} and m₂,₁ are equal to 1, of particular formula IV: 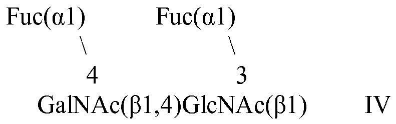 or
in particular a pharmaceutical composition comprising at least two glycans or glycopeptides comprising at least one motif of structure I, wherein m_{1,1} and m_{2,1} are equal to 1, of particular formula IV-2: 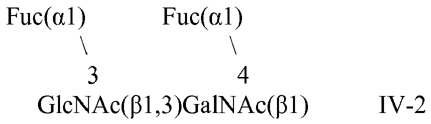 or
in particular a pharmaceutical composition comprising at least two glycans or glycopeptides comprising at least one motif of structure V: 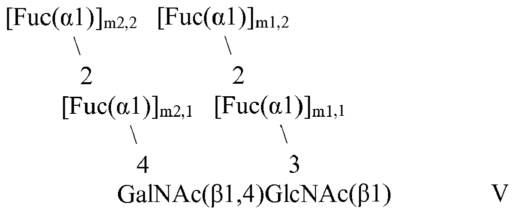 wherein:
m_{1,1}; m_{1,2}; m₂,₁ and m₂,₂ are integers equal to 0 or 1,
provided that:
• at least one of m_{1,1} and M_{1,2} is equal to 1, and
• at least one of m₂,₁ and m_{2,2} is equal to 1,

12. Composition according to claim 11, comprising at least two glycopeptides comprising independently of each other a motif of structure VI: 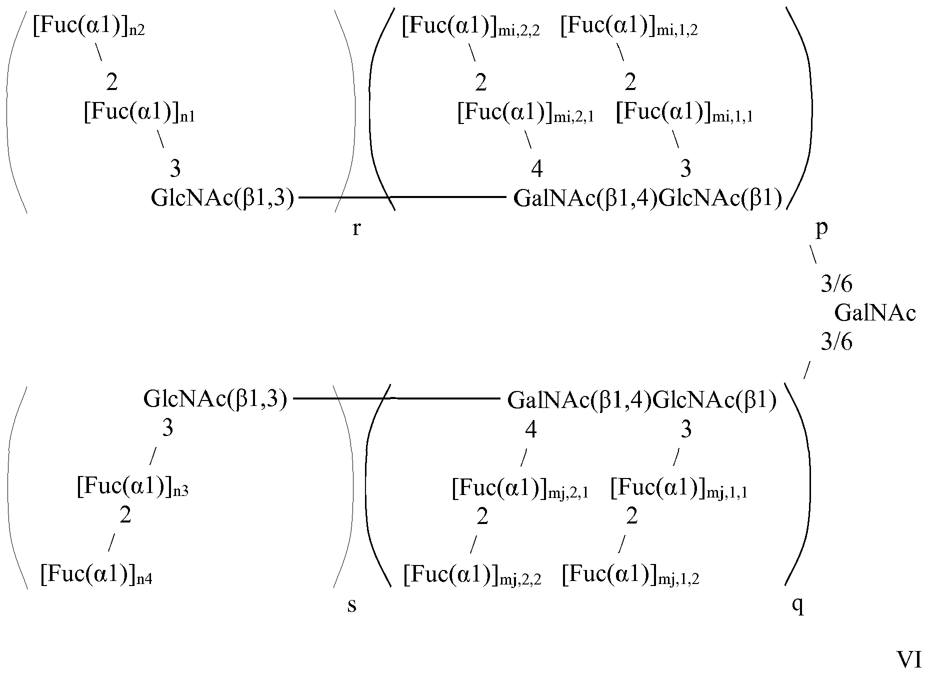 wherein:
p and q are integers equal to 0, 1, 2 or 3,
r and s are integers equal to 0 or 1,
i is an integer:
- equal to 0 when p is equal to 0,
- equal to 1 when p is equal to 1,
- and varying from 1 to 2 when p is equal to 2,
j is an integer:
- equal to 0 when q is equal to 0,
- equal to 1 when q is equal to 1,
- and varying from 1 to 2 when q is equal to 2,
m_{i,1,1}; m_{i,1,2}; m_{i,2,1} m_{i,2,2}; m_{j,1,1}; m_{j,1,2}; m_{j,2,1}; M_{j,2,2} ; n₁; n₂; n₃ and n₄ are integers equal to 0 or 1,
provided that p + q > 0, and:
- p > 0 and there is at least one i such as:
• at least one of m_{i,1,1} and m_{i,1,2} is equal to 1, and
• at least one of m_{i,2,1} and m_{i,2,2} is equal to 1,
and/or
- q > 0 and there is at least one j such as:
• at least one of m_{j,1,1} and m_{j,1,2} is equal to 1, and
• at least one of m_{j,2,1} and m_{j,2,2} is equal to 1,
and/or
- p > 0, r > 0 and:
• at least one of n₁ and n₂ is equal to 1, and
• there is at least one i such as there at least one of m_{i,2,1} and m_{i,2,2} is equal to 1,
and/or
- q > 0, s > 0 and:
• at least one of n₃ and n₄ is equal to 1, and
• there is at least one i such as there at least one of m_{j,2,1} and m_{j,2,2} is equal to 1,
in particular a composition comprising at least two glycopeptides comprising independently of each other a motif of structure VII: 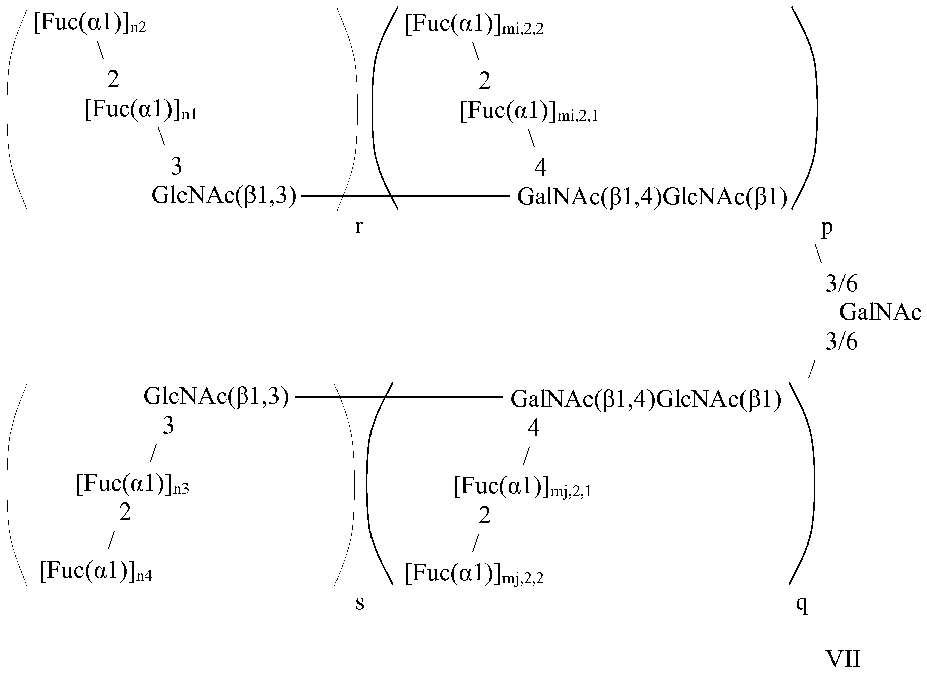
wherein r, p, q, s, m_{i,2,1} ; m_{i,2,2}; M_{j,2,1}; m_{j,2,2} ; n₁; n₂; n₃ and n₄ have the meaning indicated above,
or
in particular a pharmaceutical composition comprising at least two glycopeptides comprising independently of each other a motif of structure IX: 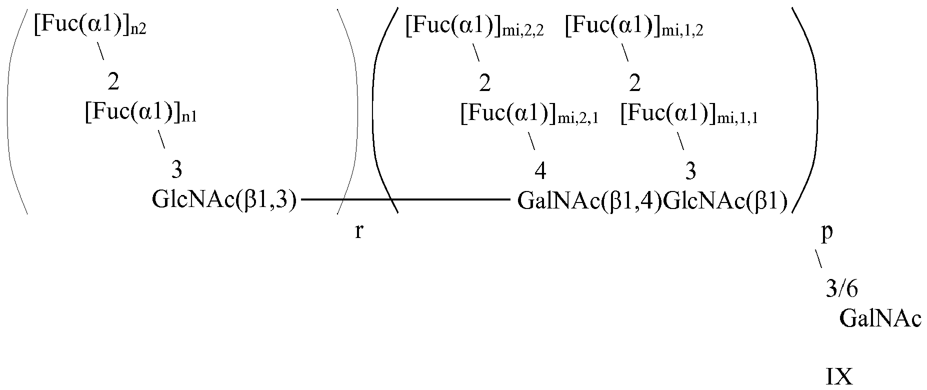
wherein r, p, m_{i,1,1}; m_{i,1,2}; m_{i,2,1} ; m_{i,2,2}; n₁ and n₂ have the meaning indicated above,
or
in particular a pharmaceutical composition comprising at least two glycopeptides comprising independently of each other a motif of structure X: 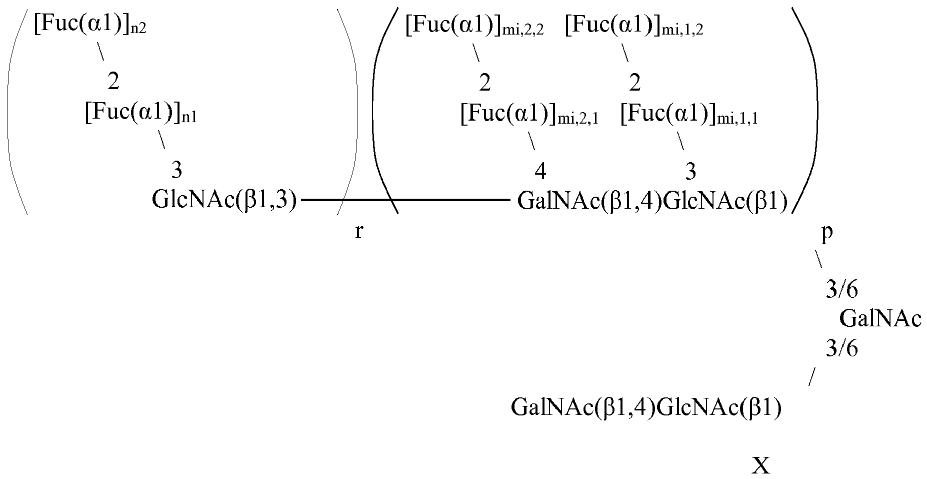
wherein r, p, m_{i,1,1}; m_{i,1,2}; m_{i,1,1} ; m_{i,2,2}; n₁ and n₂ have the meaning indicated above,
or
in particular a pharmaceutical composition comprising at least two glycopeptides comprising independently of each other a motif of structure XI: 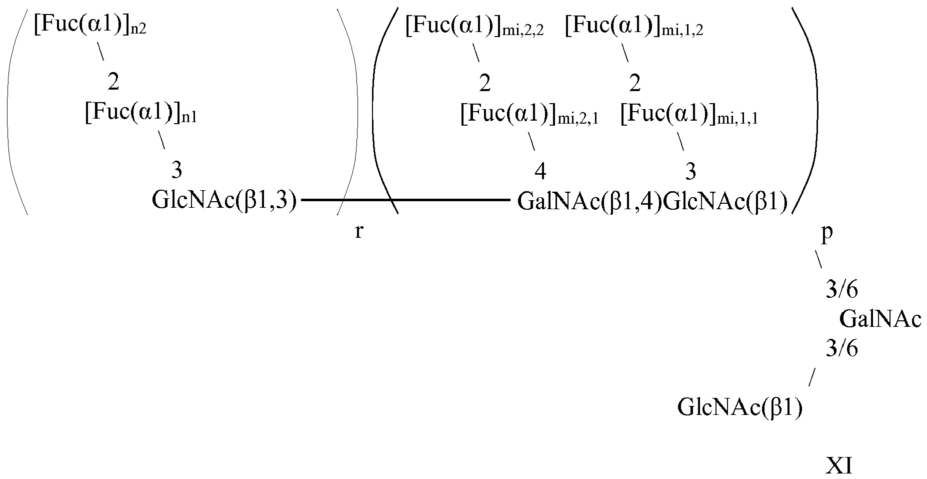
wherein r, p, m_{i,1,1}; m_{i,1,2}; m_{i,2,1} ; m_{i,2,2}; n₁ and n₂ have the meaning indicated above,
or
in particular a pharmaceutical composition comprising at least two glycopeptides comprising independently of each other a motif of structure VI, of the following particular formula XII: 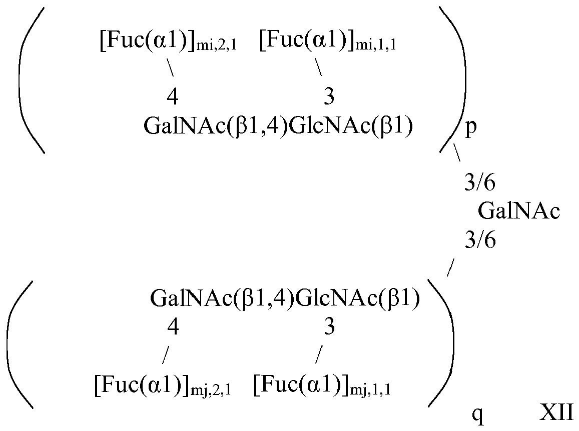
wherein p, q, m_{i,1,1}; m_{i,2,1} ; m_{j,1,1,} and m_{j,2,1} have the meaning indicated above.

13. Pharmaceutical composition according to claim 11, comprising at least two glycans of structure VI-2: 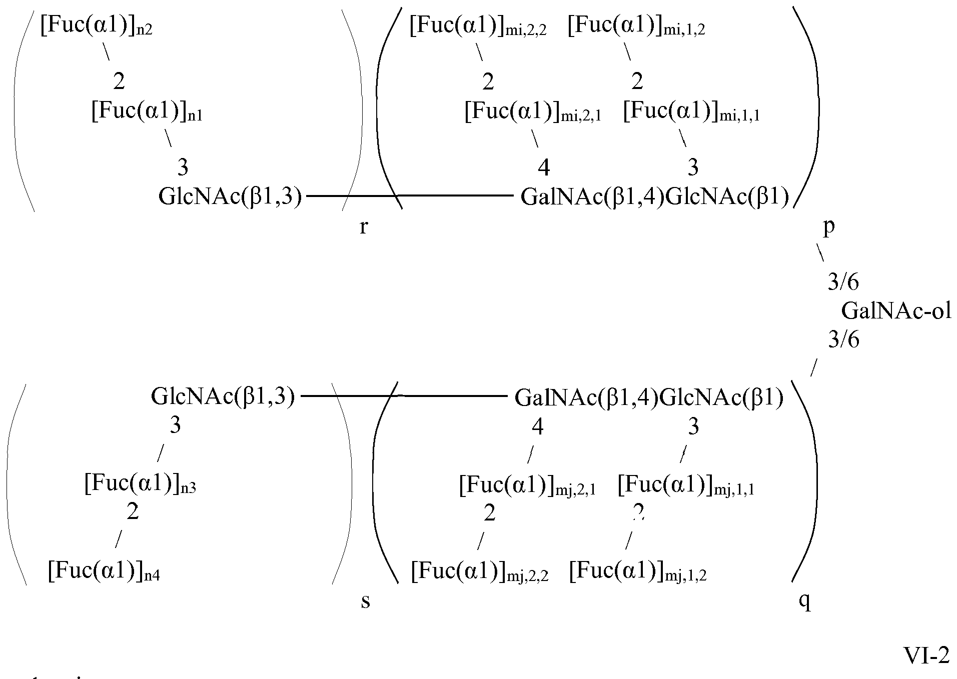 wherein:
p and q are integers equal to 0, 1, 2 or 3,
r and s are integers equal to 0 or 1,
i is an integer:
- equal to 0 when p is equal to 0,
- equal to 1 when p is equal to 1,
- and varying from 1 to 2 when p is equal to 2,
j is an integer:
- equal to 0 when q is equal to 0,
- equal to 1 when q is equal to 1,
- and varying from 1 to 2 when q is equal to 2,
m_{i,1,1}; m_{i,1,2}; m_{i,2,1} ; m_{i,2,2}; m_{j,1,1}; m_{j,1,2}; m_{j,2,1}; m_{j,2,2} ; n₁; n₂; n₃ and n₄ are integers equal to 0 or 1,
provided that p + q > 0, and:
- p > 0 and there is at least one i such as:
• at least one of m_{i,1,1} and m_{i,1,2} is equal to 1, and
• at least one of m_{i,2,1} and m_{i,2,2} is equal to 1,
and/or
- q > 0 and there is at least one j such as:
• at least one of m_{j,1,1} and m_{j,1,2} is equal to 1, and
• at least one of m_{j,2,1} and m_{j,2,2} is equal to 1,
and/or
- p > 0, r > 0 and:
• at least one of n₁ and n₂ is equal to 1, and
• there is at least one i such as there at least one of m_{i,2,1} and m_{i,2,2} is equal to 1,
and/or
- q > 0, s > 0 and:
• at least one of n₃ and n₄ is equal to 1, and
• there is at least one i such as there at least one of m_{j,2,1} and m_{j,2,2} is equal to 1,
in particular a pharmaceutical composition comprising at least two glycans of structure VII-2: 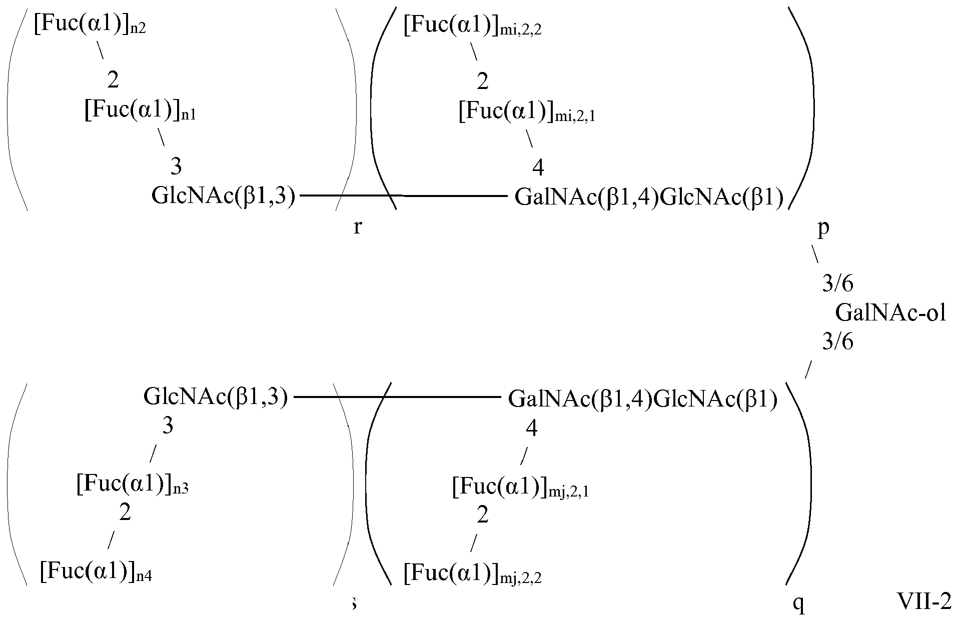
wherein r, p, s, q, m_{i,2,1} ; m_{i,2,2}; m_{j,2,1}; m_{j,2,2} ; n₁ ; n₂; n₃ and n₄ have the meaning indicated above,
or
in particular a pharmaceutical composition comprising at least two glycans of structure IX-2 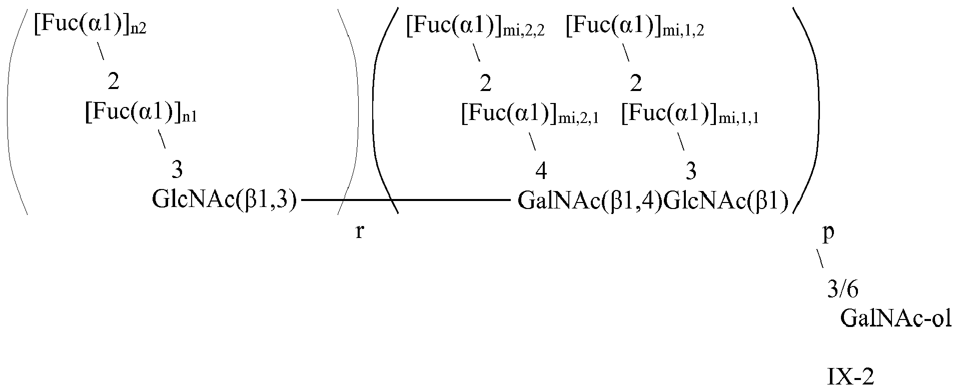
wherein r, p, m_{i,1,1} ; m_{i,1,2}; m_{i,2,1} ; m_{i,2,2}; n₁ and n₂ have the meaning indicated above,
or
in particular a pharmaceutical composition comprising at least two glycans of structure X-2: 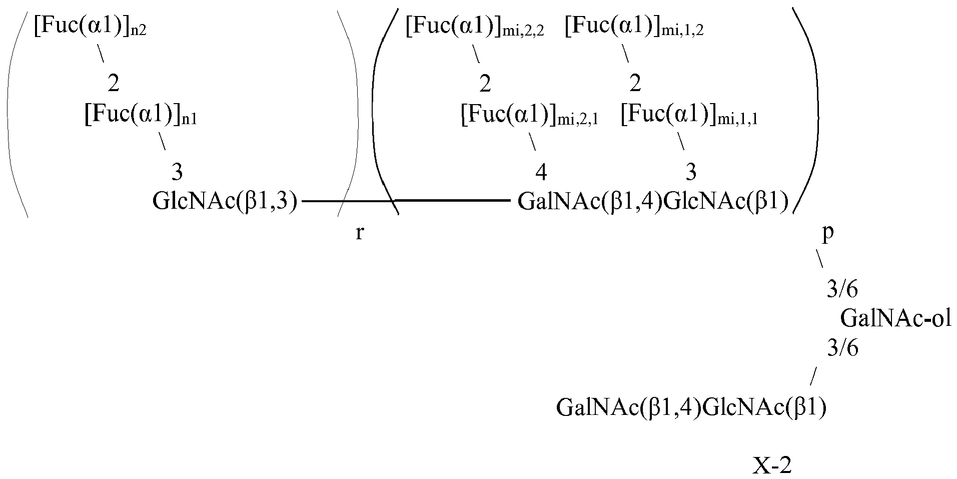
wherein r, p, m_{i,1,1}; m_{i,1,2}; m_{i,1,1} ; m_{i,2,2}; n₁ and n₂ have the meaning indicated above,
or
in particular a pharmaceutical composition comprising at least two glycans of structure XI-2: 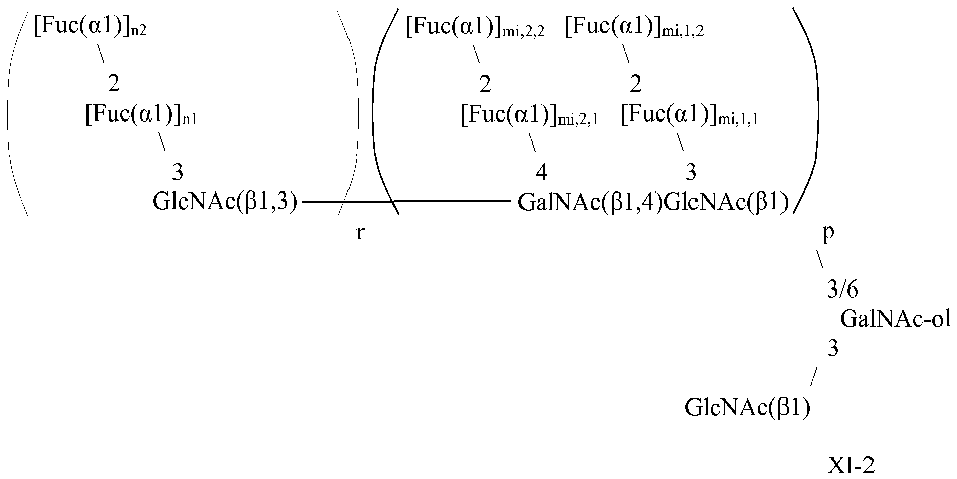
wherein r, p, m_{i,1,1} ; m_{i,1,2}; m_{i,2,1} ; m_{i,2,2}; n₁ and n₂ have the meaning indicated above,
or
in particular a pharmaceutical composition comprising at least two glycans of structure VI-2, of the following particular formula XII-2: 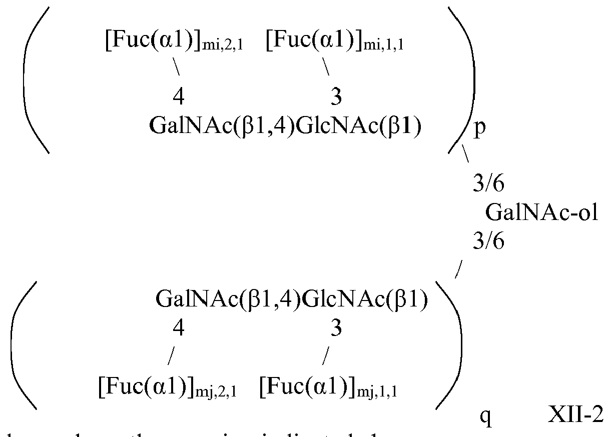
wherein p, q, m_{i,1,1}; m_{i,1,1} ; m_{j,1,1} and m_{j,2,1} have the meaning indicated above.

14. Pharmaceutical composition according to claim 11, comprising at least two glycans of structure XV or glycopeptides comprising independently of each other a motif of structure XV: 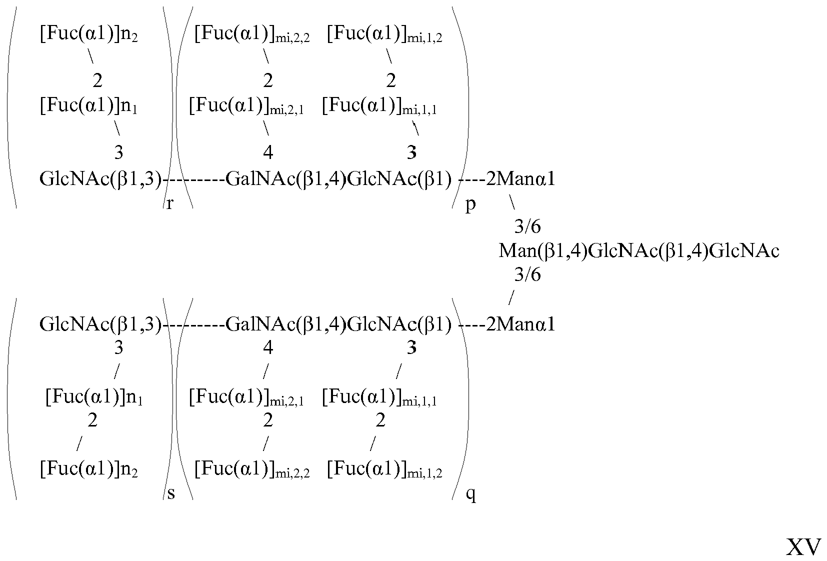 wherein:
p and q are integers equal to 0, 1, 2 or 3
i is an integer:
- equal to 0 when p is equal to 0,
- equal to 1 when p is equal to 1,
- and varying from 1 to 2 when p is equal to 2,
j is an integer:
- equal to 0 when q is equal to 0,
- equal to 1 when q is equal to 1,
- and varying from 1 to 2 when q is equal to 2,
m_{i,1,1}; m_{i,1,2}; m_{i,2,1} ; m_{i,2,2}; m_{j,1,1}; m_{j,1,2}; m_{j,2,1}; m_{j,2,2} ; n₁; n₂; n₃ and n₄ are integers equal to 0 or 1,
provided that p + q > 0, and:
- p > 0 and there is at least one i such as:
• at least one of m_{i,1,1} and m_{i,1,2} is equal to 1, and
• at least one of m_{i,2,1} and m_{i,2,2} is equal to 1,
and/or
- q > 0 and there is at least one j such as:
• at least one of m_{j,1,1} and m_{j,1,2} is equal to 1, and
• at least one of m_{j,2,1} and m_{j,2,2} is equal to 1,
and/or
- p > 0, r > 0 and:
• at least one of n₁ and n₂ is equal to 1, and
• there is at least one i such as there at least one of m_{i,2,1} and m_{i,2,2} is equal to 1,
and/or
- q > 0, s > 0 and:
• at least one of n₃ and n₄ is equal to 1, and
• there is at least one i such as there at least one of m_{j,2,1} and m_{j,2,2} is equal to 1,
in particular a pharmaceutical comprising at least two glycans of structure XV or glycopeptides comprising independently of each other a motif of structure XV, of particular structure XVI: 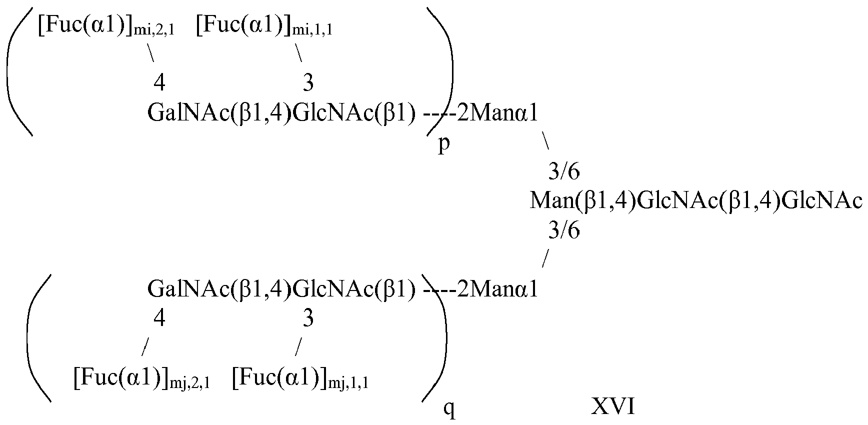
wherein p, q, m_{i,1,1}; m_{i,2,1} ; m_{j,1,1} and m_{j,2,1} have the meaning indicated above.

15. Composition, according to claim 11, comprising at least two glycopeptides comprising independently of each other a motif selected from the following group:
